(19) Europäisches Patentamt
European Patent Office
Office européen des brevets

(11) EP 2 474 627 B1

(12) **EUROPEAN PATENT SPECIFICATION**

(45) Date of publication and mention of the grant of the patent:
**20.05.2015 Bulletin 2015/21**

(51) Int Cl.:
***C12Q 1/68*** (2006.01)

(21) Application number: **12154265.8**

(22) Date of filing: **21.02.2008**

(54) **New markers for cancer**

Neue Krebsmarker

Nouveaux marqueurs du cancer

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.02.2007 DK 200700273**
**24.04.2007 DK 200700601**

(43) Date of publication of application:
**11.07.2012 Bulletin 2012/28**

(62) Document number(s) of the earlier application(s) in accordance with Art. 76 EPC:
**08709178.1 / 2 129 796**

(73) Proprietor: **Oslo Universitetssykehus HF**
**0450 Oslo (NO)**

(72) Inventors:
- **Skotheim, Rolf I.**
**0372 Oslo (NO)**
- **Lothe, Ragnhild A.**
**0267 Oslo (NO)**
- **Lind, Guro E.**
**0770 Oslo (NO)**
- **Ahlquist, Terje C.**
**N-0655 Oslo (NO)**

(74) Representative: **Plougmann & Vingtoft A/S**
**Rued Langgaards Vej 8**
**2300 Copenhagen S (DK)**

(56) References cited:
**WO-A-00/52204** **WO-A-01/49879**
**WO-A-2005/005601** **WO-A-2005/042725**
**WO-A2-2008/073303**

- **KATRIN BEYER: "[alpha]-Synuclein structure, posttranslational modification and alternative splicing as aggregation enhancers", ACTA NEUROPATHOLOGICA, SPRINGER, BERLIN, DE, vol. 112, no. 3, 15 July 2006 (2006-07-15) , pages 237-251, XP019426131, ISSN: 1432-0533, DOI: 10.1007/S00401-006-0104-6**
- **BÖNSCH DOMINIKUS ET AL: "DNA hypermethylation of the alpha synuclein promoter in patients with alcoholism.", NEUROREPORT 8 FEB 2005 LNKD- PUBMED: 15671870, vol. 16, no. 2, 8 February 2005 (2005-02-08), pages 167-170, XP008152360, ISSN: 0959-4965**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 474 627 B1

## Description

### Technical field of the invention

The present invention relates to novel markers for hypermethylation of gene promoters in cancers. In particular the present invention relates to a method of determining whether a tumour is developing in the aero-digestive system, or whether a subject is relapsing after treatment of such a tumour. The methods of the present invention comprise determining the methylation state of CpG sites in the promoter region/sequence of one or more particular genes. The invention further relates to the use of such methylated genes and to diagnostic kits for detecting cancer.

### Background of the invention

Impaired epigenetic regulation is as common as gene mutations in human cancer. These mechanisms lead to quantitative and qualitative gene expression changes causing a selective growth advantage, which may result in cancerous transformation. Aberrantly hypermethylated CpG islands in the gene promoter associated with transcriptional inactivation are among the most frequent epigenetic changes in cancer. Since early detection of disease can result in improved clinical outcome for most types of cancer, the identification of cancer-associated aberrant gene methylation represents promising novel biomarkers. For cancers in the aero-digestive system, including colorectal cancer, initial studies have identified the presence of aberrantly methylated DNA in patient blood and feces. Genes aberrantly hypermethylated in high frequencies already among benign tumours and only rarely in normal mucosa would be good candidate diagnostic biomarkers due to the potential clinical benefit of early detection of high risk adenomas as well as of low risk stages of carcinomas.

In general, however, the sensitivity and specificity of existing early markers for cancers in the aero-digestive system remain poor and, this far, only a few of the genes that have actually been screened for methylation have shown a reasonably high sensitivity and specificity. Specific hypermethylation is seen in *VIM* (vimentin) and *SFRP2* as reported by Muller et al. and Chen et al. and recently, *ADAMTS1* and *CRABP1* were suggested to have a high frequency of cancer specific hypermethylation in colorectal tumours in a report from Lind et al. while the frequency of hypermethylation of *NR3C1* was considerably lower. Lind et al. further identified 18 genes as potential markers for colorectal cancers. In a study by Mori et al. it was concluded that the T-cell differentiation protein MAL, one of the 18 candidate genes discussed by Lind et al. would not be an appropriate diagnostic biomarker for cancers in the aero-digestive tract since the methylation frequency of *MAL* is low, corresponding to only 6% methylation (2/34 samples). Further analyses conducted by the inventors of a number of the 18 candidate genes did not provide encouraging results: NDRG1 was unmethylated in all samples analyzed, NR3C1 was methylated but only at a very low frequency and in subsequent sequence analyses of SDHA it was impossible to confirm the identity of this gene, rendering it unsuitable as a marker for cancer development.

WO2005/005601 discloses genes which are differentially expressed in cancer including SNCA. This document does not analyse the methylation level of the genes.

WO2005/042725 discloses SNCA as a marker for liver cancer. This document does not analyse the methylation level of SNCA.

WO00/52204 discloses NACP (=SNCA) as a marker for bladder cancer. This document does not analyse the methylation level of SNCA.

WO01/49879 discloses NACP (=SNCA) as a marker for colon cancer. This document does not analyse the methylation level of SNCA.

Katrin Beyer, Acta Neuropathologica, Springer, vol. 112, no. 3, 15 July 2006, pages 237-251 discloses the detection of the expression level of SNCA.

Bönsch Dominikus et al., Neuroreport, 8 Feb 2005, vol.16, no. 2, pages 167-170 discloses the analysis of methylation of SNCA in association with alcoholism.

WO01/19845 discloses a method for detecting the methylation state of FBN1 for the noninvasive detection of various tumor types. This document furthermore discloses that the detection of aberrant methylation comprises detecting hypermethylation of the markers concerned as shown in figure 4c for FBN1.

In conclusion, there is no indication that any of the genes discussed by Lind et al. would provide any improvement to current technology for detection of cancer. Consequently, there is a need for a panel of genes in which each gene is hypermethylated at a high frequency and specificity in cancers. In particular, there is a need for a gene panel which is useful in non-invasive techniques, such as techniques involving the use of stool samples, or in techniques which may be used on sample material which is easily obtained, such as blood or mucus. Such a gene panel would greatly improve the possibility for early detection of these cancers. The ultimate goal would be to develop diagnostic tests determining hypermethylation in only a few, such as 2 or 3, high frequency gene markers.

Hence, identification of further genes in which CpG islands in the promoter region are hypermethylated at a high frequency in cancers is desirable.

**Summary of the invention**

The present invention is based on the realization by the inventors that a particular subset of the genes which were identified as potential markers by Lind et al. contain CpG sites that are methylated at an exceptionally high frequency in aero-digestive cancers.

Thus, it is an object of the present invention to provide SNCA as a diagnostic markers for cancer e.g. cancers in the aero-digestive system, in particular colon cancer and colorectal cancer. SNCA solves the problems relating to the low specificity and frequency of methylation in the majority of known markers for cancers.

Accordingly, one aspect of the invention relates to a method for determining whether a subject has developed, is developing or is predisposed for developing cancer, or whether a subject is relapsing after treatment of cancer, comprising the step of:

a) determining the methylation level, the number of methylated CpG sites or the methylation state of CpG sites in a nucleic acid sequence in the promoter region, first exon or intron, of at least one gene in a sample, obtained from said subject, wherein said gene is:

SNCA, e.g. as identified by ensembl gene id ENSG00000145335, entrez id 6622; and wherein said sequence comprises the nucleic acid sequence of SEQ ID NO:13 or SEQ ID NO:14.

The method may further comprise the steps of;

b) comparing the methylation level, the number of methylated CpG sites or the methylation state of CpG sites to a reference; and

c) identifying said subject as being likely to develop, being developing or being predisposed for developing cancer, or relapsing after treatment of cancer, if the methylation level, the number of methylated CpG sites or the methylation state of CpG sites is higher than the methylation level, the number of methylated CpG sites or the methylation state of CpG sites of the reference and identifying a subject as unlikely to develop, being developing or being predisposed for developing cancer, or relapsing after treatment of cancer, if the methylation level, the number of methylated CpG sites or the methylation state of CpG sites is below the methylation level, the number of methylated CpG sites or the methylation state of CpG sites of said reference.

Another aspect of the present disclosure relates to a method for determining whether a subject has developed, is developing or is predisposed for developing cancer, or whether a subject is relapsing after treatment of cancer, comprising the step of;

a) determining the methylation level, the number of methylated CpG sites or the methylation state of CpG sites in a sample from a subject

b) constructing a percentile plot of the methylation level, the number of methylated CpG sites or the methylation state of CpG sites of said at least one gene obtained from a sample from a healthy population;

c) constructing a ROC (receiver operating characteristics) curve based on the methylation level, the number of methylated CpG sites or the methylation state of CpG sites determined in the healthy population and on the methylation level, the number of methylated CpG sites or the methylation state of CpG sites determined in a population with cancer;

d) selecting from the ROC-curve the desired combination of sensitivity and specificity

e) determining from the percentile plot the methylation level, the number of methylated CpG sites or the methylation state of CpG sites corresponding to the determined or chosen specificity; and

f) predicting that the subject is likely to have cancer, if methylation level, the number of methylated CpG sites or the methylation state of CpG sites of said at least one gene in the sample is equal to or higher than said methylation level, the number of methylated CpG sites or the methylation state of CpG sites corresponding to the desired combination of sensitivity/specificity, and predicting that the subject is unlikely or not to have cancer, if the methylation level, the number of methylated CpG sites or the methylation state of CpG sites in the sample is lower than said methylation level, the number of methylated CpG sites or the methylation state of CpG sites corresponding to the desired combination of sensitivity/specificity.

The disclosure further concerns a diagnostic kit for the determination cancer comprising one or more oligonucleotide primers or one or more sets of oligonucleotide primers, which are each complementary to a nucleic acid sequence of the genes selected from:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

The invention also concerns the use of markers according to the invention. Thus the invention further concerns: The use of

SNCA, e.g. as identified by ensembl gene id ENSG00000145335, entrez id 6622.

in a diagnostic assay wherein the methylation level, the number of methylated CpG sites or the methylation state of CpG sites is assessed as an indicator of whether a subject has developed, is developing or is predisposed for developing cancer, within the aero-digestive system or whether a subject is relapsing after treatment of cancer within the aero-digestive system.

In addition, the disclosure concerns the use of a nucleic acid sequence, wherein said nucleic acid comprises a nucleic acid sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer

The disclosure further provides an antibody recognizing a methylated nucleic acid sequences selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in a);
C) A sub-sequence of a nucleic acid sequence as defined in a) or b);
D) A nucleic acid sequence which is at least 75% identical to a sequence as defined in A), B) or C).

**Brief description of the figures**

Figure 1
Show representative methylation-specific polymerase chain reaction results from the analysis of MAL in three normal mucosa samples, three adenomas, and three carcinomas. A visible PCR product in lanes U indicates the presence of unmethylated alleles whereas a PCR product in lanes M indicates the presence of methylated alleles. Abbreviations: A, adenoma; C, carcinoma, N normal mucosa; POS, positive control consisting of normal blood (control for unmethylated samples) and in vitro methylated DNA (control for methylated samples); NEG, negative control (containing water as template); U, lane for unmethylated MSP product; M, lane for methylated MSP product. The illustration is a merge of two gel panels as the adenomas were run on a separate gel.

Figures 2-4
Show up-regulation of gene expression after epigenetic drug treatment of initially methylated cell lines. Up-regulated mRNA expression of CNRIP1, INA, and SPG20 was found in colon cancer cell lines after treatment with the demethylating 5-aza-2'deoxycytidine, alone and in combination with the deacetylase inhibitor trichostatin A. The panels demonstrate the relative expression values of CNRIP1, INA, and SPG20, respectively (linear scale) in six colon cancer cell lines, HT29, SW48, HCT15, SW480, RKO and LS1034, treated with 5-aza-2'deoxycytidine alone (1uM and 10uM), trichostatin A alone, and the two drugs in combination (1 μM 5-aza-2'deoxycytidine alone and 0.5 μM trichostatin A). The two doses (low and high) of 5-aza-2'deoxycytidine gave comparable increases in relative expression values for all three genes. This means that demethylation of cell lines can be achieved by culturing them in the presence of low doses of 5-aza-2'-deoxycytidine, which is an advantage considering the cytotoxicity of this drug. For CNRIP1 and INA the combined treatment was more effective than the individual treatment with 5-aza-2'-deoxycytidine alone and trichostatin A alone. The combined treatment also increased SPG20 expression, however similar or higher reactivation could be achieved by 5-aza-2'-deoxycytidine treatment alone. As expected, treatment with the deacetylase inhibitor trichostatin A alone did not increase the gene expression of neither CNRIP, INA nor SPG20. Abbreviation: AZA, 5-aza-2'deoxycytidine; TSA, trichostatin A.

Figure 5

Show methylation status of the *MAL* promoter in normal colon mucosa samples and colorectal carcinomas. Representative results from methylation-specific polymerase chain reaction are shown. A visible PCR product in lanes U indicates the presence of unmethylated alleles whereas a PCR product in lanes M indicates the presence of methylated alleles. N, normal mucosa; C, carcinoma; Pos, positive control (unmethylated reaction: DNA from normal blood, methylated reaction: *in vitro* methylated DNA); Neg, negative control (containing water as template); U, lane for unmethylated MSP product; M, lane for methylated MSP product.

Figure 6

Show site specific methylation within the *MAL* promoter. Bisulphite sequencing of the *MAL* promoter verifies methylation status assessed by methylation-specific polymerase chain reaction. The upper part of the figure is a schematic presentation of the CpG sites successfully amplified by the two anatyzed bisulphite sequencing fragments, A (-68 to +168; to the right) and B (-427 to -85; to the left). The transcription start site is represented by +1 and the vertical bars indicate the location of individual CpG sites. The two arrows indicate the location of the MSP primers. For the lower part of the figure, filled circles represent methylated CpGs; open circles represent unmethylated CpGs; and open circles with a slash represent partially methylated sites (the presence of approximately 20-80% cytosine, in addition to thymine). The column of U, M and U/M at the right side of this lower part lists the methylation status of the respective cell lines as assessed by us using MSP analyses. Abbreviations: MSP, methylation-specific PCR; s, sense; as, antisense; U, unmethylated; M, methylated; U/M, presence of both unmethylated and methylated band.

Figure 7

Show the "bisulphite sequence" of the *MAL* promoter. Representative bisulphite sequencing electropherograms of the *MAL* promoter in colon cancer cell lines. A subsection of the bisulphite sequence electropherogram, covering CpG sites +11 to +15 relative to transcription start. Cytosines in CpG sites are indicated by a black arrow, whereas cytosines that have been converted to thymines are underlined in red. The *MAL* promoter sequencing electropherograms illustrated here, are from the unmethylated V9P cell line and the hypermethylated ALA and HCT116.

Figure 8

Show MAL expression in cancer cell lines and colorectal carcinomas. Promoter hypermethylation of *MAL* was associated with reduced or lost gene expression in *in vitro* models. The quantitative gene expression level of *MAL* is displayed as a ratio between the average of two *MAL* assays (detecting various splice variants) and the average of the two endogenous controls, *GUSB* and *ACTB*. The value has been multiplied by a factor of 1000. Below each sample the respective methylation status is shown, as assessed by methylation-specific polymerase chain reaction. Filled circles represent promoter hypermethylation of *MAL,* open circles represent unmethylated *MAL*, and open circles with a slash represent the presence of both unmethylated and methylated alleles. Colorectal carcinomas are divided in an unmethylated group (n = 3) and a hypermethylated group (n = 13), and the median expression is displayed here. The tissue of origin for the individual cell lines can be found in table 1.

Figure 9

Show up-regulation of MAL expression after drug treatment. Decreased promoter methylation of MAL followed by up-regulated mRNA expression in colon cancer cell lines was found after treatment with the demethylating 5-aza-2'deoxycytidine, alone and in combination with the deacetylase inhibitor trichostatin A. Upper panel demonstrate the relative expression values of MAL (linear scale) in two colon cancer cell lines, HT29 and HCT15, treated with 5-aza-2'deoxycytidine alone, trichostatin A alone, and the two drugs in combination. Lower panel illustrate MAL MSP results for the same samples. A visible PCR product in lanes U indicates the presence of unmethylated alleles whereas a PCR product in lanes M indicates the presence of methylated alleles. Abbreviation: AZA, 5-aza-2'deoxycytidine; TSA, trichostatin A; Pos, positive control (unmethylated reaction: DNA from normal blood, methylated reaction: in vitro methylated DNA); Neg, negative control (containing water as template); U, lane for unmethylated MSP product; M, lane for methylated MSP product.

Figure 10
Show MAL expression in colorectal carcinomas. Positive cytoplasmic staining of MAL was found in kidney tubuli (A), and no staining was observed in heart muscle (B), in agreement with earlier reports (Marazuela M, et al J Histochem Cytochem 2003, 51: 665-674). The epithelial cells of colorectal carcinomas were MAL negative (C, D), whereas in normal colon tissue, cytoplasmic expression of MAL was found in both epithelia and connective tissue (E, F). All images were captured using the 40x lens (400x magnification).

The present invention will now be described in more detail in the following.

**Detailed description of the invention**

Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. For purposes of the present invention, the following terms are defined:

Epigenetics

Methylation is an epigenetic change which is defined as non-sequence-based alterations that are inherited through cell division.

*Methylation*

"Hypermethylation" is in this context simply methylation above reference methylation. Reference methylation is methylation of the gene in a sample from a healthy subject, or from normal tissue. Thus a methylated gene which in normal tissues is unmethylated will be classified as hypermethylated. The "methylation state" is a measure of the presence or absence of a methyl modification in one or more CpG sites in at least one nucleic acid sequence. It is to be understood that the methylation state of one or more CpG sites is preferably determined in multiple copies of a particular gene of interest.

The "methylation level" is an expression of the amount of methylation in one or more copies of a gene or nucleic acid sequence of interest. The methylation level may be calculated as an absolute measure of methylation within the gene or nucleic acid sequence of interest. Also a "relative methylation level" may be determined as the amount of methylated DNA, relative to the total amount DNA present or as the number of methylated copies of a gene or nucleic acid sequence of interest, relative to the total number of copies of the gene or nucleic acid sequence. Additionally, the "methylation level" can be determined as the percentage of methylated CpG sites within the DNA stretch of interest.

The term methylation level also encompasses the situation wherein one or more CpG site in e.g. the promoter region is methylated but where the amount of methylation is below amplification threshold. Thus methylation level may be an estimated value of the amount of methylation in a gene of interest.

The invention is not in any way limited to certain types of assays for measuring methylation status or methylation level of the genes according to the invention.

In one embodiment if the methylation level of the gene of interest is 15% to 100%, such as 50% to 100%, more preferably 60%- 100 %, more preferably 70-100 %, more preferably 80% to 100%, more preferably 90% to 100%. Thus in one embodiment of the present invention the methylation level of the genes according to the invention is 80%, 81%, 82%, 83%, 84%, 85%, 86%, 87%, 88%, 89%, 90%, 91%, 92%, 93%, 94%, 95%, 96%, 97%, 98%, 99% or 100%.

*CpG*

A "CpG site" is a region of DNA where a cytosine nucleotide occurs next to a guanine nucleotide in the linear sequence of bases along its length. "CpG" stands for cytosine and guanine separated by a phosphate, which links the two nucleosides together in DNA. The "CpG" notation is used to distinguish a cytosine followed by a guanine from a cytosine base paired to a guanine.

A CpG islands may be defined as a contiguous window of DNA of at least 200 base pairs in which the G:C content is at least 50% and the ratio of observed CpG frequency over the expected frequency exceeds 0.6. However, they may also be defined by a more stringent definition as a 500-base-pair window with a G:C content of at least 55% and an observed over expected CpG frequency of at least 0.65.

*Promoter region or sequence*

A "promoter region or sequence" comprises a consecutive nucleic acid sequence extending 1000bp upstream from the transcription start site of a given gene and a consecutive nucleic acid sequence extending 300 base pairs downstream from the transcription start site. In the sequencelist, the upstream sequence is indicated in small letters whereas the downstream sequence is indicated in capital letters. In the 3' part of the sequences small letters indicate intronic sequence.

*Transcription start site*

"Transcription start site" is used in relation to the current invention to describe the point at which transcription is initiated. Transcription can initiate at one or more sites within the gene, and a single gene may have multiple transcriptional start sites, some of which may be specific for transcription in a particular cell-type or tissue.

*Methylation of nucleic acid sequences*

A gene is a region of DNA that is responsible for the production and regulation of a polypeptide chain. Genes include both coding and non-coding portions, including introns, exons, promoters, initiators, enhancers, terminators, microRNAs, and other regulatory elements. As used herein, "gene" is intended to mean at least a portion of a gene. Thus, for example, "gene" may be considered a promoter for the purposes of the present invention. Accordingly, in one embodiment of the present invention, at least one member of the panel of genes comprises a non-coding portion of the entire gene. In a particular embodiment, the non-coding portion of the gene is a promoter. In another embodiment, all members of the entire panel of genes comprise non-coding portions of the genes, such as but not limited to, introns. In another particular embodiment, the non-coding portions of the members of the genes are promoters. In another embodiment of the present invention, at least one member of the panel of genes comprises a coding portion of the gene. In another embodiment, all members of the entire panel of genes comprise coding portions of the genes.

The term "nucleic acid sequence" refers to a polymer of deoxyribonucleotides in either single- or double-stranded form.

A "subsequence" is any portion of an entire sequence. Thus, a subsequence refers to a consecutive sequence of amino acids or nucleic acids which is part of a longer sequence of nucleic acids (e.g. polynucleotide).

The term "sequence identity" indicates a quantitative measure of the degree of homology between two nucleic acid sequences of equal length. If the two sequences to be compared are not of equal length, they must be aligned to give the best possible fit, allowing the insertion of gaps or, alternatively, truncation at the ends of the polypeptide sequences or nucleotide sequences. The sequence identity can be calculated as $\frac{(N_{ref}-N_{dif})100}{N_{ref}}$, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC ($N_{dif}$=2 and $N_{ref}$=8).

With respect to all claims of the invention relating to nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB). Alternatively, the sequences may be analysed using the program DNASIS Max and the comparison of the sequences may be done at www.paralign.org. This service is based on the two comparison algorithms called Smith-Waterman (SW) and ParAlign. The first algorithm was published by Smith and Waterman (1981) and is a well established method that finds the optimal local alignment of two sequences The other algorithm, ParAlign, is a heuristic method for sequence alignment; details on the method is published in Rognes et al. Default settings for score matrix and Gap penalties as well as E-values were used.

In the context of the present invention "complementary" refers to the capacity for precise pairing between two nucleotides sequences with one another. For example, if a nucleotide at a certain position of an oligonucleotide is capable of hydrogen bonding with a nucleotide at the corresponding position of a DNA molecule, then the oligonucleotide and the DNA are considered to be complementary to each other at that position. The DNA strand are considered complementary to each other when a sufficient number of nucleotides in the oligonucleotide can form hydrogen bonds with corresponding nucleotides in the target DNA to enable the formation of a stable complex.

In the present context the expressions "complementary sequence" or "complement" therefore also refer to nucleotide sequences which will anneal to a nucleic acid molecule of the invention under stringent conditions.

The term "stringent conditions" refers to general conditions of high, weak or low stringency.

The term "stringency" is well known in the art and is used in reference to the conditions (temperature, ionic strength and the presence of other compounds such as organic solvents) under which nucleic acid hybridizations are conducted. With "high stringency" conditions, nucleic acid base pairing will occur only between nucleic acid fragments that have a high frequency of complementary base sequences, as compared to conditions of "weak" or "low" stringency. Suitable conditions for testing hybridization involve pre-soaking in 5xSSC and pre-hybridizing for 1 hour at ~40°C in a solution of 20% formamide, 5xDenhardt's solution, 50mM sodium phosphate, pH 6.8, and 50mg of denatured .sonicated calf thymus DNA, followed by hybridization in the same solution supplemented with 100mM ATP for 18 hours at ~40°C, followed by three times washing of the filter in 2xSSC, 0.2% SDS at 40°C for 30 minutes (low stringency), preferred at 50°C (medium stringency), more preferably at 65°C (high stringency), even more preferably at ~75°C (very high stringency). More details about the hybridization method can be found in Sambrook et al., Molecular Cloning: A Laboratory Manual, 2nd Ed., Cold Spring Harbor, 1989.

*Cancer*

"Cancer" is a group of diseases in which cells are aggressive (grow and divide without respect to normal limits), invasive (invade and destroy adjacent tissues), and sometimes metastatic (spread to other locations in the body). These three malignant properties of cancers differentiate them from benign tumours, which are self-limited in their growth and do usually not invade or metastasize. Cancer is usually classified according to the tissue from which the cancerous cells originate, as well as the normal cell type they most resemble. A definitive diagnosis usually requires histologic examination of a tissue biopsy specimen by a pathologist. The prognosis of cancer patients is most influenced by the type of cancer, as well as the stage, or extent of the disease. An early diagnose is usually associated with a more successful treatment and increased survival rate.

"Tumour suppressor genes" are genes often inactivated in cancer cells, resulting in the loss of normal functions in those cells, such as accurate DNA replication, control over the cell cycle, orientation and adhesion within tissues, and interaction with protective cells of the immune system. In several cancers including colorectal cancer, several tumour suppressor genes have been identified to be epigenetically inactivated by CpG island promoter hypermethylation.

A tumour may be any abnormal swelling, lump or mass however as the term is interpreted herein the term means neoplasm, specifically solid neoplasm.

Neoplasm is defined as an abnormal proliferation of genetically altered cells. Neoplasms can be benign or malignant. Malignant neoplasm or malignant tumour, is to be understand here as cancer. Benign neoplasm or benign tumour is a tumour (solid neoplasm) that normally stops growing by it self, and does not invade other tissues and does not form metastases. However, benign tumours may become malignant.

Tumours invading surrounding tissues are to be understood herein as cancer. Pre-malignancy, pre-cancer or non-invasive tumour is to be understood herein as a neoplasm that is not invasive but has the potential to progress to cancer (become invasive) if left untreated.

The methods according to the invention can be used to determine the degree of severity i.e. stages, such as Dukes system, the Astler-Coller system and TNM staging AJCC (American joint committee on cancer). Duke system is a four-class staging system that classifies colorectal carcinoma from A to D based on the extent of the tumour: A, penetration into but not through the bowel wall; B, penetration through the bowel wall; C, lymph node involvement regardless of extent of bowel wall penetration; D, spreading of cancer to distant organs, e.g. liver and lung. Many modifications of this classification exist, e.g. TNM staging

*Biomarker*

A biomarker can be a substance whose detection indicates a particular disease state. A biomarker may also indicate a change in expression or state of a protein that correlates with the risk or progression of a disease, or with the susceptibility of the disease to a given treatment. A good biomarker can be used to diagnose disease risk, presence of disease in an individual, or to tailor treatments for the disease in an individual. The terms biomarker and marker is used interchangeably in the present context.

Cancer marker, tumour marker and in this context methylation marker is a marker for detecting cancer and/or tumour. The marker may be used for detecting in a subject the presence of cancer and/or tumour, or a developing cancer and/or tumour, or whether the subject is predisposed or relapsing from cancer and/or tumour

The genes according to the invention may be a marker, a biomarker, a cancer marker or a tumour marker respectively.

*Tumour progression*

In addition to determining whether a subject has developed, is developing or is predisposed for developing cancer, or whether a subject is relapsing after treatment of cancer, the methods according to the invention may also be used for detecting the progression of cancer in a subject. This may be done by determining the methylation state or level of one or more genes in a subject at different time points, and then determine the difference in methylation state or level of one or more genes over time. A difference in methylation state or level over time may be indicative of whether the subject has developed, is developing or is predisposed for developing cancer, or whether a subject is relapsing after treatment of cancer.

The present invention also provides a method for making a prognosis about disease course in a human cancer patient. For the purposes of this invention, the term "prognosis" is intended to encompass predictions and likelihood analysis of disease progression, particularly tumour recurrence, metastatic spread and disease relapse. The prognostic methods of the invention are intended to be used clinically in making decisions concerning treatment modalities, including therapeutic intervention, diagnostic criteria such as disease staging, and disease monitoring and surveillance for metastasis or recurrence of neoplastic disease. Treatment is to be understood herein as both preventive and curative treatment.

The present invention also provides methods for conforming the results or indications obtained by a preceding method such as a test or screening method.

Thus the phrase "developed, are developing or is predisposed for developing cancer, or whether a subject is relapsing after treatment of cancer" as used herein encompasses determination and/or prediction such as estimation or determination the likelihood of current presence of, future occurrence of or future recurrence of cancer.

*Sample*

A sample may be but is not limited to a tissue section or biopsy, such as a portion of the neoplasm that is being treated or it may be a portion of the surrounding normal tissue. The sample may preferably be but is not limited to blood, stool (feaces), urine, pleural fluid, gall, bronchial fluid, oral washings, tissue biopsies, ascites, pus, cerebrospinal fluid, aspirate, follicular fluid, tissue or mucus. The sample may be processed prior to being assayed. For example, the sample may be diluted, concentrated or purified and/or at least one compound, such as an internal standard, may be added to the sample. The procedures for handling different samples are known to the skilled artisan.

It is to be understood that all methods according to the invention preferably concern in vitro analyses of a sample.

*Sample methylation frequency*

The term "sample methylation frequency" is defined herein as a quantitative measurement of methylated samples i.e. the relative number of samples in which the gene of interest is methylated. As an example, the sample methylation frequency of CNRIP1 is 100 %, 20 out of 20 samples from colon cell lines are methylated, as apparent from table 3. The relative amount of methylated samples is compared to a reference or cut-off level which is estimated on basis of the sensitivity and the specificity of each gene.

*Reference*

In order to determine whether a subject has developed, is developing or is predisposed for developing cancer, or whether a subject is relapsing after treatment of cancer, a reference or reference level or value has to be established. The reference also makes it possible to count in assay and method variations, kit variations, handling variations, variations related to combining the markers with each other or with other known markers, and other variations not related directly or indirectly to methylation.

In the context of the present invention, the term "reference" relates to a standard in relation to quantity, quality or type, against which other values or characteristics can be compared, such as a standard curve.

The reference or reference level is to be understood In the present context as a value or level, which has been determined by measuring the parameter (methylation state or methylation level) in both a healthy control population and a population with known cancer thereby determining the reference value which identifies the cancer population with either a predetermined specificity or a predetermined sensitivity based on an analysis of the relation between the parameter values and the known clinical data of the healthy control population and the cancer patient population.

As will be generally understood by those of skill in the art, methods for screening for cancers are processes of decision making by comparison. For any decision-making process, reference-values, reference-levels or cut-off points based on subjects having cancer or a condition of interest and/or subjects not having cancer, or a condition of interest are needed.

The reference level (or the cut-off point or cut-off level) can be established by taking into account several criteria including the acceptable number of subjects who would go on for further invasive diagnostic testing, the average risk of having and/or developing e.g. cancer to all the subjects who go on for further diagnostic testing, a decision that any subject whose patient specific risk is greater than a certain risk level such as 1 in 400 or 1:250 (as defined by the screening organization or the individual subject) should go on for further invasive diagnostic testing or other criteria known to those skilled in the art.

The reference level can be adjusted based on several criteria such as but not restricted to certain groups of individuals tested. As an example the cut-off level may be set lower in individuals with immunodeficiency and in patients at great risk of progressing to active disease or the reference level may be set higher in groups of otherwise healthy individuals with low risk of developing active disease.

The reference level may be different for various stages of disease (e.g. benign tumour or malign tumour), the source of normal mucosa (from cancer free individuals versus cancer patients) or from the source of blood and faces. In addition, the reference level may be different for subjects predisposed for or subjects relapsing from treatment of disease.

Reference levels can be customized to accommodate a specific sensitivity or specificity: If one desires a test with high sensitivity the reference level can be set low. If one seeks a test with high specificity the reference level can be set higher.

Depending on the prevalence or expected prevalence of presence of disease, the reference level can be adjusted for obtaining as few false positive or as few false negative results as wanted, depending on the severity of the disease and the consequences of determining, whether the patient is positive for the test or negative for the test.

The methods for measuring methylation, the chosen part of nucleic acid sequences comprising the promoter region of the marker genes or other parameters will result in other reference values, which can be determined in accordance with the teachings herein.

The reference level can be different, if a single patient with symptoms has to be diagnosed or the test is to be used in a screening of a large number of individuals in a population.

The reference level can be based on combined methylation state or level measurements of different markers such as but not limited to CNRIP1, SPG20, FBN1, SNCA, INA, MAL, ADAMTS1, VIM, SFRP1 and/or SFRP2. A compound reference level may result in other values, which can be determined in accordance with the teachings of the present invention.

The level of methylation is compared to a set of reference data or a reference-level, such as the cut-off value, to determine whether the subject is at an increased risk or likelihood of cancer.

*Specificity and sensitivity*

The sensitivity of any given screening test is the proportion of individuals with the condition who are correctly identified or diagnosed by the test, e.g. the sensitivity is 100%, if all individuals with a given condition have a positive test. The specificity of a given screening test is the proportion of individuals without the condition who are correctly identified or diagnosed by the test, e.g. 100 % specificity is, if all individuals without the condition have a negative test result.

Thus the sensitivity is defined as the (number of true-positive test results) / (number of true-positive + number of false- negative test results).

The specificity is defined as (number of true-negative results) / (number of true-negative + number of false-positive results)

The genes according to the present application is characterized by having high sensitivity (the relative amount of samples comprising the methylated gene of interest from subjects with cancer is high) and high specificity (the relative amount of samples comprising the methylated gene of interest from subjects without cancer is low).

A good marker for cancer is a gene which is methylated in almost all samples when a subject has cancer, and not methylated when in samples from subject not having cancer.

The specificity of the method according to the present invention is preferably from 70% to 100%, such as from 75% to 100%, more preferably 80% to 100%, more preferably 90% to 100%. Thus in one embodiment of the present invention the specificity of the invention is 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

The sensitivity of the method according to the present invention is preferably from 80% to 100%, more preferably 85% to 100%, more preferably 90% to 100%. Thus in one embodiment of the present invention the sensitivity of the invention is 80%, 81%, 82%, 83%, 84%, 85%, 86%, 87%, 88%, 89%, 90%, 91%, 92%, 93%, 94%, 95%, 96%, 97%, 98%, 99% or 100%.

It is to be understood that the markers according to the invention may be used in combinations in the methods according to the invention. Using several markers in combination is likely to increase the specificity and/or sensitivity of the assay as compared with an assay involving the use of a single marker. When several markers are used in combination it may therefore be acceptable that the specificity and sensitivity of each marker is lower than as specified above.

As an example illustrated in table 3 the gene CNRIP1 is methylated in all samples 20 of 20 from a colon cancer cell line (100%) and in 45 of 48 (94 %) adenoma samples - that is this gene has high sensitivity, the probability of detecting disease is 100 and 94 % from the respective samples. Whereas the methylation of the same genes in samples from normal tissue is 0 of 21 the gene has thus high specificity the chance of detecting false positive are 0 and all cancer free individuals are detected. Samples from normal mucosa from cancer patients showed methylation in 9 of 21 samples indicating that cancer can be detected at a distance from a tumour.

*Receiver-operating characteristics*

Accuracy of a diagnostic test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the reference level over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease, latent or recent infection versus no infection, or benign versus malignant disease.

In each case, the ROC plot depicts the overlap between the two distributions by plotting the sensitivity versus 1 - specificity for the complete range of decision thresholds. On the y-axis is the sensitivity, which is calculated entirely from the affected subgroup. On the x axis is the false-positive fraction, or 1 - specificity, which is calculated entirely from the unaffected subgroup.

Because the sensitivity and specificity are calculated entirely separately, by using the test results from two different subgroups, the ROC plot is independent of the prevalence of disease in the sample. Each point on the ROC plot represents a sensitivity/specificity pair corresponding to a particular decision threshold. A test with perfect discrimination (no overlap in the two distributions of results) has an ROC plot that passes through the upper left corner, where the true- positive fraction is 1.0, or 100% (perfect sensitivity), and the false- positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always $\geq 0.5$ (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Clinical utility of the novel cancer marker genes may be assessed in comparison to and in combination with other markers for the given cancer e.g. clinical utility of the novel cancer markers CNRIP1, SPG20, FBN1, SNCA, INA and MAL assessed in comparison to: established diagnostic tools e.g. measuring the expression level of the corresponding or established methylation markers such as but not limited to ADAMTS1, VIM, SFRP1, SFRP2 and CRABP1.

*Risk assessment and cut-off*

To determine whether the subject is at increased risk of developing e.g. cancer, a cut-off limit for positive test must be established. This.cut-off may be established by the laboratory, the physician or on a case by case basis by each subject.

Alternatively a cut-off point can be determined as the mean, median or geometric mean of the negative control group (e.g. not having cancer) +/- one or more standard deviations or a value derived from the standard deviation)

The cut-off limit for positive test result according to the invention is the methylation state or level for which methylation is an indicator of cancer.

Another cut-off point may be the amount of CpG sites which needed to be methylated for a gene to be determined to be methylated.

The present inventors have successfully identified new markers for cancer. The methylation state of CpG sites or level of methylation in the promoter region of the nucleic acid sequence of a gene selected from CNRIP1, SPG20, FBN1, SNCA, INA and MAL is increased in subjects with cancer and thus these genes are efficient markers for detection of e.g. cancer.

Cut-off points can vary based on specific conditions of the individual tested such as but not limited to the risk of having the disease, occupation, geographic residence or exposure.

Cut-off points can vary based on specific conditions of the individual tested such as but not limited to age, sex, genetic background (i.e. HLA-type), acquired or inherited compromised immune function (e.g. HIV infection, diabetes, patients with renal or liver failure, patients in treatment with immune-modifying drugs such as but not limited to corticosteroids, chemotherapy, TNF-$\alpha$ blockers, mitosis inhibitors).

Doing adjustment of decision or cut-off limit will thus determine the test sensitivity for detecting cancer, if present, or its specificity for excluding cancer or disease if below this limit. Then the principle is that a value above the cut-off point indicates an increased risk and a value below the cut-off point indicates a reduced risk.

*Expression*

Several tumour suppressor genes have been identified to be inactivated by CpG island promoter methylation. As an example the *MLH1* gene in which hypermethylation of a limited number of CpG sites approximately 200 base pairs upstream of the transcription start point invariably correlates with the lack of gene expression.

The present analyses of cancer cell lines from several tissues indicate that the hypermethylation of a limited area in the proximity of the transcription start point of MAL is associated with reduced or lost gene expression. Quantitative gene expression results from colon cancer cell lines analyzed before and after epigenetic drug treatment indicate that this also holds true for SPG20, INA and CNRIP1.

Thus measuring of methylation state or level and, in addition, the expression of the gene of interest increases the specificity and sensitivity of the method.

Methods

The present invention is based on the finding that SNCA, which is hypermethylated at an exceptionally high frequency in cancers such as colorectal cancer.

In a first aspect the present invention provides a method for determining whether a subject has developed is developing or is predisposed for developing cancer, or whether a subject is relapsing after treatment of cancer, comprising the step of:

a) determining the methylation level, the number of methylated CpG sites or the methylation state of CpG sites in a nucleic acid sequence in the promoter region, first exon or intron, of at least one gene in a sample, obtained from said subject, wherein said gene is:

SNCA, e.g. as identified by ensembl gene Id ENSG00.000145335, entrez id 6622; and wherein said sequence comprises the nucleic acid sequence of SEQ ID NO:13 or SEQ ID NO:14.

In one embodiment cancer is a tumour such as a tumour in the aero-digestive system (benign or malignant),

The method may further comprise the steps of:

b) comparing the methylation level, the number of methylated CpG sites or the methylation state of CpG sites to a reference; and

c) identifying said subject as being likely to develop, developing or being predisposed for developing cancer, or relapsing after treatment of cancer, if the methylation level, the number of methylated CpG sites or the methylation state of CpG sites is higher than the methylation level, the number of methylated CpG sites or the methylation state of CpG sites of the reference and identifying a subject as unlikely to develop, developing or being predisposed for developing cancer, or relapsing after treatment of cancer, if the methylation level, the number of methylated CpG sites or the methylation state of CpG sites is below the methylation level, the number of methylated CpG sites or the methylation state of CpG sites of said reference.

In another aspect the present disclosure provides a method for determining whether a subject has developed, is developing or is predisposed for developing cancer or whether a subject is relapsing after treatment of cancer, comprising the steps of

a) determining the methylation level, the number of methylated CpG sites or the methylation state of CpG sites in a sample from a subject

b) constructing a percentile plot of the methylation level, the number of methylated CpG sites or the methylation state of CpG sites of said at least one gene obtained from a sample from a healthy population;

c) constructing a ROC.(receiver operating characteristics) curve based on the methylation level, the number of methylated CpG sites or the methylation state of CpG sites determined in the healthy population and on the methylation level, the number of methylated CpG sites or the methylation state of CpG sites determined in a population with cancer;

d) selecting from the ROC-curve the desired combination of sensitivity and specificity

e) determining from the percentile plot the methylation level, the number of methylated CpG sites or the methylation state of CpG sites corresponding to the determined or chosen specificity; and

f) predicting that the subject is likely to have cancer, if methylation level, the number of methylated CpG sites or the methylation state of CpG sites of said at least one gene in the sample is equal to or higher than said methylation level, the number of methylated CpG sites or the methylation state of CpG sites corresponding to the desired combination of sensitivity/specificity, and predicting that the subject is unlikely or not to have cancer, if the methylation level, the number of methylated CpG sites or the methylation state of CpG sites in the sample is lower than said methylation level, the number of methylated CpG sites or the methylation state of CpG sites corresponding to the desired combination of sensitivity/specificity.

In particular the method as described above comprises a method comprising determining methylation level, the number of methylated CpG sites or the methylation state of CpG sites in a nucleic acid sequence comprising a sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

The method as described above may also comprise determining the methylation state of CpG sites in a nucleic acid sequence of the additional genes selected from the group consisting of:

A) A nucleic acid sequence as defined by any of SEQ ID NO.: 1, SEQ ID NO.: 2, SEQ ID NO.: 3, SEQ ID NO.: 4, SEQ ID NO.: 5, SEQ ID NO.: 8, SEQ ID NO.: 10, SEQ ID NO.: 11, SEQ ID NO.: 12, SEQ ID NO.: 15 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

In another embodiment the method of the disclosure comprises determining the methylation state of CpG sites in the promoter region of MAL. According to this embodiment the nucleic acid sequence is

A) A nucleic acid sequence as defined by SEQ ID NO.: 1;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

Sequence identifiers 1-16 represent nucleic acids sequences of the above mentioned genes. As the person of skills in the art will realize, it is within the scope of the present invention to analyze the methylation state of CpG sites within these sequences as well as within their complementary sequences.
The following table lists the genes according to the disclosure, and corresponding id numbers, sequence identifiers and aliases.

| HGNC name | Entrez | Ensemb | SEQ ID NO. | Aliases | Approved name |
|---|---|---|---|---|---|
| MAL | 4118 | ENSG00000172005 | 1-4 | | T-cel differentiation protein |
| FBN1 | 2200 | ENSG00000166147 | 6 | FBN; SGS; WMS; MASS; MFS1; OCTD | fibrillin 1 |
| CNRIP1 | 25927 | ENSG00000119865 | 7 | DKFZP566K1924, CRIP1, CRIP1a, CRIP1b, chromosome 2 open reading frame 32, C2orf32 | cannabinoid receptor interacting protein 1 |
| SPG20 | 23111 | ENSG00000133104 | 9 | SPARTAN; TAHCCP1; KIAA0610 | spastic paraplegia 20 |
| SNCA | 6622 | 6622 ENSG00000145335 | 13, 14 | NACP, PD1, PARK1, PARK4, MGC110988 | synuclein, alpha (non A4 component of amyloid precursor) |
| INA | 9118 | ENSG00000148798 | 16 | NEF5; NF-66; TXBP-1; MGC12702 | internexin neuronal intermediate filament protein, alpha |

The nucleic acid sequences according to the invention are listed in the sequence list. Each sequence comprises in the order of mentioning and in the 5' to 3' orientation: a consecutive sequence of nucleic acid residues located within the 1000 bp region upstream of the transcription start site (indicated in small letters) followed by a consecutive sequence of nucleic acid residues located downstream of the transcription start site (indicated in capital letters) and by intronic sequence of nucleic acid residues (indicated in small letters).

The method of the invention may be directed against analyzing particular subsequences as suggested under item C) above. According to these embodiments, the sub-sequence in C) has a length of at least 8 nucleic acid residues, such as a length of at least 9 nucleic acid residues, at least 10 nucleic acid residues, at least 11 nucleic acid residues, at least 12 nucleic acid residues, at least 13 nucleic acid residues, at least 14 nucleic acid residues, at least 15 nucleic acid residues, at least 20 nucleic acid residues, at least 25 nucleic acid residues, at least 30 nucleic acid residues, at least 35 nucleic acid residues, at least 40 nucleic acid residues, at least 45 nucleic acid residues, at least 50 nucleic acid residues, at least 70 nucleic acid residues, or such as a length of at least 90 nucleic acid residues. It is generally desirable to direct the analyses against sequences of a certain length in order to ensure that the method is sufficiently sensitive.

For practical purposes it may also be desirable to minimize the length of the sub-sequences that are subject to the methylation studies in the method of the invention. Accordingly, it may be desirable that the said sub-sequence in C) has a length of at the most 10 nucleic acid residues, such as at the most 13 nucleic acid residues, at the most 14 nucleic acid residues, at the most 15 nucleic acid residues, at the most 20 nucleic acid residues, at the most 25 nucleic acid residues, at the most 30 nucleic acid residues, at the most 35 nucleic acid residues, at the most 40 nucleic acid residues, at the most 45 nucleic acid residues, at the most 50 nucleic acid residues, at the most 70 nucleic acid residues, at the most 90 nucleic acid residues, at the most 110 nucleic acid residues, at the most 150 nucleic acid residues, or such as at the most 200 nucleic acid residues.

More particularly it may be desirable that the sub-sequence in C) have a length of between 8 and 200 nucleic acid residues, such as a length between 8 and 150 nucleic acid residues, between 8 and 100 nucleic acid residues, between 8 and 75 nucleic acid residues, between 8 and 50 nucleic acid residues, between 9 and 200 nucleic acid residues, such as a length between 9 and 150 nucleic acid residues, between 9 and 100 nucleic acid residues, between 9 and 75 nucleic acid residues, between 9 and 50 nucleic acid residues, such as a length between 10 and 200 nucleic acid residues, between 10 and 150 nucleic acid residues, between 10 and 100 nucleic acid residues, between 10 and 75 nucleic acid residues, between 10 and 50 nucleic acid residues, such as a length between 11 and 200 nucleic acid residues, between 11 and 150 nucleic acid residues, between 11 and 100 nucleic acid residues, between 11 and 75 nucleic acid residues, between 11 and 50 nucleic acid residues, or such as a length between 12 and 200 nucleic acid residues, such as a length between 12 and 150 nucleic acid residues, between 12 and 100 nucleic acid residues, between 12 and 75 nucleic acid residues, or such as a length between 12 and 50 nucleic acid residues.

The promoter regions of the genes according to the disclosure are listed in the table below:

| HGNC name | Entrez | Ensemb | SEQ ID NO. |
|---|---|---|---|
| | | | |
| MAL | 4118 | ENSG00000172005 | 17-20 |
| FBN1 | 2200 | ENSG00000166147 | 22 |
| CNRIP1 | 25927 | ENSG00000119865 | 23 |
| SPG20 | 23111 | ENSG00000133104 | 25 |
| SNCA | 6622 | ENSG00000145335 | 29, 30 |
| INA | 9118 | ENSG00000148798 | 32 |

For each of the genes mentioned above, the inventors have identified sub-sequences that are particularly useful in the method of the disclosure. For MAL the sub-sequence in C) may, accordingly, be selected from the group of sequences consisting of the sequence specified by SEQ ID NO.: 17 and its complementary sequence, the sequence specified by SEQ ID NO.: 18 and its complementary sequence, the sequence specified by SEQ ID NO.: 19 and its complementary sequence, the sequence specified by SEQ ID NO.: 20 and its complementary sequence, and sub-sequences of any of these sequences.

For the fibrillin 1 gene the sub-sequence in C) is preferably the sequence specified by SEQ ID NO.: 22, or its complimentary sequence, or a subsequence of one of these.

For the chromosome 2 open reading frame 32, (CNRIP1), the sub-sequence in C) is preferably the sequence specified by SEQ ID NO.:23, or its complementary sequence, or a subsequence of one of these.

For the spastic paraplegia 20, spartin (Troyer syndrome) the sub-sequence in C) is preferably the sequence specified by SEQ ID NO.:25, or its complementary sequence, or a subsequence of one of these.

For synuclein, alpha (non A4 component of amyloid precursor) the sub-sequence in C) are preferably selected from the group of sequences consisting of the sequence specified by SEQ ID NO.: 29 and its complementary sequence, the sequence specified by SEQ ID NO.: 30 and its complementary sequence, and sub-sequences of any of these sequences.

For internexin neuronal intermediate filament protein, alpha the sub-sequence in C) is preferably the sequence specified by SEQ ID NO.:32, or its complementary sequence, or a subsequence of one of these.

Also useful in the present invention may be the nucleic acids comprising the promoter region of additional genes selected from but not limited to the group of: myocyte enhancer factor 2C (SEQ ID NO.: 24), C3orf14/14HT021 (SEQ ID NO.:21), ubiquitin protein ligase E3A (SEQ ID NO.: 26, 27 and 28), brain expressed, X-linked 1 (SEQ ID NO.:31), or their complementary sequence, or a subsequence of one of these.

Hitherto few genes have proven useful for early detection of cancer based on methylation events in the promoter regions. It is however within the scope of the present invention to include in the method analyses of the methylation state or level in promoter regions of known markers for hypermethylation in cancer. In further embodiments of the invention, therefore, the method comprises determining the methylation state or level of CpG sites in the promoter region/sequence of one or more genes, said one or more genes being selected from the group consisting of:

Adam metallopeptidsase with thrombospondin type 1 motif (ADAMTS1, C3-C5, KIAA1346, METH1)
Vimentin (VIM)
Secreted Frizzled-related protein 1 (SFRP1); and
Secreted Frizzled-related protein 2 (SFRP2)

The promoter regions of these genes are represented by sequence identifiers 35-38. Accordingly, the method of the invention may comprise determining the methylation state of CpG sites in a nucleic acid sequence comprising a sequence selected from the group consisting of:

i) A nucleic acid sequence as defined by any of SEQ ID NO.: 33, SEQ ID NO.: 34, SEQ ID NO.: 35, and SEQ ID NO.: 36;
ii) A nucleic acid sequence which is complementary to a sequence as defined in i);
iii) A sub-sequence of a nucleic acid sequence as defined in i) or ii);
iv) A nucleic acid sequence which is at least 75%, such as at least 80%, at least 85%, at least 90%, at least 95%, at least 98% or at least 99%, identical to a sequence as defined in i), ii) or iii).

The skilled person will further realize that the various promoter regions will show some degree of degeneracy.

Accordingly, as indicated under item D) above the promoter sequence for any of the particular genes may be one which is not entirely identical to one of the sequences represented by sequence identifiers 1-16. In particular embodiments the nucleic acid sequence in D) is at least 80% identical to a sequence as defined in A), B) or C), such as at least 85%, at least 90%, at least 95%, at least 98%, at least 99%, or such as at least 99.5% identical to a sequence as defined in A), B) or C).

The specificity and sensitivity of the genes according to the invention are very high and each of the genes may be comprised in the methods of the invention.

In one embodiment the method comprises determining the methylation level, the number of methylated CpG sites or the methylation state of CpG sites in a nucleic acid sequence in the promoter region in

I) a nucleic acid sequence comprising a sequence selected from the group consisting of: SEQ ID NO.s.: 1-4, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence consisting of: SEQ ID NO: 6, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence consisting of: SEQ ID NO: 7, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I).a nucleic acid sequence comprising a sequence consisting of: SEQ ID NO: 9, and its related sequences, and
II).in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence selected from the group consisting of: SEQ ID NO: 13-14, and its related sequences, and
II) in 1, 2 or 3 nucleic acid sequences as defined in the previous paragraph.

In one embodiment the method comprises determining the methylation level, the number of methylated CpG sites or the methylation state of CpG sites in a nucleic acid sequence in the promoter region in

I) a nucleic acid sequence comprising a sequence consisting of: SEQ ID NO: 16, and its related sequences, and
II) In 1, 2 or 3 nucleic acid sequences as defined in the previous paragraph.

The methods according to the invention are aimed at detecting or diagnosing a cancer, such as a tumour, within the aero-digestive system..The "Aero-digestive system" or "aero-digestive tract" includes the lungs and the gastrointestinal tract: esophageus, stomach, pancreas, liver, gall bladder/bile duct, small bowel, and large intestine, including the colon and rectum. In particular, the tumour may be selected from the group consisting of: colorectal tumours, lung tumours (including small cell lung cancer and/or non-small cell lung cancer), esophageal tumours, gastric tumours, pancreas tumours, liver tumours, tumours of the gall bladder and/or bile duct, tumours of the small bowel and tumours of the large bowel.

Thus in one embodiment of the invention cancer is selected from the group consisting of: colorectal tumours, lung tumours (including small cell lung cancer and/or non-small cell lung cancer), esophageal tumours, gastric tumours, pancreas tumours, liver tumours, tumours of the gall bladder and/or bile duct, tumours of the small bowel and tumours of the large bowel.

In order to determine the number of methylated CpG sites, the methylation state of the CpG sites or the methylation level in said promoter region/sequence, the method according to the invention requires that a sufficient amount of DNA be isolated from the particular subject. The skilled artisan will know of suitable techniques for isolating and purifying DNA in the amounts and quality required. For most purposes DNA may be isolated from a blood sample, a fecal sample, a tissue sample or a sample of mucus from the lungs from said subject. In general, it is desirable to perform the method according to the invention, in a non-invasive manner whenever this is possible: For gastro intestinal cancers collecting DNA from faecal samples will often be practical and convenient. In relation to lung tumours isolating DNA from mucus samples from the lung may offer a convenient approach to non-invasive collection of DNA. For other tumours, including tumours in the liver and pancreas, it may be preferred to collect tissue samples for subsequent isolation of DNA.

Thus in one embodiment the sample is obtained from blood, stool, urine, pleural fluid, gall, bronchial fluid, oral washings, tissue biopsies, ascites, pus, cerebrospinal fluid aspirate, follicular fluid, tissue or mucus.

When the methods according to the invention is used for the purpose of merely determining the presence of cancer or a tumour in the aero-digestive system, in particular where a "yes/no"- type of result is required. It is desirable if the method can be limited to analyzing the methylation level or methylation state of CpG sites in the promoter regions of 2-4 genes. This clearly requires that the genes have an extremely high frequency of hypermethylation during cancer development and progression.

Thus for simple diagnostic purposes it is mostly preferred to limit the analysis to the promoter regions within very few genes. As mentioned above this requires the availability of a panel of markers for hypermethylation, wherein each marker has a high sensitivity and specificity. For other more subtle purposes, however, it may be necessary to analyze the methylation state or level of promoter regions in a larger number of marker genes. The methods according to the invention may therefore comprise determining the methylation state of CpG sites in a nucleic acid sequence in the promoter region/sequence of at least 2 genes, such as at least 3 genes, such as at least 4 genes, such as at least 5 genes, such as at least 7 genes, at least 8 genes, at least 9 genes, at least 10 genes, at least 11 genes, at least 12 genes, at least 13 genes, at least 14 genes, at least 15 genes, at least 16 genes, at least 17 genes, at least 18 genes, at least 19 genes or at least 20 genes, including at least 1 gene as defined in claim 1 in order to determine the risk level for tumour initiation and/or progression in a subject.

In accordance with what is explained above the method of the invention further comprising determining the methylation state of CpG sites in at least one such as at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or such as at least 20, additional nucleic acid sequences as defined above or their related sequences.

Thus for most purposes it will be insufficient to analyze the methylation level, the number of methylated CpG sites or the methylation state of CpG sites of the promoter region of a single gene. The methods according to the invention may therefore comprise determining the methylation state of CpG sites in a nucleic acid sequence in the promoter region/sequence of at least 2 genes, such as at least 3 genes, such as at least 4 genes, such as at least 5 genes, such as at least 7 genes, at least 8 genes, at least 9 genes, at least 10 genes, at least 11 genes, at least 12 genes, at least 13 genes, at least 14 genes, at least 15 genes, at least 16 genes, at least 17 genes, at least 18 genes, at least 19 genes or at least 20 genes, wherein at least one gene is selected from the group of genes defined above

Thus in another aspect the invention concern a method wherein the methylation level, the number of methylated CpG sites or the methylation state of CpG sites of at least one additional marker is determined.

Wherein at least one additional marker is selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118
MAL, e.g. as identified by ensembl gene id ENSG00000172005

*Combination of marker genes*

Thus for the reasons explained above the methylation level or methylation state may be combined with measurements of one or more other markers, and compared to a combined reference-level. The measured marker levels can be combined by arithmetic operations such as addition, subtraction, multiplication and arithmetic manipulations of percentages, square root, exponentiation, and logarithmic functions. Levels can also be combined following manipulations using various models e.g. logistic regression and maximum likelihood estimates. Various biomarker combinations and various means of calculating the combined reference-value can be performed by means known to the skilled addressee.

Thus, another embodiment of the invention concerns a method according to any of the proceeding claims, where the methylation level or-methylation state of at least one additional marker is determined.

The at least one additional marker may be but are not limited to CNRIP1, SPG20, FBN1, SNCA, INA, MAL, ADAMTS1, VIM, SFRP1 or SFRP2, CRABP1. The markers can be compared to a set of reference data to determine whether the subject has cancer or is at increased risk of developing cancer.

A method of constructing a diagnostic test based on a combined marker may be achieved by combining the methylation levels or methylation state (or a value derived hereof) of two or more individual markers by arithmetic manipulation (e.g. addition). As there may be variety in methylation level or methylation state of the different markers it is relevant to weigh the measurements in order for a combination to be achieved independent of differences in e.g. the level or state of methylation. This can be done by simple normalization from a median or.mean from a standard material.

*Synergy*

By combination of the different marker genes according to the invention a synergistic effect may be achieved.

Specifically as used herein synergy refers to the phenomenon in which several markers acting together creates a "combined marker signal" with greater sensitivity or specificity for diagnosis, than that predicted by knowing only the separate markers sensitivity or specificity.

Thus in one embodiment of the present invention the combined use of at least one additional marker (e.g. CNRIP1, SPG20, FBN1, SNCA, INA, MAL) provides a synergistic effect in relation to sensitivity and/or specificity.

In another embodiment of the present invention the combined use of the markers CNRIP1 and INA provides a synergistic effect in relation to sensitivity and/or specificity.

In another embodiment of the present invention the combined use of the markers CNRIP1 and SNCA provides a synergistic effect in relation to sensitivity and/or specificity.

In another embodiment of the present invention the combined use of the markers CNRIP1 and FBN1 provides a synergistic effect in relation to sensitivity and/or specificity.

In another embodiment of the present invention the combined use of the markers CNRIP1 and SPG20 provides a synergistic effect in relation to sensitivity and/or specificity.

In another embodiment of the present invention the combined use of the markers INA and SNCA provides a synergistic effect in relation to sensitivity and/or specificity.

In another embodiment of the present invention the combined use of the markers INA and FBN1 provides a synergistic effect in relation to sensitivity and/or specificity.

In another embodiment of the present invention the combined use of the markers INA and SPG20 provides a synergistic effect in relation to sensitivity and/or specificity.

In another embodiment of the present invention the combined use of the markers SNCA and FBN1 provides a synergistic effect in relation to sensitivity and/or specificity.

In another embodiment of the present invention the combined use of the markers SNCA and SPG20 provides a synergistic effect in relation to sensitivity and/or specificity.

In another embodiment of the present invention the combined use of the markers FBN1 and SPG20 provides a synergistic effect in relation to sensitivity and/or specificity.

In another embodiment of the present invention the combined use of the markers CNRIP1 and MAL provides a synergistic effect in relation to sensitivity and/or specificity.

In another embodiment of the present invention the combined use of the markers SPG20 and MAL provides a synergistic effect in relation to sensitivity and/or specificity.

In another embodiment of the present invention the combined use of the markers FBN1 and MAL provides a synergistic effect in relation to sensitivity and/or specificity.

In another embodiment of the present invention the combined use of the markers SNCA and MAL provides a synergistic effect in relation to sensitivity and/or specificity.

In another embodiment of the present invention the combined use of the markers INA and MAL provides a synergistic effect in relation to sensitivity and/or specificity.

In another embodiment of the present invention the combined use of the markers CNRIP1, SPG20 and INA provides a synergistic effect in relation to sensitivity and/or specificity.

In another embodiment of the present invention the combined use of the markers CNRIP1, SPG20 and FBN1 provides a synergistic effect in relation to sensitivity and/or specificity.

In another embodiment of the present invention the combined use of the markers CNRIP1, SPG20 and SNCA provides a synergistic effect in relation to sensitivity and/or specificity.

In another embodiment of the present invention the combined use of the markers CNRIP1, INA and SNCA provides a synergistic effect in relation to sensitivity and/or specificity.

In another embodiment of the present invention the combined use of the markers CNRIP1, INA and FBN1 provides a synergistic effect in relation to sensitivity and/or specificity.

In another embodiment of the present invention the combined use of the markers CNRIP1, SNCA and FBN1 provides a synergistic effect in relation to sensitivity and/or specificity.

In another embodiment of the present invention the combined use of the markers SNCA, SPG20 and FBN1 provides a synergistic effect in relation to sensitivity and/or specificity.

In another embodiment of the present invention the combined use of the markers INA, SPG20 and FBN1 provides a synergistic effect in relation to sensitivity and/or specificity.

In another embodiment of the present invention the combined use of the markers INA, SNCA and FBN1 provides a synergistic effect in relation to sensitivity and/or specificity.

In another embodiment of the present invention the combined use of the markers INA, SPG20 and SNCA provides a synergistic effect in relation to sensitivity and/or specificity.

In another embodiment of the present invention the combined use of the markers MAL, SPG20 and SNCA provides a synergistic effect in relation to sensitivity and/or specificity.

In another embodiment of the present invention the combined use of the markers MAL, INA and SNCA provides a synergistic effect in relation to sensitivity and/or specificity

In another embodiment of the present invention the combined use of the markers MAL, INA and SPG20 provides a synergistic effect in relation to sensitivity and/or specificity

In another embodiment of the present invention the combined use of the markers MAL, FBN1 and SNCA provides a synergistic effect in relation to sensitivity and/or specificity

In another embodiment of the present invention the combined use of the markers MAL, FBN1 and SPG20 provides a synergistic effect in relation to sensitivity and/or specificity

In another embodiment of the present invention the combined use of the markers MAL, FBN1 and INA provides a synergistic effect in relation to sensitivity and/or specificity

In another embodiment of the present invention the combined use of the markers MAL, FBN1 and CNRIP1 provides a synergistic effect in relation to sensitivity and/or specificity

In another embodiment of the present invention the combined use of the markers MAL, CNRIP1 and SNCA provides a synergistic effect in relation to sensitivity and/or specificity

In another embodiment of the present invention the combined use of the markers MAL, CNRIP1 and SPG20 provides a synergistic effect in relation to sensitivity and/or specificity

In another embodiment of the present invention the combined use of the markers MAL, CNRIP1 and INA provides a synergistic effect in relation to sensitivity and/or specificity

In another embodiment of the present invention the combined use of the markers CNRIP1, FBN1, SNCA, and INA provides a synergistic effect in relation to sensitivity and/or specificity.

In another embodiment of the present invention the combined use of the markers SPG20, FBN1, SNCA, and INA provides a synergistic effect in relation to sensitivity and/or specificity.

In another embodiment of the present invention the combined use of the markers CNRIP1, SNCA, INA and SPG20 provides a synergistic effect in relation to sensitivity and/or specificity.

In another embodiment of the present invention the combined use of the markers CNRIP1, FBN1, INA and SPG20 provides a synergistic effect in relation to sensitivity and/or specificity.

In another embodiment of the present invention the combined use of the markers CNRIP1, FBN1, SNCA and SPG20 provides a synergistic effect in relation to sensitivity and/or specificity.

In another embodiment of the present invention the combined use of the markers MAL, FBN1, SNCA and SPG20 provides a synergistic effect in relation to sensitivity and/or specificity.

In another embodiment of the present invention the combined use of the markers CNRIP1, MAL, SNCA and SPG20 provides a synergistic effect in relation to sensitivity and/or specificity.

In another embodiment of the present invention the combined use of the markers CNRIP1, FBN1, MAL and SPG20 provides a synergistic effect in relation to sensitivity and/or specificity.

In another embodiment of the present invention the combined use of the markers CNRIP1, FBN1, SNCA and MAL provides a synergistic effect in relation to sensitivity and/or specificity.

In another embodiment of the present invention the combined use of the markers INA, FBN1, SNCA and MAL provides a synergistic effect in relation to sensitivity and/or specificity.

In another embodiment of the present invention the combined use of the markers CNRIP1, INA, SNCA and MAL provides a synergistic effect in relation to sensitivity and/or specificity.

In another embodiment of the present invention the combined use of the markers CNRIP1, FBN1, INA and MAL provides a synergistic effect in relation to sensitivity and/or specificity.

In another embodiment of the present invention the combined use of the markers CNRIP1, SPG20, FBN1, SNCA, and INA provides a synergistic effect in relation to sensitivity and/or specificity.

In another embodiment of the present invention the combined use of the markers MAL, SPG20, FBN1, SNCA, and INA provides a synergistic effect in relation to sensitivity and/or specificity.

In another embodiment of the present invention the combined use of the markers CNRIP1, MAL, FBN1, SNCA, and INA provides a synergistic effect in relation to sensitivity and/or specificity.

In another embodiment of the present invention the combined use of the markers CNRIP1, SPG20, MAL, SNCA, and INA provides a synergistic effect in relation to sensitivity and/or specificity.

In another embodiment of the present invention the combined use of the markers CNRIP1, SPG20, FBN1, MAL, and INA provides a synergistic effect in relation to sensitivity and/or specificity.

In another embodiment of the present invention the combined use of the markers CNRIP1, SPG20, FBN1, SNCA, and MAL provides a synergistic effect in relation to sensitivity and/or specificity.

Thus in one embodiment the methods according to the invention comprises determining the methylation level, the number of methylated CpG sites or the methylation state of CpG sites of CNRIP1 combined with determining the methylation level, the number of methylated CpG sites or the methylation state of CpG sites of at least one additional marker selected from the group comprising:

SPG20, e.g. as identified by ensembl gene id ENSG00000133104, entrez id 23111
FBN1, e.g. as identified by ensembl gene id ENSG00000166147, entrez id 2200
SNCA, e.g. as identified by Ensembl gene id ENSG00000145335, entrez id 6622; and
INA, e.g. as identified by ensembl gene id ENSG00000148798, entrez id 9118

Thus in one embodiment the methods according to the invention comprises determining the methylation level, the number of methylated CpG sites or the methylation state of CpG sites of SPG20 combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Thus in one embodiment the methods according to the invention comprises determining the methylation level, the number of methylated CpG sites or the methylation state of CpG sites of FBN1 combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
SPG20, e.g. as identified by ensembl gene id ENSG00000133104, entrez id 23111
SNCA, e.g. as identified by ensembl gene id ENSG00000145335, entrez id 6622; and
INA, e.g. as identified by ensembl gene id ENSG00000148798, entrez id 9118

Thus in one embodiment the methods according to the invention comprises determining methylation level, the number of methylated CpG sites or the methylation state of CpG sites of SNCA combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200; and
INA, e.g. as identified by ensembl gene id ENSG00000148798, entrez id 9118

Thus in one embodiment the methods according to the invention comprises determining methylation level, the number of methylated CpG sites or the methylation state of CpG sites of INA combined with determining methylation level, the number of methylated CpG sites or the methylation state of CpG sites of at least one additional marker selected from the group comprising:

CNRIP1, e.g. as identified by ensembl gene id ENSG00000119865, entrez id 25927;
SPG20, e.g. as identified by ensembl gene id ENSG00000133104, entrez id 23111;
FBN1, e.g. as identified by ensembl gene id ENSG00000166147, entrez id 2200; and
SNCA, e.g. as identified by ensembl gene id ENSG00000145335, entrez id 6622

*Bisulphite treatment and methylation-specific polymerase chain reaction*

The invention is not limited by the types of assays used to assess methylation state of the members of the gene or gene panel. Indeed, any assay that can be employed to determine the methylation state or level of the gene or gene panel should suffice for the purposes of the present invention.

A practical approach to determining the methylation state of CpG islands in a promoter region may comprise a step of treating the promoter sequence with bisulphite. Bisulphite treatment of DNA leads to sequence variations as unmethylated but not methylated cytosines are converted to uracil. Bisulphite treatment followed by sequence analyses allows a positive display of 5-methyl cytosines in the gene promoter after bisulphite modification as unmethylated cytosines appear as thymidines, whereas 5-methyl cytosines appear as cytosines in the final sequence. In particular embodiments of the invention the methylation state of said promoter region/sequence is therefore determined by nucleic acid sequencing (bisulphite sequencing).

In further embodiments of the invention, the number of methylated CpG sites, the methylation state of CpG sites or the methylation level of said promoter region/sequence is determined by methylation specific PCR. In the examples of the present application a set of suitable PCR conditions and primer designs is given. In general, however, the skilled person will have the knowledge required in order for him to be able to determine appropriate conditions and primer designs for PCR analyses.

As the skilled person will know real-time fluorescence offers a convenient and rapid approach to the detection of PCR products and may readily be applied in diagnostic procedures where a high throughput is required. In currently preferred embodiment of the invention said methylation specific PCR thus comprises real-time fluorescence detection of the PCR products.

As for most other PCR procedures the method of the invention may comprise a step of separating the products according to size. In particular, the methods of the invention may comprise a step of separating the resulting PCR products by gel- or capillary electrophoresis.

As part of the analyses the resulting PCR products may be detected by the use of a label selected from the group consisting of fluorescent labels, chemiluminescent label and radioactive labels. For safety and practical reasons non-radioactive labels are preferred for most purposes.

The methylation state or level of said promoter region/sequence may also be determined by pyrosequencing, mass spectrometry or by use of methylation specific restriction enzymes.

The methylation level, the number of methylated CpG sites or the methylation state of CpG sites is determined by, but are not limited to, bisulphite sequencing, quantitative and/or qualitative methylation specific polymerase chain reaction (MSP), pyrosequencing, Southern blotting, restriction landmark genome scanning (RLGS), single nucleotide primer extension, CpG island microarray, SNUPE, COBRA, mass spectrometry, by use of methylation specific restriction enzymes, by measuring the expression level of said genes or a combination thereof.

In preferred embodiments the methylation specific PCR used in the method of the invention comprises the use of nucleic acid primers which are capable of hybridizing to a nucleic acid sequence comprising 2 CpG sites and a cytosine residue which is not within a CpG site. The inclusion of such a cytosine residue which is not methylated, is desired in order to better distinguish bisulphite converted DNA from non-bisulphite converted DNA. Primers for methylated sequences will always bind to methylated CpG sites, which are sites that remain CpG after bisulphite conversion. In the presence of unconverted DNA this will contain CpG sites independently of methylation status and the methylation specific primers will then bind to the unconverted DNA, creating false positives. The inclusion of a "C" which is not in a CpG site in the area targeted by the primer will prevent the primer from binding to un-converted DNA as this DNA will contain "C" while the converted DNA will contain "T" at the same site.

In still further embodiments the methylation specific PCR comprises the use of nucleic acid primers which are capable of hybridizing to a nucleic acid sequence comprising 2 CpG sites and a cytosine residue which is not within a CpG site.

The methods according to the invention can be combined with any other known parameter for cancer. Thus the methylation level or state of a gene of the invention may be combined with but not limited to any of the following parameters for cancer: a genetic DNA integrity assay, ploidi, mutation status of genes, genomic changes, fusions genes, splice variants, differences in expression, miRNAs.

Use

SNCA is due to its high sensitivity and specificity very suitable for use as markers for cancer. Thus another aspect of the invention concern the use of

SNCA, e.g. as identified by ensembl gene id ENSG00000145335, entrez id 6622

in a diagnostic assay wherein the methylation level, the number of methylated CpG sites or the methylation state of CpG sites is assessed as an indicator of whether a subject has developed, is developing or is predisposed for developing cancer within the aero-digestive system, or whether a subject is relapsing after treatment of cancer within the aero-digestive system.

Another embodiment concerns the use of a nucleic acid sequence, wherein said nucleic acid comprises a nucleic acid sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer.

Antibody

The disclosure also concerns an antibody for the methylated sequences. Thus another embodiment of the invention concerns an antibody recognizing a methylated nucleic acid sequences selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in a);
C) A sub-sequence of a nucleic acid sequence as defined in a) or b);
D) A nucleic acid sequence which is at least 75% identical to a sequence as defined in A), B) or C).

Diagnostic kit

In a preferred aspect the disclosure provides a diagnostic kit for the determination of cancer comprising one or more oligonucleotide primers or one or more sets of oligonucleotide primers, which are each complementary to a nucleic acid sequence of the genes selected from:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez Id 2200
SNCA, e.g. as identified by ensembl gene id ENSG00000145335, entrez id 6622; and
INA, e.g. as identified by ensembl gene id ENSG00000148798, entrez id 9118

A second aspect of the disclosure provides a diagnostic kit comprising one or more oligonucleotide primers or one or more sets of oligonucleotide primers, such as 2 or more, 3 or more, 4 or more, 5 or more, 6 or more, 7 or more, 8 or more, 9 or more, 10 or more, 11 or more, 12 or more, 13 or more, 14 or more, 15 or more 16 or more, 17 or more, 18 or more, 19 or more or such as 20 or more oligonucleotide primers or sets of oligonucleotide primers which are each complementary to/capable of hybridizing to a nucleic acid sequence in the promoter region/sequence of one or more genes, said one or more genes being selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ensembl gene id ENSG00000148798, entrez id 9118

In particular, the kit according to this aspect of the disclosure comprises one or more oligonucleotide primers or sets of oligonucleotide primers which are each complementary to/capable of hybridizing to a nucleic acid sequence comprising a sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
A nucleic acid sequence which is at least 75% identical to a sequence as defined in A), B) or C).

In particular embodiments the kit comprises one or more oligonucleotide primers or one or more sets of oligonucleotide primers which are each complementary to/capable of hybridizing to a nucleic acid sequence in the promoter region/sequence of a gene being selected from the group consisting of:

Adam metallopeptidsase with thrombospondin type 1 motif (ADAMTS1, C3-C5, KIAA1346, METH1)
Vimentin (VIM)
Secreted Frizzled-related protein 1 (SFRP1); and
Secreted Frizzled-related protein 2 (SFRP2)
MAL (T cell differentiation protein)
chromosome 3 open reading frame (C3orf14/14HT021),
ubiquitin protein ligase E3A (UBE3A, AS, ANCR, E6-AP, FU26981),
brain expressed, X-linked 1 (BEX1),
myocyte enhancer factor 2C, MEF2c

In another aspect of the disclosure, the kit comprises one or more oligonucleotide primers or sets of oligonucleotide primers which are each complementary to/capable of hybridizing to a nucleic acid sequence comprising a sequence selected from the group consisting of:

A) A nucleic acid sequence as defined by any of SEQ ID NO.: 1, SEQ ID NO.: 2, SEQ ID NO.: 3, SEQ ID NO.: 4, SEQ ID NO.: 5, SEQ ID.NO.: 8, SEQ ID NO.: 10, SEQ ID NO.: 11, SEQ ID NO.: 12 and SEQ ID NO.: 15
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

According to these embodiments the kit comprises one or more oligonucleotide primers or one or more sets of oligonucleotide primers which are each complementary to/capable of hybridizing to a nucleic acid sequence comprising a sequence selected from the group consisting of:

i) A nucleic acid sequence as defined by any of SEQ ID NO.: 33, SEQ ID NO.: 34, SEQ ID NO.: 35, and SEQ ID NO.: 36;
ii)A nucleic acid sequence which is complementary to a sequence as defined in i);
iii) A sub-sequence of a nucleic acid sequence as defined in i) or ii);
iv) A nucleic acid sequence which is at least 75%, such as at least 80%, at least 85%, at least 90%, at least 95%, at least 98% or at least 99%, identical to a sequence as defined in i), ii) or iii).

In view of the extremely high specificity of MAL as a marker for cancer the kit comprises one or more oligonucleotide primers or one or more sets of oligonucleotide primers which are each complementary to/capable of hybridizing to a nucleic acid sequence selected from the group consisting of

A) A nucleic acid sequence as defined by any of SEQ ID NO.: 1;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

Various designs may be contemplated for the kit of the disclosure. In one embodiment each of the said primers or sets of primers are in separate containers. This will allow the end user of the kit to prepare different primer mixtures for different purposes. According to other embodiments, however, the primers or sets of primers may be supplied in a mixture.

For certain use, such as in traditional diagnostic purposes, the diagnostic kit may include few primers or sets of primers. In particular, this is relevant when the kit is to be used in applications where a "yes/no"- type of result is required, such as when the kit is used simply in order to determine whether a tumour or carcinoma is developing in the aero-digestive system. For such purposes the number of primers or sets of primers in the kit may be limited to 2 to 4, wherein at least one primer or one set of primers, such as at least 2, at least 3 or at least 4 primers or sets of primers, is complementary to/capable of hybridizing to a nucleic acid sequence according to SEQ ID NO.: 1-16 or sequences that are complementary or partly identical thereto as defined above under items C) and D).

As discussed above, however, in relation to the method of the invention, the methylation state of CpG islands of the marker genes of the invention may also be used for more complex analyses, as in order to determine the risk level for tumour initiation and/or progression in a subject.

In such embodiments the diagnostic kit of the disclosure will typically contain primers or sets of primers that are able to target a larger number of marker genes. A kit for such purposes will typically need to include 5 or more primers or sets of primers, wherein at least one primer or one set of primers, such as at least 2, at least 3, at least 4, at least 5, at least 6, at least 7, at least 8, at least 9, at least 10, at least 12, at least 13, at least 14, at least 15, or such as at least 16 primers or one set of primers, is complementary to/capable of hybridizing to a nucleic acid sequence of a marker gene according to the invention.

The diagnostic kit according to the disclosure may further comprise any reagent or media needed in order to perform the required analyses, such as PCR analyses, such as methylation-specific polymerase chain reaction (MSP) sequence analyses, bisulphite treatment, bisulphite sequencing, electrophoresis, pyrosequencing, mass spectrometry and sequence analyses by restriction digestion, quantitative and/or qualitative methylation, pyrosequencing, Southern blotting, restriction landmark genome scanning (RLGS), single nucleotide primer extension, CpG island microarray, SNUPE, COBRA, mass spectrometry, by use of methylation specific restriction enzymes or by measuring the expression level of said genes. In particular, the kit may further comprise one or more components selected from the group consisting of: deoxyribonucleoside triphosphates, buffers, stabilizers, thermostable DNA polymerases, restriction endonucleases (including methylation specific endonucleases), and labels (including fluorescent, chemiluminescent and radioactive labels). The diagnostic assay according to the invention may further comprise one or more reagents required for isolation of DNA.

When an object according to the present invention or one of its features or characteristics is referred to in singular this also refers to the object or its features or characteristics in plural. As an example, when referring to "a polypeptide" it is to be understood as referring to one or more polypeptides.

Throughout the present specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention will now be described in further details in the following non-limiting examples.

## Examples

Example 1:

*Bisulphite treatment and methylation-specific PCR*

DNA from cell lines and colorectal carcinomas was bisulphite treated as previously described (Grunau et al. and Fraga et al.). Whereas DNA from the adenomas was bisulphite treated according to the protocol of the CpGenome™ DNA modification kit (Intergen Boston, MA) (Smith-Sørensen et al.). The promoter methylation status of MAL, C3orf14, FBN1, MEF2c, CNRIP1, SPG20, UBE3A, SNCA, BEX and INA was subsequently analyzed by methylation-specific PCR (MSP), a method allowing for distinction between unmethylated and methylated alleles (Herman et al. and Derks et al.). All primers were designed with MethPrimer (Li and Dahiya) or Methyl Primer Express (Applied Biosystems). Their sequences are listed in Table 1, along with the product fragment length, primer location, and annealing temperature for each PCR. The fragments were amplified using the HotStarTaq DNA Polymerase (QIAGEN Inc., Valencia, CA), and all results were confirmed with a second independent round of MSP.

Table 1: Primers for methylation-specific PCR

| Gene | Methylated sequence (forward/reverse primer sequence) | SEQ ID NO. | Unmethylated sequence (forward/reverse primer sequence) | SEQ ID NO. |
|---|---|---|---|---|
| *MAL* | TTCGGGTTTTTTTGTTTTTAATTC/<br>GAAAACCATAACGACGTACTAACGT | 37/38 | TTTTGGGTTTTTTTGTTTTTAATTT/<br>ACAAAAACCATAACAACATACTAACATC | 39/40 |
| *C3orf14* | GTAATTTAGATTTCGGAGGGC/<br>CGACCAAAAAAAACGAAAA | 41/42 | TTTGTAATTTAGATTTTGGAGGGT/<br>CCAACCAAAAAAAACAAAAACA | 43/44 |
| *FBN1* | GTATTTTTTTCGCGAGAAATC/<br>AATCGTAACCGCTACAACC | 45/46 | AAAGTATTTTTTTTGTGAGAAATT/<br>CCCAATCATAACCACTACAACC | 47/48 |
| *MEF2c* | GTTATTTTTAATTCGATCGGTC/<br>AAACCGCTCGAAAAAAAA | 49/50 | TTGGTTATTTTTAATTTGATTGGTT/<br>CCAAAACCACTCAAAAAAAAA | 51/52 |
| *CNRIP1* | TCGTTTTTTGGTATAGTGGTC/<br>CAAATCCGCGCAACTAAA | 53/54 | GTTTTGTTTTTTGGTATAGTGGTT/<br>CAAATCCACACAACTAAAAAC | 55/56 |
| *SPG20* | TGGAACGTTTTGGTTGTTAC/<br>TACCTCGAAAACTCCCTACG | 57/58 | GTGGAATGTTTTGGTTGTTAT/<br>TTACCTCAAAAACTCCCTACA | 59/60 |
| *UBE3A* | CGTTGTTTGTCGGGATATTC/<br>CCCGTCGTCTCCTATAATCA | 61/62 | GTGTTGTTTGTTGGGATATTT/<br>CCCCATCATCTCCTATAATCA | 63/64 |
| *SNCA* | CGGGTTGTAGCGTAGATTTC/<br>CGTCGAATAACCACTCCC | 65/66 | GTGTGGGTTGTAGTGTAGATTTT/<br>TCATCAAATAACCACTCCCAA | 67/68 |
| *BEX1* | AGTTAATTGGTCGTCGGTTC/<br>CGAATAACGACTACACCGAA | 69/70 | ATTAGTTAATTGGTTGTTGGTTT/<br>ACACAAATAACAACTACACCAAA | 71/72 |

(continued)

| Gene | Methylated sequence (forward/reverse primer sequence) | SEQ ID NO. | Unmethylated sequence (forward/reverse primer sequence) | SEQ ID NO. |
|---|---|---|---|---|
| *INA* | AGGAGTTTCGTTTTTAGCGC/ ACGACTTCAACGCGAACTAC | 73/74 | AGTAGGAGTTTTGTTTTTAGTGT/ ACAACTTCAACACAAACTACAAA | 75/76 |

*Bisulphite sequencing*

All fragments were amplified with the HotStarTaq DNA Polymerase and eluted from a 2% agarose gel by the MinElute™Gel Extraction kit (QIAGEN). The samples were subsequently sequenced using the dGTP BigDye Terminator Cycle Sequencing Ready Reaction kit (Applied Biosystems, Foster City, CA) in a 3730 Sequencer (Applied Biosystems). The approximate amount of methyl cytosine of each CpG site in the various fragments was calculated by comparing the peak height of the cytosine signal with the sum of the cytosine and thymine peak height signals, as previously described by Melki, et al.

Table 2: Primers for bisulphite sequencing.

| Gene | Primer sequence (forward/reverse) | SEQ ID NO. |
|---|---|---|
| *MAL* | GGGTTTTTTTGTTTTTAATT/ACCAAAAACCACTCACAAACTC | 77/78 |
| *C3orf14* | GGAGGGTAGATGATTTTGAGAA/CTTCCCCTTCCCCTAACTACTA | 79/80 |
| *FBN1* | AGGGGGTGTTATTTTTTTTTTTTT/CCCAATCCCTATCCCTACC | 81/82 |
| *MEF2c* | TTTTTGGAYGAGTTTGGTTATT/CCACCTAATTCAAACATACAACC | 83/84 |
| *CNRIP1* | TTTTAYGTAGTTGGTYGAGG/CTCCTTAAACTATAACCCCCCT | 85/86 |
| *SPG20* | ATTTAGTTTGAGTAGGTYGGTG/CTCCATCCTAACAATCCATAAA | 87/88 |
| *UBE3A* | GGGGGGTGTTTAGAGGG/CCTCCTACCAAAAACTACAAACC | 89/90 |
| *SNCA* | AGAAGGGGTTTAAGAGAGG/ACTATCCCCAAAAPAAACC | 91/92 |
| *BEX1* | ATTTGTGGGTTTTTAGATTGGA/CCAAAAAACCACTATATTCCCA | 93/94 |
| *INA* | GATGTAGATGGTTTTGTTTYGG/CAAACRAAAACCATCCCC | 95/96 |

When analyzed by methylation-specific polymerase chain reaction (MSP) analysis hypermethylation of MAL was observed in an exceptionally high frequency among malignant (83%, 40 of 48 carcinomas) as well as in benign large bowel tumours (73%, 43 of 59 adenomas) (Figure 1).

Example 2

*Methylation-specific polymerase chain reaction (MSP) was performed for genes: MAL, C3orf14, FBN1, SPG20, SNCA, BEX1, INA, CNRIP1, UBE3A, MEF2C*

For each sample (colon cancer cell lines, colorectal carcinomas, adenomas and normal mucosa), 1.3ug DNA was bisulphite treated using the EpiTect bisulphite kit (Qiagen Inc., Valencia, CA) following the manufacturers protocol. The modified DNA was eluted in 40ul eluation buffer (included in the kit). Since bisulphite modification leads to sequence differences, two pairs of primers were used to amplify each gene (see primer list in Example 1), one specific for unmethylated template and the other specific for methylated template. The 25 μl PCR mixture contained 1 × PCR buffer, 0.75ul bisulphite treated template, 1.5-2.0 mM $MgCl_2$, 20 pmol of each primer, 200 μM dNTP, and 0.625-1U HotStarTaq DNA Polymerase (Qiagen). Human placental DNA (Sigma Chemical Co, St. Louis, MO, USA) treated *in vitro* with SssI methyltransferase (New England Biolabs Inc., Beverly, MA, USA) was used as a positive control for the methylated MSP reaction, whereas DNA from normal lymphocytes was used as a positive control for unmethylated alleles. Water was used as a negative PCR control in both reactions.

The PCR program consisted of 15 min denaturation at 95°C, followed by 35 cycles of 30sek at 95°C, 30sek at annealing temperature, and 30sek at 72°C. A final elongation was performed at 72°C in 7minutes..

Annealing temperature and $MgCl_2$ content for the respective genes tested so far:
MAL: 56°C, 1.5mM $MgCl_2$
C3orf14: 53°C, 1.5mM $MgCl_2$
FBN1: 48°C, 1.7 mM $MgCl_2$ for unmethylated reaction and 2.0 for methylated reaction
SPG20: 56°C, 1.5mM $MgCl_2$
SNCA: 53°C, 1.5mM $MgCl_2$
BEX1: 51°C, 1.5mM $MgCl_2$
INA: 55°C, 1.5mM $MgCl_2$
CNRIP1: 52°C, 1.5mM $MgCl_2$

Results: Methylation of MAL, UBE3A, MEF2C, FBN1, C3orf14, BEX1; INA, SNCA, SPG20, and CNRIP1

The results obtained in methylation-specific polymerase chain reaction (MSP) are presented in table 3 below:

Table 3.

| | **MAL** | **UBE3A** | **MEF2C** | **FBN1** | **c3orf14** |
|---|---|---|---|---|---|
| **Colon cancer cell lines** | 19/20 (95%) | 0/20 (0%) | 4/20 (20%) | 18/20 (90%) | 18/20 (90%) |
| **Colorectal carcinomas** | 49/61 (80%) | | | 49/49 (82%) | 27/49 (55%) |
| **Adenomas** | 45/63 (71%) | | | 34/59 (58%) | 33/59 (56%) |
| **Mucosa (cancer normal)** | 2/21 (10%) | | | 2/21 (10%) | 9/21 (43%) |
| **Mucosa (normal normal)** | 1/23 (4%) | | | 1/19 (5%) | 5/21 (24%) |
| | **BEX1** | **INA** | **SNCA** | **SPG20** | **CNRIP1** |
| **Colon cancer cell lines** | 18/19 (95%) | 19/20 (95%) | 19/20 (95%) | 20/20 (100%) | 20/20 (100%) |
| **Colorectal carcinomas** | 45/49 (92%) | 33/48 (69%) | 37/48 (77%) | 44/49 (90%) | 45/48 (94%) |
| **Adenomas** | 52/58 (90%) | 31/59 (53%) | 42/61 (69%) | 48/58 (83%) | 53/59 (88%) |
| **Mucosa (cancer normal)** | 15/21 (71%) | 2/20 (10%) | 14/21 (67%) | | 9/21 (43%) |
| **Mucosa (normal normal)** | 9/22 (45%) | 0/21 (0%) | 2/21 (10%) | 1/20 (5%) | 0/21 (0%) |

In general, the marker genes analyzed here are methylated at an extremely high frequency in colorectal cancer cell lines and colorectal carcinomas while methylation frequency is low in normal mucosa from non-cancerous donors. These results confirm utility of the marker genes in the diagnosis of tumour in the aero-digestive system and in the monitoring of tumour development.

In particular, MAL is methylated in 1/23 (4%) normal mucosa samples from non-cancerous donors, in 2/21 (10%) of normal mucosa samples taken in distance from the primary tumour, in 45/63 (71%) of adenomas, in 49/61 (80%) of carcinomas and in 19/20 (95%) of colon cancer cell lines. It will be noted that the methylation frequencies observed for MAL deviate slightly from those seen in example 1. This deviation is primarily due to the fact that the panel of samples analysed has been expanded.

FBN1 and INA are also rarely methylated in normal mucosa samples from both non-cancerous donors and normal mucosa samples taken in distance from the primary tumour (1/19, 5% and 2/21, 10%, respectively for FBN1; (0/21, 0% and 2/20, 10%, respectively for INA). Simultaneously, both FBN1 and INA are frequently methylated in carcinomas (40/49, 82%, and 33/48, 69%, respectively) as well as in adenomas (37/59, 58% and (31/59, 53%, respectively). The low methylation frequencies among both cohorts of normal mucosa samples and the high methylation frequencies in both benign and malignant tumours indicate that FBN1 and INA are particularly promising for detection of tumors early in the development. Simultaneously, a test comprising of these two markers would most likely have a high specificity.

SNCA, SPG20 and CNRIP1 have in general higher methylation frequencies in carcinomas than the latter group (37/48 (77%), 44/49 (90%) and 45/48 (94%), respectively), as well as in adenomas (42/61 (69%), 48/58 (83%) and 52/59 (88%), respectively). By including these markers in a non-invasive test, the sensitivity is likely to increase. In addition to having low methylation frequencies in normal mucosa samples from non-cancerous donors, these markers have relatively high methylation frequencies in normal mucosa samples taken in distance from the primary tumour (14/21 (67%), (29%-90%) and 9/21 (43%), respectively). This may indicate a "field effect" around the tumour, where normal appearing cells in the close vicinity of the tumour also harbour methylation of these three genes. The presence of such a field effect could increase the sensitivity of a non-invasive fecal based test, as more cells harbouring methylation of SNCA, SPG20 and/or CNRIP1 would be shed into the lumen of the colon and excreted with the faeces.

Example 3

*Methylation of the marker genes in different samples*

DNA was purified from stool and MSP was performed for the following genes MAL, FBN1, CNRIP1, INA, SPG20 and SNCA as described in the above examples.

Purification of DNA from 10 stool samples were analysed for all six genes. The methylation status of the corresponding primary tumour was known from 4-5 of the corresponding tumours.

In addition in nine of the 10 patients from which the stool samples were obtained a blood samples was also taken and the results are compared in table 5 DNA was isolated from 250mg faeces using the QIAamp DNA stool kit (QIAGEN).

The results of methylation state in genes from different samples are presented in table 4 below:

| Tissue/gene | MAL | CNRIP1 | INA | FBN1 | SPG20 | SNCA |
|---|---|---|---|---|---|---|
| Stool | 0/3 | 1/4 | 2/6 | 0/4 | 2/5 | 3/8 |
| Blood | 3/9 | 8/9 | 3/9 | 0/9 | 6/9 | 9/9 |

With the exception of MAL and FBN1 methylation was detected in all markers in the samples from stool. In the blood samples methylation was detected in all genes except FBN1. In general the sample methylation frequency was high for all genes from blood samples. Particularly the sample methylation frequency in blood samples comprising SNCA and CNRIP1 was very high. These markers seem particularly well suited as cancer markers since they can be tested in a non-invasive procedure, such as in blood samples.

DNA was purified from blood (using a standard phenol/chloroform method) and MSP was performed for the following genes MAL, CNRIP1, INA, FBN1, SPG20 and SNCA as described in the above examples.

DNA was purified from 14 blood samples from patients with a corresponding primary tumour which was methylated from all six genes.

Table 5 expanded sample panel

| Tissue/gene | MAL | CNRIP1 | INA | FBN1 | SPG20 | SNCA |
|---|---|---|---|---|---|---|
| Blood | 4/13 | 11/13 | 6/13 | 3/13 | 12/13 | 12/13 |

This example confirms the high sample methylation frequency of the genes in the blood samples and especially CNRIP1 and SNCA is highly suitable markers for diagnosis and/or screening for cancer or development of cancer. Also SPG20 seem as a very promising marker for blood sample screening.

Example 4

*Tissue-specific sample methylation frequencies of INA, SNCA, CNRIP1, SPG20 or FBN1 in different cell lines*

For each sample (cell lines from breast, kidney, ovary, pancreas, prostate, uterus and gastric.) the DNA was bisulphite treated before MSP as described in example 2.
The results of sample methylation frequency of genes in different samples are presented in table 6 below:

| | INA | SNCA | CNRIP1 | SPG20 | FBN1 |
|---|---|---|---|---|---|
| Breast | 4/6 | 6/7 | 2/6 | 2/6 | 4/6 |
| Kidney | 2/4 | 1/4 | 0/4 | 0/4 | 0/4 |
| Ovary | 2/4 | 0/4 | 0/4 | 1/4 | 1/4 |
| Pancreas | 3/6 | 4/6 | 5/6 | 4/6 | 2/6 |
| Prostate | 1/1 | 1/1 | 0/1 | 0/1 | 0/1 |
| Uterus | 2/4 | 2/4 | 1/3 | 2/4 | 2/4 |
| Gastric | 3/3 | 3/3 | 3/3 | 3/3 | 3/3 |

The promoter methylation status of INA, SNCA, CNRIP1, SPG20 and FBN1 was analyzed with MSP. In all samples from gastric cell lines the tested genes were methylated and thus the methylation frequency was (100 %) for all genes tested. In general, INA was methylated in at least one sample from all the tested tissues. The highest sample methylation frequency for this gene was seen in cell lines from gastric, 3/3 (100%), breast 4/6 (66%) and prostate 1/1 (100%). For cell lines from all other tissues the sample methylation frequency was 50%. SNCA was methylated in cell lines from all tested tissues, except for ovary. The highest samples methylation frequency was in cell lines from gastric, 3/3 (100%), breast 6/7 (85%), pancreas 4/6 (66%) and prostate 1/1 (100%). For uterus the sample methylation frequency was 50%. The sample methylation frequency of CNRIP1 was high in gastric, 3/3 (100%), and pancreas (83%) where 5 of 6 samples were methylated. SPG20 was methylated in 4 out of 6 (66%) pancreatic cell lines and in 2 of 4 uterus cell lines (50%). FBN1 was methylated in 4 of 6 breast cancer cell lines and 2 of 4 (50 %) samples was methylated in cell lines from uterus.

In general all genes were methylated in samples from gastric cell lines thus the sample methylation frequency was high for all genes. In addition, all genes were methylated in breast cell lines although the frequencies were varying among the genes. Further, all genes were methylated in samples from pancreas and for all genes except FBN1 (33 %) the frequency was at or above 50 %.

This experiment clearly indicates that the genes according to the invention are methylated in cell lines from various cancer tissues and thus could be used as cancer markers for various cancers. It is obvious to the skilled artisan that each of the genes according to the invention may be combined differently dependent on the type of cancer to be detected. Thus the genes showing best results in breast cell lines would be selected as markers when detected breast cancer.

The result of tissue specific sample methylation frequency of MAL is listed in table 8.

Example 5

*Quantitative gene expression analyses were performed for the following genes: SPG20, INA and CNRIP1.*

Gene expression was measured in 6 colon cancer cell lines before and after treatment with epigenetic drugs. The relative expression levels of SPG20, INA and CNRIP1 in the colon cancer cell lines (n = 6) was measured. The expression levels are displayed as fold changes calculated from the deltadeltaCT method using the untreated sample as a calibrator. The mean expression of ACTB and GUSB was used as endogenous control.

TaqMan real-time fluorescence detection (Applied Biosystems, Foster city, CA) was used to quantify mRNA levels in the colon cancer cell lines, as previously described [ Gibson et al. and Heid et al.]. cDNA was generated from five μg total RNA using a High-Capacity cDNA Archive kit (Applied Biosystems), including random primers according to the manufacturers' protocol. cDNA from the genes of interest (SPG20, INA and CNRIP1) and the endogenous controls (*ACTB* and *GUSB*) were amplified separately by the 7900HT Sequence Detection System (Applied Biosystems) following the protocol recommended by supplier. All samples were analyzed in triplicates. The expression levels were calculated as fold changes using the deltadeltaCT method and the untreated sample as a calibrator. In order to adjust for the possibly variable amounts of cDNA input in each PCR, we normalized the expression quantity of the target genes with the housekeeping genes *ACTB* and *GUSB.*

For SPG20, INA, and CNRIP1, the gene expression was significantly up-regulated in the majority of initially methylated colon cancer cell lines after promoter demethylation induced by the combined treatment 5-aza-2'-deoxycytidine and trichostatin A (Figures 2, 3 and 4). For INA and CNRIP1 the combined treatment was more effective than the individual treatment with 5-aza-2'-deoxycytidine alone and trichostatin A alone. The combined treatment also increased SPG20 expression, however similar or higher reactivation could be achieved by 5-aza-2'-deoxycytidine treatment alone. Treatment with the deacetylase inhibitor trichostatin A alone did not increase the gene expression of neither SPG20, INA nor CNRIP1. The two doses of 5-aza-2'-deoxycytidine tested here (1uM and 10uM) gave comparable effects. This means that demethylation of cell lines can be achieved by culturing them in the presence of low doses of 5-aza-2'-deoxycytidine, which is an advantage considering the cytotoxicity of this drug.

There was a clear relationship between methylation status and expression of SPG20, INA and CNRIP1. Thus measuring the methylation state or level by e.g. MSP and combining the result with the expression level of the corresponding gene could increase the sensitivity and specificity of a method of the invention.

Example 6

*Hypermethylation of MAL*

Patients and cell lines DNA from 218 fresh-frozen samples was subjected to methylation analysis, including 65 colorectal carcinomas (36 micro satellite stable; MSS, and 29 with micro satellite instability; MSI) from 64 patients, 63 adenomas, median size 8 mm, range 5-50 mm (61 MSS and 2 MSI) from 52 patients, 21 normal mucosa samples from 21 colorectal cancer patients (taken from distant sites from the primary carcinoma), and another 23 normal colorectal mucosa samples from 22 cancer-free individuals, along with 20 colon cancer cell lines (11 MSS and 9 MSI), and 29 cancer cell lines from various tissues (breast, gastric, kidney, ovary, pancreas, prostate, and uterus; Table 9). The mean age at diagnosis was 70 years (range 33 to 92) for patients with carcinoma, 67 years (range 62 to 72) for persons with adenomas, 64 years (ranging from 24 to 89) for the first group of normal mucosa donors, and 54 years (ranging from 33 to 86) for the second group of normal mucosa donors. The colorectal carcinomas and normal samples from cancer patients were obtained from an unselected prospective series collected from seven hospitals located in the South-East region of Norway. The adenomas were obtained from individuals attending a population based sigmoidoscopic screening program for cotorectal cancer. The normal mucosa samples from cancer-free individuals were obtained from deceased persons, and the majority of the total set of normal samples (27/44) consisted of mucosa only, whereas the remaining samples were taken from the bowel wall. Additional clinico-pathological data for the current tumour series include gender and tumour location, as well as polyp size and total number of polyps per individual for the adenoma series.

All samples belong to approved research biobanks and are part of research projects approved according to national guidelines (Biobank; registered at the Norwegian Institute of Public Health. Projects: Regional Ethics Committee and National Data Inspectorate).

Six colon cancer cell lines, HCT15, HT29, SW48, SW480, RKO and LS1034 were subjected to treatment with the demethylating drug 5-aza-2'deoxycytidine (1 μM for 72 h and 10uM for 72 h), the histone deactetylase inhibitor trichostatin A (0.5 μM for 12 h) and a combination of both (1μM 5-aza-2'deoxycytidine for 72 h, 0.5 μM trichostatin A added the last 12 h).

*Bisulphite treatment and methylation-specific polymerase chain reaction (MSP)*

DNA from primary tumours and normal mucosa samples was bisulphite treated as previously described. DNA from colon cancer cell lines was bisulphite treated using the EpiTect bisulphite kit (Qiagen Inc., Valencia, CA, USA). The promoter methylation status of all genes was analyzed by methylation-specific polymerase chain reaction (MSP) using the HotStarTaq DNA polymerase (Qiagen). All results were confirmed with a second independent round of MSP. Human placental DNA (Sigma Chemical Co, St. Louis, MO, USA) treated *in vitro* with *Sss1* methyltransferase (New. England Biolabs Inc., Beverly, MA, USA) was used as a positive control for the methylated MSP reaction, whereas DNA from normal lymphocytes was used as a positive control for unmethylated alleles. Water was used as a negative control in both reactions. The primers were designed with MethPrimer and Methyl Primer Express and their sequences are listed in Table 7.

| Primer set | Sense primer/Antisense primer | Frg. Size bp | Annealing temp. | Fragment location | SEQ ID NO. |
|---|---|---|---|---|---|
| MAL MSP-M | TTCGGGTTTTTTTGTTTTTAATTC/ GAAAACCATAACGACGTACTAACGT | 139 | 56 | -71 to 68 | 37/38 |
| MAL MSP-U | TTTTGGGTTTTTTTTGTTTTTAATTT/ ACAAAAACCATAACAACATACTAACATC | 142 | 56 | -72 to 70 | 39/40 |
| MAL BS_A | GGGTTTTTTTGTTTTTAATT/ ACCAAAAACCACTCACAAACTC | 236 | 53 | -68 to 168 | 97/98 |
| MAL BS_B | GGAAAAATGAAGGAGATTTAAATTT/ AATAACCTAAACRCCCCC | 404 | 50 | -427 to -23 | 99/100 |

Abbreviations: MSP, methylation-specific polymerase chain reaction; BS, bisulphite sequencing; M, methylated-specific primers; U, unmethylated-specific primers; Frg. Size, fragment size; An. Temp, annealing temperature (in degrees celsius). Fragment location lists the start and end point (in base pairs) of each fragment relative to the transcription start point provided by NCBI (RefSeq ID NM_002371), http://www.ncbi.nlm.nih.gov/mapview/map/search_cg*.

*Bisulphite sequencing*

All colon cancer cell linens (n = 20) were subjected to direct bisulphite sequencing of the MAL promoter. Two fragments were amplified; fragment A, covering bases - 68 to 168 relative to the transcription start point (overlapping with our MSP product), and fragment B covering bases -427 to -23. Fragment A covered altogether 24 CpG sites and was amplified using the HotStarTaq DNA polymerase and 35 PCR cycles. Fragment B covered altogether 32 CpG sites and was amplified using the same polymerase and 36 PCR cycles. The primer sequences are listed in Table 8. Excess primer and nucleotides were removed by ExoSAP-IT treatment following the protocol of the manufacturer (GE Healthcare, USB Corporation, Ohio, USA). The purified products were subsequently sequenced using the dGTP BigDye Terminator Cycle Sequencing Ready Reaction kit (Applied Biosystems, Foster City, CA, USA) in an AB Prism 3730 sequencer (Applied Biosystems). The approximate amount of methyl cytosine of each CpG site was calculated by comparing the peak height of the cytosine signal with the sum of the cytosine and thymine peak height signals, as previously described. CpG sites with ratios ranging from 0 - 0.20 were classified as unmethylated, CpG sites within the range 0.21 - 0.80 were classified as partially methylated, and CpG sites ranging from 0.81 - 1.0 were classified as hypermethylated.

*cDNA preparation and real-time quantitative gene expression*

Total RNA was extracted from cell lines (n = 46), tumours (n = 16), and normal tissue (n = 3) using Trizol (Invitrogen, Carlsbad, CA, USA) and the RNA concentration was determined using ND-1000 Nanodrop (NanoDrop Technologies, Wilmington, DE, USA). For each sample, total RNA was converted to cDNA using a High-Capacity cDNA Archive kit (Applied Biosystems), including random primers. MAL (Hs00242749_m1 and Hs00360838_m1) and the endogenous controls *ACTB* (Hs99999903_m1) and *GUSB* (Hs99999908_m1) were amplified separately in 96 well fast plates following the recommended protocol (Applied Biosystems), and the real time quantitative gene expression was measured by the 7900HT Sequence Detection System (Applied Biosystems). All samples were analyzed in triplicate, and the median value was used for data analysis. The human universal reference RNA (containing a mixture of RNA from ten different cell lines; Stratagene) was used to generate a standard curve, and the resulting quantitative expression levels of MAL were normalized against the mean value of the two endogenous controls.

*Tissue microarray*

For *in situ* detection of protein expression in colorectal cancers, a tissue microarray (TMA) was constructed, based on the technology previously described Embedded in the TMA are 292 cylindrical tissue cores (0.6 mm in diameter) from ethanol-fixed and paraffin embedded tumour samples derived from 281 individuals. Samples from the same patient series has been examined for various biological variables and clinical end-points. In addition, the array contains normal tissues from kidney, liver, spleen, and heart as controls. Ethanol-fixed normal colon tissues from four persons with no known history of colorectal cancer were obtained separately.

*Immunohistochemical in situ protein expression analysis*

Five μm thick sections of the TMA blocks were transferred onto glass slides for immunohistochemical analyses. The sections were deparaffinized in a xylene bath for 10 minutes and rehydrated via a series of graded ethanol baths. Heat-induced epitope retrieval was performed by heating in a microwave oven at full effect (850 W) for 5 minutes followed by 15 minutes at 100 W immersed in 10 mM citrate buffer at pH 6.0 containing 0.05% Tween-20. After cooling to room temperature, the immunohistochemical staining was performed according to the protocol of the DAKO Envision+™ K5007 kit (Dako, Glostrup, Denmark). The primary antibody, mouse clone 6D9 anti-MAL, was used at a dilution of 1:5000, which allowed for staining of kidney tubuli as positive control, while the heart muscle tissue remained unstained as negative control. The slides were counterstained with haematoxylin for 2 minutes and then dehydrated in increasing grades of ethanol and finally in xylene. Results from the immunohistochemistry were obtained by independent scoring by one of the authors and a reference pathologist.

*Statistics*

All P values were derived from two tailed statistical tests using the SPSS 13.0 software (SPSS, Chicago, IL, USA). Fisher's exact test was used to analyze 2 x 2 contingency tables. A 2 x 3 table and Chi-square test was used to analyze the potential association between quantitative gene expression of *MAL* and promoter methylation status. Samples were divided into two categories according to their gene expression levels: low expression included samples with gene expression equal to, or lower than, the median value across all cell lines or all tumours, high expression included samples with gene expression higher that the median. The methylation status was divided into three categories: unmethylated, partial methylation, and hypermethylated.

*Promoter methylation status of MAL in tissues and cell lines*

The promoter methylation status of *MAL* was analyzed with MSP (Figure 5). One of 23 (4%) normal mucosa samples from non-cancerous donors and two of 21 (10%) normal mucosa samples taken in distance from the primary tumour were methylated but displayed only low-intensity band compared with the positive control after gel electrophoresis. Forty-five of 63 (71%) adenomas and 49/61 (80%) carcinomas showed promoter hypermethylation. Nineteen of twenty colon cancer cell lines (95%), and 15/26 (58%) cancer cell lines from various tissues (breast, kidney, ovary, pancreas, prostate, and uterus) were hypermethylated (Table 9 lists tissue-specific frequencies).

Table 8. Promoter methylation status of *MAL* in cell lines of various tissues.

| Cell line | Tissue | Promoter methylation status | Methylation frequency |
|---|---|---|---|
| BT-20 | Breast | M | |
| BT-474 | Breast | U/M | |
| Hs 578T | Breast | U | |
| SK-BR-3 | Breast | U | 57% |
| T-47D | Breast | U/M | |
| ZR-75-1 | Breast | U | |
| ZR-75-38 | Breast | M | |
| Co115 | Colon | M | |
| HCT15 | Colon | M | |
| HCT116 | Colon | M | |
| LoVo | Colon | M | |
| LS174T | Colon | M | |
| RKO | Colon | M | |
| SW48 | Colon | M | |
| TC7 | Colon | M | |
| TC71 | Colon | M | |
| ALA | Colon | M | 95% |
| Colo320 | Colon | M | |
| EB | Colon | M | |
| FRI | Colon | U/M | |
| HT29 | Colon | M | |
| IS1 | Colon | M | |
| IS2 | Colon | M | |
| IS3 | Colon | M | |
| LS1034 | Colon | M | |
| SW480 | Colon | M | |
| V9P | Colon | U | |
| ACHN | Kidney | U | |
| Caki-1 | Kidney | U | 50% |
| Caki-2 | Kidney | M | |
| 786-O | Kidney | U/M | |
| ES-2 | Ovary | U/M | |
| OV-90 | Ovary | U/M | 50% |
| Ovcar-3 | Ovary | U | |
| SK-OV-3 | Ovary | U | |
| AsPC-1 | Pancreas | M | |
| BxPC-3 | Pancreas | U | |
| CFPAC-1 | Pancreas | U | 67% |
| HPAF-II | Pancreas | M | |
| PaCa-2 | Pancreas | M | |
| Panc-1 | Pancreas | U/M | |
| LNCaP | Prostate | U | 0% |
| AN3 CA | Uterus | U/M | |
| HEC-1-A | Uterus | M | 75% |
| KLE | Uterus | U | |
| RL95-2 | Uterus | M | |

The promoter methylation status of the individual cell lines was assessed by methylation-specific polymerase chain reaction (MSP). The methylation frequency reflects the number of methylated (M and U/M) samples from each tissue. Abbreviations: U, unmethylated; M, methylated.

The hypermethylation frequency found in normal samples was significantly lower than in adenomas ($P < 0.0001$) and carcinomas ($P < 0.0001$). Hypermethylation of the *MAL* promoter was not associated with MSI status, gender, or age in neither malignant nor benign tumours. Among carcinomas, tumours with distal location in the bowel (left side and rectum) were more frequently hypermethylated than were tumours with proximal location, although not statistically significant ($P = 0.088$). Among adenomas, no significant association could be found between promoter methylation status of *MAL* and polyp size or number.

*Bisulphite sequencing verification of the promoter methylation status of MAL*

Two overlapping fragments of the *MAL* promoter were bisulphite sequenced in 20 colon cancer cell lines. The results are summarized in Figure 6, and representative raw data can be seen in Figure 7. A good association was seen between the methylation status, as assessed by MSP, and the bisulphite sequences of the overlapping fragment A. However, in fragment B there was poor association with the MSP data. For this fragment, which is located farther upstream relative to the transcription start point, several consecutive CpG sites were frequently unmethylated and/or partially methylated. This held true also in cell lines shown to be heavily methylated around the transcription start point (fragment A; Figure 6).

*Real-time quantitative gene expression*

The level of MAL mRNA expression in cell lines (n = 46), primary colorectal carcinomas (n = 16), and normal mucosa (n = 3) was assessed by quantitative real time PCR. There was a strong association between MAL promoter hypermethylation and reduced or lost gene expression among cell lines ($P = 0.041$; Figure 8). Furthermore, the gene expression of MAL was up-regulated in colon cancer cell lines after promoter demethylation induced by the combined treatment 5-aza-2'-deoxycytidine and trichostatin A (Figure 9). Treatment with the deacetylase inhibitor trichostatin A alone did not increase MAL expression, whereas treatment with the DNA demethylating 5-aza-2'-deoxycytidine led to high expression in HT29 cells, but more moderate levels in HCT15 cells (Figure 9). Among primary colorectal carcinomas, those harbouring promoter hypermethylation of MAL (n = 13) expressed somewhat lower levels of MAL mRNA compared with the unmethylated tumours (n = 3), although not statistically significant (Figure 8).

*MAL protein expression is lost in colorectal carcinomas*

To evaluate the immunohistochemistry analyses of MAL, kidney and heart muscle tissues were included as positive and negative controls, respectively (Figure 10 A-B) From the 231 scorable colorectal tissue cores, *i.e.* those containing malignant colorectal epithelial tissue, 198 were negative for MAL staining (Figure 10 C-D). Twenty-nine of these had positive staining in non-epithelial tissue components within the same tissue cores, mainly in neurons and blood vessels (not shown). In comparison, all the sections of normal colon tissue contained positive staining for MAL in the epithelial cells (Figure 10 E-F).

These experiments conclude that the *MAL* promoter close to the transcription start is hypermethylated in the vast majority of malignant, as well as in benign colbrectal tumours, in contrast to normal colon mucosa samples which are unmethylated, and we contend that *MAL* remains a promising diagnostic biomarker for early colorectal tumourigenesis. In addition, hypermethylated *MAL* was found in cancer cell lines from breast, kidney, ovary, and uterus.

Hypermethylation of MAL has, by quantitative methylation-specific polymerase chain reaction (MSP), previously been shown by others to be present only in a small fraction (6%, 2/34) of colon carcinomas (Mori et al.). In contrast, the applicants demonstrate here a significantly higher methylation frequency of MAL in both benign and malignant colorectal tumours (71% in adenomas and 80% in carcinomas). The discrepancy in methylation frequencies between the present report and the previous study by Mori et al. is probably a consequence of study design. From direct bisulphite sequencing of colon cancer cell lines, we have now shown that the DNA methylation of MAL is unequally distributed within the CpG islands of its promoter (Figure 6). CpG islands often span more than one kilobase of the gene promoter, and the methylation status within this region is sometimes mistakenly assumed to be equally distributed. Since the results of an MSP analysis rely on the match or mismatch of the unmethylated and methylated primer sequences to bisulphite treated DNA, one should ensure that the primers anneal to relevant CpG sites in the gene promoter. In the present study, the applicants designed the MSP primers close to the transcription start point of the gene (-72 to +70) and found, by bisulphite sequencing, concordance between the overall methylation status of *MAL* as assessed by MSP and the methylation status of the individual CpG sites covered by our MSP primer set (Figure 6). This part of the CpG island was hypermethylated in the majority of colon cancer cell lines (95%). We also found that these cell lines, as well as those of other tissues, showed loss of MAL RNA expression from quantitative real time analyses, and that removal of DNA hypermethylation by the combined treatment of 5-aza-2'-deoxycytidine and Trichostatin A re-induced the expression of MAL in colon cancer cell lines (Figure 9). Furthermore, by analyzing a large series of clinically representative samples by protein immunohistochemistry we confirmed that the expression of MAL was lost in malignant colorectal epithelial cells as compared to normal mucosa.

The inventors have further analyzed the same region of the MAL promoter as Mori *et al.,* which is located -206 to -126 base pairs upstream of the transcription start point By direct bisulphite sequencing, we showed that only a minority of the CpG sites covered by the Mori antisense primer were methylated in the 19 colon cancer cell lines that were heavily methylated around the transcription start point (Figure 6). We therefore conclude that the very low (six percent) methylation frequency initially reported for MAL in colon carcinomas (Mori et al.) is most likely a consequence of the primer design and choice of CpG sites to be examined.

Inactivating hypermethylation of the MAL promoter might be prevalent also in other cancer types. In the present study, hypermethylated MAL was found in cancer cell lines from breast, kidney, ovary, and uterus.

The present analyses of cancer cell lines from seven tissues indicate that the hypermethylation of a limited area in the proximity of the transcription start point of MAL is associated with reduced or lost gene expression.

A sensitive non-invasive screening approach for colorectal cancer could markedly improve the clinical outcome for the patient. Such a diagnostic test could in principle measure the status of a single biomarker.

Hypermethylation of the MAL promoter represents a frequently hypermethylated gene among pre-malignant colorectal lesions, and is accompanied by low methylation frequencies in normal colon mucosa. The presence of such epigenetic changes in pre-malignant tissues might also have implications for cancer chemoprevention. By inhibiting or reversing these epigenetic alterations, the progression to a malignant phenotype might be prevented (Kopelovich et al.).

Promoter hypermethylation of MAL remains one of the most promising diagnostic biomarkers for early detection of colorectal tumours.

**References**

1. Mori Y, Cai K, Cheng Y, Wang S, Paun B, Hamilton JP, Jin Z, Sato F, Berki AT, Kan T, Ito T, Mantzur C, Abraham JM, Meltzer SJ. A genome-wide search identifies epigenetic silencing of somatostatin, tachykinin-1, and 5 other genes in colon cancer. Gastroenterology 2006;131:797-808.

2. Lind GE, Kleivi K, Meling GI, Teixeira MR, Thiis-Evensen E, Rognum TO, Lothe RA. ADAMTS1, CRABP1, and NR3C1 Identified as Epigenetically Deregulated genes in Colorectal Tumourigenesis. Cell Oncol 2006;28:259-272.

3. Laird PW. The power and the promise of DNA methylation markers. Nat Rev Cancer 2003;3:253-266.

4. Muller HM, Oberwalder M, Fiegl H, Morandell M, Goebel G, Zitt M, Muhlthaler M, Ofner D, Margreiter R, Widschwendter M. Methylation changes in faecal DNA: a marker for colorectal cancer screening? Lancet 2004;363:1283-1285.

5. Chen WD, Han ZJ, Skoletsky J, Olson J, Sah J, Myeroff L, Platzer P, Lu S, Dawson D, Willis J, Pretlow TP, Lutterbaugh J, Kasturi L, Willson JK, Rao JS, Shuber A, Markowitz SD. Detection in fecal DNA of colon cancer-specific methylation of the nonexpressed vimentin gene. J Natl Cancer Inst 2005;97:1124-1132.

6. C.Grunau, S.J.Clark and A.Rosenthal, Bisulfite genomic sequencing: systematic investigation of critical experimental parameters, Nucleic Acids Res. 29 (2001), E65.

7. M.F.Fraga and M.Esteller, DNA methylation: a profile of methods and applications, Biotechniques 33 (2002), 632-649.

8. B.Smith-Sørensen, G.E.Lind, R.I.Skotheim, S.D.Fossa, Ø.Fodstad, A.E.Stenwig, K.S.Jakobsen and R.A.Lothe, Frequent promoter hypermethylation of the 06-Methylguanine-DNA Methyltransferase (MGMT) gene in testicular cancer, Oncogene 21 (2002), 8878-8884.

9. J.G.Herman, J.R.Graff, S.Myöhänen, B.D.Nelkin and S.B.Baylin, Methylation-specific PCR: a novel PCR assay for methylation status of CpG islands, Proc. Natl. Acad. Sci. U. S. A. 93 (1996), 9821-9826.

10. S.Derks, M.H.Lentjes, D.M.Hellebrekers, A.P.de Bruine, J.G.Herman and E.M.van, Methylation-specific PCR unraveled, Cell Oncol. 26 (2004), 291-299.

11. L.C.Li and R.Dahiya, MethPrimer: designing primers for methylation PCRs, Bioinformatics. 18 (2002), 1427-1431.

12. J.R.Melki, P.C.Vincent and S.J.Clark, Concurrent DNA hypermethylation of multiple genes in acute myeloid leukemia, Cancer Res. 59 (1999), 3730-3740.

13. Zweig, M. H., and Campbell, G., Clin. Chem. 39 (1993) 561-577

**Items**

Item 1. A method for determining whether a subject has developed, is developing or is predisposed for developing cancer, or whether a subject is relapsing after treatment of cancer, comprising the step of:

a) determining the methylation level, the number of methylated CpG sites or the methylation state of CpG sites in a nucleic acid sequence in the promoter region, first exon or intron, of at least one gene in a sample, obtained from said subject, wherein said gene is selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Item 2. The method according to item 1, said method further comprising the steps of:

b) comparing the methylation level, the number of methylated CpG sites or the methylation state of CpG sites to a reference; and

c) identifying said subject as being likely to develop, being developing or being predisposed for developing cancer, or relapsing after treatment of cancer, if the methylation level, the number of methylated CpG sites or the methylation state of CpG sites is higher than the methylation level, the number of methylated CpG sites or the methylation state of CpG sites of the reference and identifying a subject as unlikely to develop, being developing or being predisposed for developing cancer, or relapsing after treatment of cancer, if the methylation level, the number of methylated CpG sites or the methylation state of CpG sites is below the methylation level, the number of methylated CpG sites or the methylation state of CpG sites of said reference.

Item 3. A method for determining whether a subject has developed, is developing or is predisposed for developing cancer, or whether a subject is relapsing after treatment of cancer, comprising the step of;

a) determining the methylation level, the number of methylated CpG sites or the methylation state of CpG sites in a sample from a subject

b) constructing a percentile plot of the methylation level, the number of methylated CpG sites or the methylation state of CpG sites of said at least one gene obtained from a sample from a healthy population;

c) constructing a ROC (receiver operating characteristics) curve based on the methylation level, the number of methylated CpG sites or the methylation state of CpG sites determined in the healthy population and on the methylation level, the number of methylated CpG sites or the methylation state of CpG sites determined in a population with cancer;

d) selecting from the ROC-curve the desired combination of sensitivity and specificity

e) determining from the percentile plot the methylation level, the number of methylated CpG sites or the methylation state of CpG sites corresponding to the determined or chosen specificity; and

f) predicting that the subject is likely to have cancer, if methylation level, the number of methylated CpG sites or the methylation state of CpG sites of said at least one gene in the sample is equal to or higher than said methylation level, the number of methylated CpG sites or the methylation state of CpG sites corresponding to the desired combination of sensitivity/specificity, and predicting that the subject is unlikely or not to have cancer, if the methylation level, the number of methylated CpG sites or the methylation state of CpG sites in the sample is lower than said methylation level, the number of methylated CpG sites or the methylation state of CpG sites corresponding to the desired combination of sensitivity/specificity.

Item 4. A method according to any of the proceeding items, where the methylation level, the number of methylated CpG sites or the methylation state of CpG sites of at least one additional marker is determined.

Item 5. The method according to item 4 wherein said at least one additional marker is selected from the group consisting of:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118
MAL, e.g. as identified by ensembl gene id ENSG00000172005

Item 6. The method according to item 4, comprising determining the methylation level, the number of methylated CpG sites or the methylation state of CpG sites of CNRIP1 combined with determining the methylation level, the number of methylated CpG sites or the methylation state of CpG sites of at least one additional marker selected from the group comprising:

SPG20, e.g. as identified by ensembl gene id ENSG00000133104, entrez id 23111 "
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Item 7. The method according to item 4, comprising determining the methylation level, the number of methylated CpG sites or the methylation state of CpG sites of SPG20 combined with determining the methylation level, the number of methylated CpG sites or the methylation state of CpG sites of at least one additional marker selected from the group comprising:

CNRIP1, e.g. as identified by ensembl gene id ENSG00000119865, Entrez id 25927
FBN1, e.g. as identified by ensembl gene id ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Item 8. The method according to item 4, comprising determining the methylation level, the number of methylated CpG sites or the methylation state of CpG sites of FBN1 combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
SPG20, e.g. as identified by ensembl gene id ENSG00000133104, entrez id 23111
SNCA, e.g. as identified by ensembl gene id ENSG00000145335, entrez id 6622; and
INA, e.g. as identified by ensembl gene id ENSG00000148798, entrez id 9118

Item 9. The method according to item 4, comprising determining methylation level, the number of methylated CpG sites or the methylation state of CpG sites of SNCA combined with determining the methylation level, the number of methylated CpG sites or the methylation state of CpG sites of at least one additional marker selected from the group comprising:

CNRIP1, e.g. as identified by ensembl gene id ENSG00000119865, entrez id 25927
SPG20, e.g. as identified by ensembl gene id ENSG00000133104, entrez id 23111

FBN1, e.g. as identified by ensembl gene id ENSG00000166147, entrez id 2200; and
INA, e.g. as identified by ensembl gene id ENSG00000148798, entrez id 9118

Item 10. The method according to item 4, comprising determining methylation level, the number of methylated CpG sites or the methylation state of CpG sites of INA combined with determining methylation level, the number of methylated CpG sites or the methylation state of CpG sites of at least one additional marker selected from the group comprising:

CNRIP1, e.g. as identified by ensembl gene id ENSG00000119865, entrez id 25927;
SPG20, e.g. as identified by ensembl gene id ENSG00000133104, entrez id 23111;
FBN1, e.g. as identified by ensembl gene id ENSG00000166147, entrez id 2200; and
SNCA, e.g. as identified by ensembl gene id ENSG00000145335, entrez id 6622

Item 11. The method according to item 10 wherein the methylation level, the number of methylated CpG sites or the methylation state of CpG sites is determined by bisulphite sequencing, quantitative and/or qualitative methylation specific polymerase chain reaction (MSP), pyrosequencing, Southern blotting, restriction landmark genome scanning (RLGS), single nucleotide primer extension, CpG island microarray, SNUPE, COBRA, mass spectrometry, by use of methylation specific restriction enzymes, by measuring the expression level of said genes or a combination thereof.

Item 12. The method according to item 11, wherein said methylation specific PCR comprises the use of nucleic acid primers which are capable of hybridizing to a nucleic acid sequence comprising 2 CpG sites and a cytosine residue which is not within a CpG site.

Item 13. The method according to any of the preceding items, wherein said cancer is selected from the group consisting of: colorectal tumours, lung tumours (including small cell lung cancer and/or non-small cell lung cancer), esophageal tumours, gastric tumours, pancreas tumours, liver tumours, tumours of the gall bladder and/or bile duct, tumours of the small bowel and tumours of the large bowel.

Item 14. The method according to any of the preceding items, wherein sample is obtained from blood, stool, urine, pleural fluid, gall, bronchial fluid, oral washings, tissue biopsies, ascites, pus, cerebrospinal fluid, aspirate, follicular fluid, tissue or mucus.

Item 15. A method according to any of the preceding items comprising determining methylation level, the number of methylated CpG sites or the methylation state of CpG sites in a nucleic acid sequence comprising a sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), B) or C).

Item 16. A diagnostic kit for the determination cancer comprising one or more oligonucleotide primers or one or more sets of oligonucleotide primers, which are each complementary to a nucleic acid sequence of the genes selected from:

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id.ENSG00000166147, entrez id 2200
SNCA, e.g. as identified by ensembl gene id ENSG00000145335, entrez id 6622; and
INA, e.g. as identified by ensembl gene id ENSG00000148798, entrez id 9118

Item 17. The use of one or more genes selected from the group comprising of

CNRIP1, e.g. as identified by ensembl gene id ENSG00000119865, entrez id 25927
SPG20, e.g. as identified by ensembl gene id ENSG00000133104, entrez id 23111
FBN1, e.g. as identified by ensembl gene id ENSG00000166147, entrez id 2200
SNCA, e.g. as identified by ensembl gene id ENSG00000145335, entrez id 6622; and

INA, e.g. as identified by ensembl gene id ENSG00000148798, entrez id 9118

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer.

Item 18. The use of a nucleic acid sequence, wherein said nucleic acid comprises a nucleic acid sequence selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in A);
C) A sub-sequence of a nucleic acid sequence as defined in A) or B);
D) A nucleic acid sequence which is at least 75% identical to a sequence as defined in A), A) or C).

in a diagnostic assay wherein the methylation level, the number of methylated CpG sites or the methylation state of CpG sites is assessed as an indicator of whether a subject has developed, is developing or is predisposed for developing cancer, or whether a subject is relapsing after treatment of cancer.

Item 19. An antibody recognizing a methylated nucleic acid sequences selected from the group consisting of:

A) A nucleic acid sequence as defined by any of SEQ ID NO.: 6, SEQ ID NO.: 7, SEQ ID NO.: 9, SEQ ID NO.: 13, SEQ ID NO.: 14 and SEQ ID NO.: 16;
B) A nucleic acid sequence which is complementary to a sequence as defined in a);
C) A sub-sequence of a nucleic acid sequence as defined in a) or b);
D) A nucleic acid sequence which is at least 75% identical to a sequence as defined in A), B) or C).

SEQUENCE LISTING

<110> Rikshospitalet-Radiumhospitalet HF Lothe, Ragnhild A. Lind, Guro E. Ahlquist, Terje Skotheim, Rolf I

<120> New Markers For Cancer

<130> 42139pc01

<150> PA 2007 00273 <151> 2007-02-27

<150> PA 2007 00601 <151> 2007-04-24

<160> 100

<170> PatentIn version 3.5

<210> 1
<211> 1300
<212> DNA
<213> Homo Sapiens

<400> 1

```
gactcgggcg gatttcaggc ttcagtgttt gtaggaggaa acacagcaat cacactatta      60
atagtaaatt aaaataaatg ggcaactgct gcatggtaat actttttttt ttaaggcaaa     120
aaataaaaaa tagtgaaaca gagaaacaaa acatgaaaca ccggcagtca acaggcaggc     180
aaagaacctg gggtggggg tagcagcggt cccaccctca aaaggcccgg gctgcccaga      240
ccaagagaaa gcgatgaatc tcttctggta acgtcccttc ctgtcgcatg gattcaaggc     300
cgacctgccc cagcaccacc accagcagcc ttctgctggg gccggcacag ctgggagcaa     360
cctcctactc tcaggcagac gcgcagcacc aagcagagag gcccggtgca ggatcccagc     420
gccgaaccag cgccggctca gtggacgcgg aaggggccgg cggccgcggc cggtcccatc     480
ccccactgca gaccccagc ctgtggcggt ggtccagttc cgccaggaaa ccgccgcctg      540
gagctgtggg tcgcgcacat taacgcatcc agcggaaaaa tgaaggagac ccaaattcaa     600
agttaaagta atggtgaccc gagaggtgcc ttgatgagaa ggtttggggt cccggttact     660
gatggttatc attcttacga gatgctggtc acctacgaag ggagaaaggc acgaggagcg     720
cctgaccaaa gtggttttgc cctgcttccc gcaagaggtg gcacccacgg ctggaacgca     780
ggagtcagac ccacagtccc cagctctgga cgcccgcagc ggggcctcga agaggttcag     840
ggcggtgccc gcggcgctcg ggccgggtct cccggggcgt ggggcggggg gcggggttgg     900
gcggcggccg gggctcctcc ctcttctgcc ccgggctccc ctgctcttaa cccgcgcgcg     960
ggggcgccca ggccactggg ctccgcggag ccagcgagag gtctgcgcgg agtctgagcg    1020
gcgctcgtcc cgtcccaagg ccgacgccag cacgccgtca tggccccgc agcggcgacg     1080
gggggcagca ccctgcccag tggcttctcg gtcttcacca ccttgcccga cttgctcttc    1140

atctttgagt ttgtgagtgg ctcctggccg gggaagggac ggggtgggct gagccgtgcg    1200
ctctctcggg cgcccagcac agctgtcgga cgggatccgc tagctgcgca ggttctggga    1260
gcatcggggc agcaggcgca gggcggggac taagccaggg                          1300
```

<210> 2
<211> 1300
<212> DNA
<213> Homo Sapiens

<400> 2

gactcgggcg gatttcaggc ttcagtgttt gtaggaggaa acacagcaat cacactatta 60
atagtaaatt aaaataaatg ggcaactgct gcatggtaat actttttttt ttaaggcaaa 120
aaataaaaaa tagtgaaaca gagaaacaaa acatgaaaca ccggcagtca acaggcaggc 180
aaagaacctg gggtgggggg tagcagcggt cccaccctca aaaggcccgg gctgcccaga 240
ccaagagaaa gcgatgaatc tcttctggta acgtcccttc ctgtcgcatg gattcaaggc 300
cgacctgccc cagcaccacc accagcagcc ttctgctggg gccggcacag ctgggagcaa 360
cctcctactc tcaggcagac gcgcagcacc aagcagagag gcccggtgca ggatcccagc 420
gccgaaccag cgccggctca gtggacgcgg aaggggccgg cggccgcggc cggtcccatc 480
ccccactgca gaccccccagc ctgtggcggt ggtccagttc cgccaggaaa ccgccgcctg 540
gagctgtggg tcgcgcacat taacgcatcc agcggaaaaa tgaaggagac ccaaattcaa 600
agttaaagta atggtgaccc gagaggtgcc ttgatgagaa ggtttggggt cccggttact 660
gatggttatc attcttacga gatgctggtc acctacgaag ggagaaaggc acgaggagcg 720
cctgaccaaa gtggttttgc cctgcttccc gcaagaggtg gcacccacgg ctggaacgca 780
ggagtcagac ccacagtccc cagctctgga cgcccgcagc ggggcctcga agaggttcag 840
ggcggtgccc gcggcgctcg ggccgggtct cccggggcgt ggggcggggg gcggggttgg 900
gcggcggccg gggctcctcc ctcttctgcc ccgggctccc ctgctcttaa cccgcgcgcg 960
ggggcgccca ggccactggg ctccgcggag ccagcgagag gtctgcgcgg agtctgagcg 1020
gcgctcgtcc cgtcccaagg ccgacgccag cacgccgtca tggcccccgc agcggcgacg 1080
gggggcagca ccctgcccag tggcttctcg gtcttcacca ccttgcccga cttgctcttc 1140
atctttgagt ttgtgagtgg ctcctggccg gggaagggac ggggtgggct gagccgtgcg 1200
ctctctcggg cgcccagcac agctgtcgga cgggatccgc tagctgcgca ggttctggga 1260
gcatcggggc agcaggcgca gggcggggac taagccaggg 1300

<210> 3
<211> 1300
<212> DNA
<213> Homo Sapiens

<400> 3

gactcgggcg gatttcaggc ttcagtgttt gtaggaggaa acacagcaat cacactatta 60
atagtaaatt aaaataaatg ggcaactgct gcatggtaat actttttttt ttaaggcaaa 120
aaataaaaaa tagtgaaaca gagaaacaaa acatgaaaca ccggcagtca acaggcaggc 180
aaagaacctg ggggtggggg tagcagcggt cccaccctca aaaggcccgg gctgcccaga 240
ccaagagaaa gcgatgaatc tcttctggta acgtcccttc ctgtcgcatg gattcaaggc 300
cgacctgccc cagcaccacc accagcagcc ttctgctggg gccggcacag ctgggagcaa 360
cctcctactc tcaggcagac gcgcagcacc aagcagagag gcccggtgca ggatcccagc 420
gccgaaccag cgccggctca gtggacgcgg aaggggccgg cggccgcggc cggtcccatc 480
ccccactgca gacccccagc ctgtggcggt ggtccagttc cgccaggaaa ccgccgcctg 540
gagctgtggg tcgcgcacat taacgcatcc agcggaaaaa tgaaggagac ccaaattcaa 600
agttaaagta atggtgaccc gagaggtgcc ttgatgagaa ggtttggggt cccggttact 660
gatggttatc attcttacga gatgctggtc acctacgaag ggagaaaggc acgaggagcg 720
cctgaccaaa gtggttttgc cctgcttccc gcaagaggtg gcacccacgg ctggaacgca 780
ggagtcagac ccacagtccc cagctctgga cgcccgcagc ggggcctcga agaggttcag 840
ggcggtgccc gcggcgctcg ggccgggtct cccggggcgt ggggcggggg gcggggttgg 900
gcggcggccg gggctcctcc ctcttctgcc ccgggctccc ctgctcttaa cccgcgcgcg 960
ggggcgccca ggccactggg ctccgcggag ccagcgagag gtctgcgcgg agtctgagcg 1020
gcgctcgtcc cgtcccaagg ccgacgccag cacgccgtca tggcccccgc agcggcgacg 1080
gggggcagca ccctgcccag tggcttctcg gtcttcacca ccttgcccga cttgctcttc 1140
atctttgagt ttgtgagtgg ctcctggccg gggaagggac ggggtgggct gagccgtgcg 1200
ctctctcggg cgcccagcac agctgtcgga cgggatccgc tagctgcgca ggttctggga 1260
gcatcggggc agcaggcgca gggcggggac taagccaggg 1300

<210> 4
<211> 1300
<212> DNA
<213> Homo Sapiens

<400> 4

gactcgggcg gatttcaggc ttcagtgttt gtaggaggaa acacagcaat cacactatta 60
atagtaaatt aaaataaatg ggcaactgct gcatggtaat actttttttt ttaaggcaaa 120
aaataaaaaa tagtgaaaca gagaaacaaa acatgaaaca ccggcagtca acaggcaggc 180
aaagaacctg gggtggggg tagcagcggt cccaccctca aaaggcccgg gctgcccaga 240
ccaagagaaa gcgatgaatc tcttctggta acgtcccttc ctgtcgcatg gattcaaggc 300
cgacctgccc cagcaccacc accagcagcc ttctgctggg gccggcacag ctgggagcaa 360
cctcctactc tcaggcagac gcgcagcacc aagcagagag gcccggtgca ggatcccagc 420

gccgaaccag cgccggctca gtggacgcgg aaggggccgg cggccgcggc cggtcccatc 480
ccccactgca gacccccagc ctgtggcggt ggtccagttc cgccaggaaa ccgccgcctg 540
gagctgtggg tcgcgcacat taacgcatcc agcggaaaaa tgaaggagac ccaaattcaa 600
agttaaagta atggtgaccc gagaggtgcc ttgatgagaa ggtttggggt cccggttact 660
gatggttatc attcttacga gatgctggtc acctacgaag ggagaaaggc acgaggagcg 720
cctgaccaaa gtggttttgc cctgcttccc gcaagaggtg gcacccacgg ctggaacgca 780
ggagtcagac ccacagtccc cagctctgga cgcccgcagc ggggcctcga agaggttcag 840
ggcggtgccc gcggcgctcg ggccgggtct cccggggcgt ggggcggggg gcggggttgg 900
gcggcggccg gggctcctcc ctcttctgcc ccgggctccc ctgctcttaa cccgcgcgcg 960
ggggcgccca ggccactggg ctccgcggag ccagcgagag gtctgcgcgg agtctgagcg 1020
gcgctcgtcc cgtcccaagg ccgacgccag cacgccgtca tggcccccgc agcggcgacg 1080
gggggcagca ccctgcccag tggcttctcg gtcttcacca ccttgcccga cttgctcttc 1140
atctttgagt ttgtgagtgg ctcctggccg gggaagggac ggggtgggct gagccgtgcg 1200
ctctctcggg cgcccagcac agctgtcgga cgggatccgc tagctgcgca ggttctggga 1260
gcatcggggc agcaggcgca gggcggggac taagccaggg 1300

<210> 5
<211> 1300
<212> DNA
<213> Homo Sapiens

<400> 5

gcttcactct ctgaaactct ttcaacatat gcaggtctca ctttaaaagg tcttgaaatg 60
gagttcctac cagacggcct ttccctaggg ttcataaaga ccctttcaag ccaccctccc 120
gcgcgccgcg ttaccacacg cccacctcct ggttgccgcg actacacctc acgaactttt 180
cgcgccccaa ggcggccctt tataaagatg cggtgacgtc accgcgcgcc gcgcgtgcgc 240
aaatatggta acgtgaccac gccctggcct gcgctaaggc cccggcgtcg gcgcagtagc 300
tggtgccttc ccggaagggc tcagaggcgg gctcggtgag tggagtcgga cagtgggtgg 360
gtgccctgac tgcctgacgg ccgcgcaggg ccttgcagga ccgctgggga ggatgttgtt 420
aacgtaaaga gccaggctga tgaggaaggt tcatgagtcc tttgggtata agcgggagtt 480
ggggcggccg gagggcagat gactctgaga aggtgcggat cttcgattct aaggggagaa 540
tgtgatttgg ttttgtctaa agtgctgata gtgggcctca cagaaaagtt accatgaacc 600
gactttccgg gattcccttg gcgctgcatt aattggcctc gttgcaaaag gaaaccgaga 660
ggaaggcggc aagacttggc ctgcgggtga tgttagcgga aaatatctgt aatttagacc 720
ccggagggcg ggcctgtgag cgattgctgt ccgcggtgct gaaggaaagg cagccacgcc 780
cagaggagga aacctaatga gggcagggcg gtgtttccgt tttccttggc cgggttcagc 840

accctacact caataagcgt gggctttcta gcaggactgg acactcaggc ggtgtgtcaa 900
tctttagaca atctttatag agtgcataga tgtttaattt cataaaattt aggaaagctt 960
catttcagga aggaatttta ttcctctttt tctcccttag ggcaagcact ttaacctttt 1020
aagcccaacc agatgagttg cctgcagttt tggaggcctt cagagcattt cactagacct 1080
ctgtctgtgt cggtccagtg tctttagcca aggtgagctt gctcgtggtg tgaagtgtgc 1140
cacgaaaatg ggctttcctg cactccacaa atgagagtgc ttagcagcca ggggaagggg 1200
aaggtatcta tctaattgtg cttttcctcg cagttacaca tgaaaagtac tttcctattt 1260
agaagagaga tggtagaact gggcactctt ccaataaagg 1300

<210> 6
<211> 1300
<212> DNA
<213> Homo Sapiens

<400> 6

aggccttcct aaactgttac agggtgcagg catcattggg cattatccat ataaggatgc 60
cacaaatact ataccctttca aagccactaa attcactgtg ccgcgcatat cctggggaga 120
ggcagctgtg tgtgggggag ggcgagggga ccgggagggt gtaagtagag tcctccagat 180
gaaccccagt cttaatctgg gctgggcaag aggaccatcc cttcaccgct cccatcctac 240
atcctaggtt ccaacattta caggcccctg ggttggacac cgacttagag agagcaccct 300
ggattcaggc ctgggtggct tgaattgagc ctcagaagag ccgcgtctgg agtgggctct 360
cgacacccag ggcaagtggg ggcggcagag ccctctcctc ggtcggcaca gcagcctctg 420
ccgcggtccc ggcctgcgac gcgcccagtc ttagcctccc ggcctccggc gtctgctgag 480
tgtccggcgg gagaggcgca gggagcgcgc taccgggagg cgcgggcagc ggggactggt 540
tttctctcgg gccagggcct ccggggcaac cgtctccagc gcgcattctt ggtgcaggtg 600
gaacagcttt ctgctccggt agggcttcac ctatcgcggg agaggttaat ctcggatcta 660
aacctcgcag ccgcagagcg ggctaaaacc gctactccac ctcttcccat ttctccccctc 720
cccacctcaa gacaaaaagt cccaggccgg gcaggacctg atcacctctg cctcctccca 780
ctgcgctaat cctgcgagcg agaggccccg caccgaggcg gaggctggca aaggggagtg 840
gaaagggagg atggatgggg ccgggggtg gggtggtgat gagggcgacg aaggaggggg 900
tgtcattttc tttttctttc ttttttaaa aaaagtattt ctctcgcgag aaaccgctgc 960
gcggacgata cttgaagagg tggggaaagg aggggctgc gggagccgcg gcagagactg 1020
tgggtgccac aagcggacag gagccacagc tgggacagct gcgagcggag ccgagcagtg 1080
gctgtagcgg ccacgactgg gagcagccgc cgccgcctcc tcgggagtcg gagccgccgc 1140
ttctccagtg ggtgcagccg gggtcccgac ggggtcggg cggccaccgg ggctggagct 1200
gcggccacgg aggcttttgc gtttgcgccg cgccgagggc agggacaggg actggggtga 1260

ggggctgtcc cggaacgtcc acagctggcg ctggccctcc 1300

<210> 7
<211> 1300
<212> DNA
<213> Homo Sapiens

<400> 7

```
acatatatat tatatgtata gcttattatt tcttaaaaaa gagcagtcta acttgggtaa      60
acaaaaactg ccaaggaaac gcatccctac ccatttgttc tcactagccc acttcatttc     120
ccaggtgtgc tccctggctc tggtactatc catctgggtg gtgctgaagc aggtgctctc     180
tctgctccgg gaagccattc ccagtatagt cctcaccccc tgagtctccc ctcgcttcca     240
aaagtcagct gtctaccgac ctctgccacg aaaactgtct aatcacgctt aatatgcaaa     300
tctaaactct gcggtgtcgc atatggcccc ctgccaataa tcctttgacc catcccacct     360
cttggccatt ttgtcaccag aatgccttag gctcatgtca gttgcctttt acagggttaa     420
tagctcaccc ttcaaaatct gtgtaaatat atgtgattca ttacatatat ttatgtatgt     480
gtcatttttg ggacgagcat acgtataatg gaaatgtaca cccacaagaa gatgtagctc     540
cttgaatatg gttattttct tcctcttgca tccattcctc atccagcgtg gctccatcac     600
gtggaattgc gcctttcctc ggccatttcc cctaagcttt ggaaagactc atatttcctg     660
aaatttaaat caccggctag cggtttcctg tcccccttc ccgcctccct ttaccccccc     720
tcagcctcct cccgcctggc tggcgccgga tccgcgagca gcagtgtcca cctcacgtag     780
ctggccgagg cggtatccag attccggggg tctcgctcct tggcatagtg gtcgcttaac     840
tccagcgcct tcccagtgcc tcccaaacct ctcctcctcc tgcccggggc tgttctagga     900
ggagcctaaa tgtccgcttc cccagctgaa ggggattgtg gtgcgtaaac aggattaact     960
ccctcgcccc cagctgcgcg gatctgcgcg gcggcctgcg ggccagagcg agccttcggg    1020
tgcgtggagg gcgcagcact ggagcggggg atggggaagc gcgcggcctt ggagccgtct    1080
cttgggcccg cttcctcgcc gagtggggag gggggccaca gcctaaggag ctgctcctca    1140
aaagcccggg cggacggcgg cgacggcgcg ggttcctagc ggcagcctca gctccaccac    1200
ctccctcacc ctccccgcgc ttcctctccg cccctcgcgc tccagccact cggccgcagc    1260
cggcgctgtc ctccgccccc cggagccgcc gcgccagacc                          1300
```

<210> 8
<211> 1300
<212> DNA
<213> Homo Sapiens

<400> 8

```
cccgacttta gaaggacttt cctgctggct ggctcgcagc gtccccggag agcaagcgag      60
cgcgccgcag cgggagacta ggttctctcc gagcgtcctc cgcagaggcg ccgcgagagg     120
agcagggagc cgcagctcgc cgtgttgtct tcatttcgtg caataaagaa ttgtcattag     180
```

```
gtttgcgtat ggcatgtgct attaccccac cgtaaaacta aaattagcaa aatgtcagga     240
atggaaagat tgattcacca agatgcaatt atcatttaaa agtgcttgat tgaggtactg     300
atgttcagtg atttattctg cataccatat acataattaa agtagtgtag tggagtaatt     360
tatcaatcta gttgagactg gaggggtgag gagggagctg ctgtatgttt gttttattaa     420
aatgctccga ggtctagtcc cgcccccctt ttgcaagagt gaaactgatg atttctccag     480
ctcgcgagga aagagtcaac ggtttgggat tgtgggggag agagagacgg agagaaaaag     540
gcagcgcgaa gcaaaggcaa ggacaaaatt aaataaaggg ggaaaaaagg aggcaaaaga     600
catttcatcg gacgtgctgc ttagaacccc aaccattcgt gctccgtctt ccctaccacc     660
cccgcctccc ctcccagtat ccttcaatcc cccccgcccc caccaccccc agtctttttt     720
acgcgatgtt tcaaacgctg tgagctgttc tccttttccc attcgtcttc tgtcacttcc     780
ttcctggacg cagttttctg gacgagtctg gttactttta atccgaccgg ccgctgagag     840
ccactttctc ctcctcctcc tcctcctcct tctcttcctc ctccttcttc ctcctcctcc     900
tcctcttccg agcggcctcg gcgcgcgcga atgcgcggcc ccgcgccccc cccctcgcgc     960
gcgctcccct cgcgcgcgcg cacacacgca cacatcgtct ccagctctct gctcgctctg    1020
ctcgcagtca cagacacttg agcacacgcg tacacccaga catcttcggg ctgctattgg    1080
attgactttg aaggttctgt gtgggtcgcc gtggctgcat gtttgaatca ggtggagaag    1140
cacttcaacg ctggacgaag taaagattat tgttgttatt ttttttttct ctctctctct    1200
ctcttaagaa aggaaaatat cccaaggact aatctgatcg ggtcttcctt catgtaagta    1260
cccctgatat ttctcgagga aatagaaaac ccgggtattg                          1300
```

<210> 9
<211> 1300
<212> DNA
<213> Homo Sapiens

<400> 9

```
aacctgggaa gcagaggttg cagtgagccg agatcatgcc actgcactcc agcctgggca      60
acatagtaag actctgtctc aaaacaaaac aaaaacacaa caacaacaaa actggttcta     120
aatgctccgg gcaagtacat actgcttttt ctttttctg ttttttttc cctaaggcag     180
ccctattgaa gagtctgtca tctacaatat tttactaatt taaaaacact tgtcaagaag     240
ggtaaagtat gttcatcttg attttactct ttgtatcccg gaatcaggaa ctaacagttt     300
tatgatagtg atatagactt tacacacaca ccaagactta aaacacgtgg tgcaggcggg     360
cttttggaaa ttaactgtgc tctggataca gaagtcacaa agctcaggct gcagagacat     420
tgaggggata atcagccttt attgtctctc gtgcactgtg cccagcgctc cctctagcgg     480
ctacaagcgc tcactgccca cactctctac taggctctcc ggaggcctcg gtttcgctgg     540
agagaaagag attgcgcagg cgccgagcag ccgacgtggc cgcaggggac cgtgcctggg     600
ggggtctgcg cggggccgct gactcgcgag gttctctgtc gcctgccgcc ctcgctcctt     660
ttctcccctt ctagagtgtt acggtgactc ttgggtccga aggggacgga cgtcctccca     720
cgtaccattt agaaggtcag agtgggcaga acctagtctg agcaggccgg tgatgaatcc     780
gctggcaaga cgcgcgcgtg ggtactcacg tgacctaggc tgcggcggcg gcgtgctgcg     840
ggctctgtgg cgggagcgag gccgacgggc ggggccgcgc ggccgcgtga cgcgaagcgt     900
tcgagagcgc gcgtcgtgga acgtcttggt tgccacggca agcgcgcgcg cgaggccttg     960
ggaacctcgg gaccggcccc cggcgagcgc agcggcgccc agtgtaaggg agtgggagct    1020
ggtccgtgcc gcggcggccg cgcagggagc tctcgaggca acgccggggc gcccgaggtc    1080
tggaaggcgc aggtggggcg cgggggagtt cacccctgcg cggtcccttg ggtgggcggg    1140
gtgggcgccc caggccccgc ggactcggct gggaggctga gcgggcgacg gtcacgggac    1200
aagggtggag gggtgggga cctcatccga gtctcagaga tggaatttc gaggaaggga    1260
gcgatttcga gagagaatag tttgagaagt tgttctcagt                           1300
```

<210> 10
<211> 1300
<212> DNA
<213> Homo Sapiens

<400> 10

gttgctgatg ctgtttataa aagtgaatag atagggaaat actgattcat atctcgtgaa 60
atgaaaatga aaggcttttt agtgacttag aattttaaat atttctacat gagagagcag 120
tagtatattt agaaataaca aagtaactgg caactgttta aaactgaagt taattcacag 180
ctatccagtg caaaacttca cctcaggtga tacacttttg acaggtaata catacagtaa 240
gtgtattttt agggaaacag tttcattgtt gaaccaagat aatcatcatt agaatgttgt 300
atctgattta agtgtcttta aacttaccaa ggtattagat tttagtttga attgtctgga 360
gtagcggtag cggttggcac atttgttctt aaagggccag aaaataaata ttttaggctt 420
tatgggccat gtggtttctg ccacaagtcc tcagctctgt tcttttagtg tgaaagcagc 480
catagatgat acctaaatga atgagtatgg ctgcatccca ataaaacttc atttacaaaa 540
ctacatggct ggcctaagct ttagtctgtc tgcctaggga gtagtttact gagccactaa 600
tctaaagttt aatactgtga gtgaatacca gtgagtacct ttgttaatgt ggataaccaa 660
tacttggcta taggaagttt tttagttgtg tgttttatta cacgtatttg actttgtgaa 720
taattatggc ttataatggc ttgtctgttg gtatctatgt atagcgttta cagtttcctt 780
taaaaaacat gcattgagtt ttttaatagt ccaaccctta aaataaatgt gttgtatggc 840
cacctgatct gaccactttc tttcatgttg acatctttaa ttttaaaact gttttattta 900
gtgcttaaat cttgtttaca aaattgtctt cctaagtaat atgtctacct tttttttgg 960
aatatggaat attttgctaa ctgtttctca attgcatttt acagatcagg agaacctcag 1020

tctgacgaca ttgaagctag ccgaatgtaa gtgtaacttg gttgagactg tggttcttat 1080
tttgagttgc cctagactgc tttaaattac gtcacattat ttggaaataa tttctggtta 1140
aaagaaagga atcatttagc agtaaatggg agataggaac atacctactt tttttcctat 1200
cagataactc taaacctcgg taacagttta ctaggtttct actactagat agataaatgc 1260
acacgcctaa attcttagtc tttttgcttc cctggtagca 1300

<210> 11
<211> 1300
<212> DNA
<213> Homo Sapiens

<400> 11

caaaaaaatc catcccttga taggaagtca gtatatctgg gttttagtca gtccttcaac 60
actagttcca aaatcttggg caagtcagtt aatcactcgg aggctgctta attttttttt 120
tttttaattc ttagtatatt atctgtcttg attaggtaaa gccaaagttg ttataagact 180
caaggtacta ttgaccaaaa tgcttcagaa actataacat tacataaaag tacaaggaat 240
tattacattt tctcaaatga agagatcatt taagaaaaac aaaatgctgg aaaatagtcc 300
ttaaaaactt ttctataata attactcttc tgatgaattt tacattgaca cctaatttga 360
agctttgaat attatttttt gaacaatgaa ttgggtttat atattaaagt gttctaacca 420
aaggtttgtt taatttatta gagttattaa gcctacgctc agatcaaggt agcagctaga 480
ctggtgtgac aacctgtttt taatcagtga ctcaaagctg tgatcaccct gatgtcaccg 540
aatggccaca gcttgtaaaa ggtaattttg aattatttta cagcctttaa aaggctgtca 600
ttgtaaagtg aaatacatac tgtaaaaatg aagacaatgt atagttggca ggaatactgc 660
taagaatagt gggcctgaga gttgacctac ttcatttaat ctccttaaaa tattttagtg 720
tcttttttc tcattaaaaa tatcagtagc cactatcaaa gacctatctg tatgtataaa 780
caggaaagta tttctcaaaa aaacttcaag gttttaaaca ttttactata aacattatag 840
aaaacattaa aaatttctta atatgcattt taaagtatgc cttttatgtg gtgaacgttt 900
taactaaaca tttctctaga agtttttata acattaataa aactagtata cttttctctc 960
ctagagagtt acagtggagg taaaaggagt ggcttgcagg atggagaagc tgcaccagtg 1020
ttattggaag tgagccacca tttgaatttg ctagctcatg ctgcagtatt cagattagtg 1080
ggtgttttgt gatcatattt tctgcgattc aaatcaagac ctaaaattga ttctcattct 1140
gagatttaca ttttattatc caatgttatt tggttattgt ttcagtttta aaatttctga 1200
cttgttattc tgacacactt taggataacc taagtcaata gtttaataca aaaatttccc 1260
tttggttata tgttttgaat taggttatat gttctgcatt 1300

<210> 12
<211> 1300
<212> DNA
<213> Homo Sapiens

<400> 12

agtacttcaa gggcttatca acaaagttgc aagttgtaac actatgaatt gttagtgata 60
ctcttttggc tttgctagca agtgttgtaa agctatacac acacacacac acacacacac 120
acatacacac acacaccttt taaaatggtg accctggtac caaatatgac tttaaatgga 180
tttaatttta atggctttaa ctacgttcag ctgtcatatg gatcaaaatt agcctctatc 240
cagctggggt caaccaggga gccacttttc ttaaccgacg acctactgaa cgtcaacaac 300
tgcaggagac gggactttac cttcgtctct ggtaaactag ttgacacatc ctgtgttggc 360
aagaggccta agtagatgac cttggtcctc taaaatctgg cctgcactct cggggcaccc 420
ctgcaacatc tacaaaggca gctccagata gaaaagggtt ggggtcgaaa agccaataac 480
ggcaggcacc tgccccgcct cggggctggg gggctattcc agcggcttca gctaactttc 540
agagccattc gtttcccaac aaagtctgag gcgttcctct gctgggtaca ccaaggggct 600
ctgcaacccct cctggggggg ggggtgccca gagggcttcc ggaagtccca ggtttattct 660
ttcgggtcac agacagcaga aactaaaaag agggattacc ctttctgtcc agtcgcaaga 720
tggcgaccga gcctggtggg actccgaggg gccgcaggcc acctcctctt cccaatggcc 780
cgtgcgccgg cggcgacggc aagcgggagg gaggcggggc cggcgaagga aggaggggcg 840
gagcgcggcg ccctcccgcg cgtcttggcc ccgccccacg tccccgcgtc ccggcctgga 900
gccctcgccc ggccgggcgg cgcgcgctgc ctgccgggat actcggcccg cccagccagt 960
cctcccgtct tgcgccgcgg ccgcgagatc cgtgtgtctc ccaagatggt ggcgctgggc 1020
tcggggtgac tacaggagac gacggggcct tttcccttcg ccaggacccg acacaccagg 1080
cttcgctcgc tcgcgcaccc ctccgccgcg tagccatccg ccagcgcggg cgcccgccat 1140
ccgccgccta cttacgcttc acctctgccg acccggcgcg ctcggctgcg ggcggcggcg 1200
cctccttcgg ctcctcctcg gaatagctcg cggcctgtag ccccctggcag gagggcccct 1260
cagccccccg gtgtggacag gcagcggcgg ctggcgacga 1300

<210> 13
<211> 1300
<212> DNA
<213> Homo Sapiens

<400> 13

gcgaaaagcc ttaggaccgc ttgttttaga cggctgggga atatttattc cttgttccac 60
tgatgggaaa atcagcgtct ggcaggcgct gattggtgga aaggaaaatg gtgatagtgg 120
cgtggaaaga ggatttgctg agccttctcc tgcctcctca acctgtgact cttccttagt 180
agtctccctt tcaccctcag gaccctttcc ggctcttcct agattaagag caaacgaaaa 240
ccttgaagat atttgaacta aagcgacccc taacgttgta acctgtgacc gtgattaaat 300

ttcagcgatg cgagggcaaa gcgctctcgg cggtgcggtg tgagccacct cccggcgctg 360
cctgtctcct ccagcagctc cccaagggat aggctctgcc cttggtggtc gaccctcagg 420
ccctcggctc tcccagggcg actctgacga ggggtagggg gtggtccccg ggaggaccca 480
gaggaaaggc ggggacaaga agggaggggga aggggaaaga ggaagaggca tcatccctag 540
cccaaccgct cccgatctcc acaagagtgc tcgtgaccct aaacttaacg tgaggcgcaa 600
aagcgccccc actttcccgc cttgcgcggc caggcaggcg gctggagttg atggctcacc 660
ccgcgccccc tgccccatcc ccatccgaga tagggacgag gagcacgctg cagggaaagc 720
agcgagcgcc gggagagggg cgggcagaag cgctgacaaa tcagcggtgg gggcggagag 780
ccgaggagaa ggagaaggag gaggactagg aggaggagga cggcgacgac cagaaggggc 840
ccaagagagg gggcgagcga ccgagcgccg cgacgcggaa gtgaggtgcg tgcgggctgc 900
agcgcagacc ccggcccggc ccctccgaga gcgtcctggg cgctccctca cgccttgcct 960
tcaagccttc tgcctttcca ccctcgtgag cggagaactg ggagtggcca ttcgacgaca 1020
ggttagcggg tttgcctccc actcccccag cctcgcgtcg ccggctcaca gcggcctcct 1080
ctggggacag tccccccggg gtgccgcctc cgcccttcct gtgcgctcct tttccttctt 1140
ctttcctatt aaatattatt tgggaattgt ttaaattttt tttttaaaaa aagagagagg 1200
cggggaggag tcggagttgt ggagaagcag agggactcag gtaagtacct gtggatctaa 1260
acgggcgtct ttggaaatcc tggagaacgc cggatgggag 1300

<210> 14
<211> 1300
<212> DNA
<213> Homo Sapiens

<400> 14

```
gaatggtcgt gggcaccggg agggggtggt gctgccatga ggacccgctg ggccaggtct      60
ctgggaggtg agtacttgtc cctttgggga gcctaaggaa agagacttga cctggctttc     120
gtcctgcttc tgatattccc ttctccacaa gggctgagag attaggctgc ttctccggga     180
tccgcttttc cccgggaaac gcgaggatgc tccatggagc gtgagcatcc aacttttctc     240
tcacataaaa tctgtctgcc cgctctcttg gtttttctct gtaaagtaag caagctgcgt     300
ttggcaaata atgaaatgga agtgcaagga ggccaagtca acaggtggta acgggttaac     360
aagtgctggc gcggggtccg ctagggtgga ggctgagaac gccccctcgg gtggctggcg     420
cggggttgga gacggcccgc gagtgtgagc ggcgcctgct cagggtagat agctgagggc     480
gggggtggat gttggatgga ttagaaccat cacacttggg cctgctgttt gcctgagttt     540
gaaccacacc ccgagtgagc agttagttct gttgcctacg cctttccacc atcaacctgt     600
tagccttctt ctgggattca tgttaaggat acccctgacc ctaagcctcc agcttccatg     660
cttctaactc atactgttac cctttagacc ccgggaattt aaaaaagggg ttaatctttt     720

catgcaactc cacttctgaa atgcagtaat aacaactcag aggattcatc ctaatccgtg     780
gttaggtggc tagactttta ctagccaaga tggatgggag atgctaaatt tttaatgcca     840
gagctaaaaa tgtctgcttt gtccaatggt taaatgagtg tacacttaaa agagtctcac     900
actttggagg gtttctcatg atttttcagt gtttttgtt tatttttccc cgaaagttct     960
cattcaaagt gtattttatg ttttccagtg tggtgtaaag gaattcatta gccatggatg    1020
tattcatgaa aggactttca aaggccaagg agggagttgt ggctgctgct gagaaaacca    1080
aacagggtgt ggcagaagca gcaggaaaga caaaagaggg tgttctctat gtaggtaggt    1140
aaaccccaaa tgtcagtttg gtgcttgttc atgagtgatg ggttaggata atcaatactc    1200
taaatgctgg tagttctctc tcttgattca tttttgcatc attgcttgtc aaaaaggtgg    1260
actgagtcag aggtatgtgt aggtaggtga atgtgaacgt                          1300
```

<210> 15
<211> 1300
<212> DNA
<213> Homo Sapiens

<400> 15

```
gccaaagtga tatattaagg tcaccacctt ggtcacctgt ccatctatat ttttggagta      60
agcaacagaa ttaggagtct gaacatagga attaagaagc tctcgaatat aacagtggaa     120
gaccagcacc ttcccaacct cttgtcaggg cagggatact ctatctctgc gtcactcaac     180
ctgccttggc ctcacagctc ttccttttgc ttaagtcttc aaaccatcaa cagtgatagc     240
cactaatttt agcaggtggc attagttgtc agcaaatgtt ttttcatatt taatgaatgt     300
ttattataat agaataaaat agaaatgttt attaataaaa cttgaaattt gtcccagaag     360
gataaaaacg tagattattt acataaaatg gccaagatct gacaacagaa ctgtttcctg     420
aagatttact tcacagatta ttttgaaaac accaactgtg ttcaggaaaa ttttctctcc     480
tttttttcat aaaattttca aacaagtgag aatgctctaa aaatgatggt gtggagccta     540
gagaaaatgt ttctgtgtg ctatagatga acataaacta tatatatata ttactttccc     600
aggttgaagt gtttctttca aaaataaaat ttgattattt tcaatttttt gagggatgct     660
gatttttaaa aaggaaataa agagaaatta ttaggaagca tatacacagc cagtgagaag     720
aggaaagaag aaaaggccaa ggccagagga aggggtttg gggtgggga aaggaagggt       780
acagaccctc ccaaataacc gaagatgggt aaactccaac cgcaaagcga tgatgccact     840
tgtgggccct cagattggag aggacggaga tgagtgacgg ggctgtgcgc actggggcac     900
cgcgcacaga cgggaggagg ggggtgtctg tgtgttccaa agggggagt accagttaac      960
tggccgccgg cccgtggggg ttggtgagaa ggagggtgag cttggcggtg acgcacggcc    1020
ctcacgtgac cgggagctgc agagctacgc agccttcggt gcagtcgtca ctcgtgtctc    1080
gctaccagct ccccgctgcc ctgcgctcgg cgggctggca tccggcccgg gggaaagcgg    1140

accaggtaag ggccccgggg cccgcgcgcc ctggctaggc cagcagaggc tcaccgaccc    1200
ccccgacgag cgcagaacag cgcaggggat ccccgccagc tttctccaca ccccaacatt    1260
cttgggaaca tagtggtccc ctggggtggg gaagcagtgg                          1300
```

<210> 16
<211> 1300
<212> DNA
<213> Homo Sapiens

<400> 16

```
actgtagtga acaggagagc acacttctta tacaaatggc aagggcaact cacctctgcc      60
actgtgggca tcccagctct tcccattttc agaagaagcc agaaatccaa tttctttaca     120
ggtgaaatct cctggtttaa aaatgctcct ttcgaaatat tttaaaatat aatgctctaa     180
acacaaaagt ctgcagcccc ctttccagca gcttgtggtc ctggcctgtg ccatgcttta     240
gtgccctaac gatgagcccc agcaattccc agcgcctcct gcttctccaa gtctggcctc     300
ttttcccctc tgtaccgccg cttctcccca ctctccgcag ctcggggtca ctttctttac     360
tgatctcccc tctcttctgg gagaggcact tgctgaacat ctttccgccc ggagcctccc     420
caggagctgc agaggggcag ctggagagat gctttctgac gtagcatctc ccacctttct     480
ccttggggac cctgaggaag gggacaaggg ctcgctctgc aggtcctcac tgtaactgga     540
aaaacacgac ctcgccctcg ggaaggcttt ctgtgcgcct cacctcagga tgagggtggg     600
tgtaggggac acctcccaga aaccccctaac ctcccagtcg gttaaagaag aggggatagg    660
gtcaagggat gcgacagagc tgtgtggttt ccggatggga aacctcagtc gtttaggcac     720
ccctccgctc gagtcacttc cgaagcagtc gattcttggg gagaagcgct gcggaaaggg     780
gcgactccga tgcagatggc cctgtcccgg cgccccaggt cgtcgcgcgc gcagctgcgg     840
tagtcactgc gcctccccgc ccccactcct ggatgccccc cttccctctc ccggccagac     900
tctgagcagg agctccgccc ccagcgcgcc gccccagccc cggcgcctta aaagccgggc     960
gcaccgcccc gccgcgccct gcctgccgca cctctccttt cttctgtagc tcgcgttgaa    1020
gccgcacgtc cggccccgat cccggcacca tgagcttcgg ctcggagcac tacctgtgct    1080
cctcctcctc ctaccgcaag gtgttcgggg atggctctcg cctgtccgcc cgcctctctg    1140
gggccggcgg cgcgggcggc ttccgctcgc agtcgctgtc ccgcagcaat gtggcctcct    1200
cggccgcctg ctcctcggcc tcgtcgctcg gcctcggcct ggcctatcgc cggccgccgg    1260
cgtccgacgg gctggacctg agccaggcgg cggcgcgcac                           1300
```

<210> 17
<211> 300
<212> DNA
<213> Homo Sapiens

<400> 17

```
cagctctgga cgcccgcagc ggggcctcga agaggttcag ggcggtgccc gcggcgctcg      60
ggccgggtct cccggggcgt ggggcggggg gcggggttgg gcggcggccg gggctcctcc     120
ctcttctgcc ccgggctccc ctgctcttaa cccgcgcgcg ggggcgccca ggccactggg     180
ctccgcggag ccagcgagag gtctgcgcgg agtctgagcg gcgctcgtcc cgtcccaagg     240
ccgacgccag cacgccgtca tggcccccgc agcggcgacg gggggcagca ccctgcccag     300
```

<210> 18
<211> 300
<212> DNA
<213> Homo Sapiens

<400> 18

cagctctgga cgcccgcagc ggggcctcga agaggttcag ggcggtgccc gcggcgctcg 60
ggccgggtct cccggggcgt ggggcggggg gcggggttgg gcggcggccg gggctcctcc 120
ctcttctgcc ccgggctccc ctgctcttaa cccgcgcgcg gggcgcccca ggccactggg 180
ctccgcggag ccagcgagag gtctgcgcgg agtctgagcg gcgctcgtcc cgtcccaagg 240
ccgacgccag cacgccgtca tggcccccgc agcggcgacg gggggcagca ccctgcccag 300

<210> 19
<211> 300
<212> DNA
<213> Homo Sapiens

<400> 19

cagctctgga cgcccgcagc ggggcctcga agaggttcag ggcggtgccc gcggcgctcg 60
ggccgggtct cccggggcgt ggggcggggg gcggggttgg gcggcggccg gggctcctcc 120
ctcttctgcc ccgggctccc ctgctcttaa cccgcgcgcg gggcgcccca ggccactggg 180
ctccgcggag ccagcgagag gtctgcgcgg agtctgagcg gcgctcgtcc cgtcccaagg 240
ccgacgccag cacgccgtca tggcccccgc agcggcgacg gggggcagca ccctgcccag 300

<210> 20
<211> 300
<212> DNA
<213> Homo Sapiens

<400> 20

cagctctgga cgcccgcagc ggggcctcga agaggttcag ggcggtgccc gcggcgctcg 60
ggccgggtct cccggggcgt ggggcggggg gcggggttgg gcggcggccg gggctcctcc 120
ctcttctgcc ccgggctccc ctgctcttaa cccgcgcgcg gggcgcccca ggccactggg 180
ctccgcggag ccagcgagag gtctgcgcgg agtctgagcg gcgctcgtcc cgtcccaagg 240
ccgacgccag cacgccgtca tggcccccgc agcggcgacg gggggcagca ccctgcccag 300

<210> 21
<211> 300
<212> DNA
<213> Homo Sapiens

<400> 21

gggcagggcg gtgtttccgt tttccttggc cgggttcagc accctacact caataagcgt 60
gggctttcta gcaggactgg acactcaggc ggtgtgtcaa tctttagaca atctttatag 120
agtgcataga tgtttaattt cataaaattt aggaaagctt catttcagga aggaatttta 180
ttcctctttt tctcccttag ggcaagcact ttaacctttt aagcccaacc agatgagttg 240
cctgcagttt tggaggcctt cagagcattt cactagacct ctgtctgtgt cggtccagtg 300

<210> 22
<211> 300
<212> DNA
<213> Homo Sapiens

<400> 22

agaggccccg caccgaggcg gaggctggca aaggggagtg gaaagggagg atggatgggg 60
ccgggggtg gggtggtgat gagggcgacg aaggaggggg tgtcattttc tttttctttc 120
ttttttaaa aaaagtattt ctctcgcgag aaaccgctgc gcggacgata cttgaagagg 180
tggggaaagg aggggctgc gggagccgcg gcagagactg tgggtgccac aagcggacag 240
gagccacagc tgggacagct gcgagcggag ccgagcagtg gctgtagcgg ccacgactgg 300

<210> 23
<211> 300
<212> DNA
<213> Homo Sapiens

<400> 23

attccggggg tctcgctcct tggcatagtg gtcgcttaac tccagcgcct tcccagtgcc 60
tcccaaacct ctcctcctcc tgcccggggc tgttctagga ggagcctaaa tgtccgcttc 120
cccagctgaa ggggattgtg gtgcgtaaac aggattaact ccctcgcccc cagctgcgcg 180
gatctgcgcg gcggcctgcg ggccagagcg agccttcggg tgcgtggagg gcgcagcact 240
ggagcggggg atggggaagc gcgcggcctt ggagccgtct cttgggcccg cttcctcgcc 300

<210> 24
<211> 300
<212> DNA
<213> Homo Sapiens

<400> 24

gacgagtctg gttactttta atccgaccgg ccgctgagag ccactttctc ctcctcctcc 60

tcctcctcct tctcttcctc ctccttcttc ctcctcctcc tcctcttccg agcggcctcg 120

gcgcgcgcga atgcgcggcc ccgcgccccc cccctcgcgc gcgctcccct cgcgcgcgcg 180

cacacacgca cacatcgtct ccagctctct gctcgctctg ctcgcagtca cagacacttg 240

agcacacgcg tacacccaga catcttcggg ctgctattgg attgactttg aaggttctgt 300

<210> 25
<211> 300
<212> DNA
<213> Homo Sapiens

<400> 25

ggtactcacg tgacctaggc tgcggcggcg gcgtgctgcg ggctctgtgg cgggagcgag 60

gccgacgggc ggggccgcgc ggccgcgtga cgcgaagcgt tcgagagcgc gcgtcgtgga 120

acgtcttggt tgccacggca agcgcgcgcg cgaggccttg ggaacctcgg gaccggcccc 180

cggcgagcgc agcggcgccc agtgtaaggg agtgggagct ggtccgtgcc gcggcggccg 240

cgcagggagc tctcgaggca acgccggggc gcccgaggtc tggaaggcgc aggtggggcg 300

<210> 26
<211> 300
<212> DNA
<213> Homo Sapiens

<400> 26

ttttaatagt ccaaccctta aaataaatgt gttgtatggc cacctgatct gaccactttc 60

tttcatgttg acatctttaa ttttaaaact gttttattta gtgcttaaat cttgtttaca 120

aaattgtctt cctaagtaat atgtctacct tttttttgg aatatggaat attttgctaa 180

ctgtttctca attgcatttt acagatcagg agaacctcag tctgacgaca ttgaagctag 240

ccgaatgtaa gtgtaacttg gttgagactg tggttcttat tttgagttgc cctagactgc 300

<210> 27
<211> 300
<212> DNA
<213> Homo Sapiens

<400> 27

aaacttcaag gttttaaaca ttttactata aacattatag aaaacattaa aaatttctta 60
atatgcattt taaagtatgc cttttatgtg gtgaacgttt taactaaaca tttctctaga 120
agtttttata acattaataa aactagtata cttttctctc ctagagagtt acagtggagg 180
taaaaggagt ggcttgcagg atggagaagc tgcaccagtg ttattggaag tgagccacca 240
tttgaatttg ctagctcatg ctgcagtatt cagattagtg ggtgttttgt gatcatattt 300

<210> 28
<211> 300
<212> DNA
<213> Homo Sapiens

<400> 28

aagcgggagg gaggcggggc cggcgaagga aggaggggcg gagcgcggcg ccctcccgcg 60
cgtcttggcc ccgccccacg tccccgcgtc ccggcctgga gccctcgccc ggccgggcgg 120
cgcgcgctgc ctgccgggat actcggcccg cccagccagt cctcccgtct tgcgccgcgg 180
ccgcgagatc cgtgtgtctc ccaagatggt ggcgctgggc tcggggtgac tacaggagac 240
gacggggcct tttcccttcg ccaggacccg acacaccagg cttcgctcgc tcgcgcaccc 300

<210> 29
<211> 300
<212> DNA
<213> Homo Sapiens

<400> 29

gaggactagg aggaggagga cggcgacgac cagaaggggc ccaagagagg gggcgagcga 60
ccgagcgccg cgacgcggaa gtgaggtgcg tgcgggctgc agcgcagacc ccggcccggc 120
ccctccgaga gcgtcctggg cgctccctca cgccttgcct tcaagccttc tgcctttcca 180
ccctcgtgag cggagaactg ggagtggcca ttcgacgaca ggttagcggg tttgcctccc 240
actcccccag cctcgcgtcg ccggctcaca gcggcctcct ctggggacag tcccccccgg 300

<210> 30
<211> 300
<212> DNA
<213> Homo Sapiens

<400> 30

ctagccaaga tggatgggag atgctaaatt tttaatgcca gagctaaaaa tgtctgcttt 60
gtccaatggt taaatgagtg tacacttaaa agagtctcac actttggagg gtttctcatg 120
atttttcagt gtttttgtt tattttttccc cgaaagttct cattcaaagt gtattttatg 180
ttttccagtg tggtgtaaag gaattcatta gccatggatg tattcatgaa aggactttca 240
aaggccaagg agggagttgt ggctgctgct gagaaaacca aacagggtgt ggcagaagca 300

<210> 31
<211> 300
<212> DNA
<213> Homo Sapiens

<400> 31

gaagatgggt aaactccaac cgcaaagcga tgatgccact tgtgggccct cagattggag 60
aggacggaga tgagtgacgg ggctgtgcgc actggggcac cgcgcacaga cgggaggagg 120
ggggtgtctg tgtgttccaa agggggggagt accagttaac tggccgccgg cccgtggggg 180
ttggtgagaa ggagggtgag cttggcggtg acgcacggcc ctcacgtgac cgggagctgc 240
agagctacgc agccttcggt gcagtcgtca ctcgtgtctc gctaccagct ccccgctgcc 300

<210> 32
<211> 300
<212> DNA
<213> Homo Sapiens

<400> 32

cctgtcccgg cgccccaggt cgtcgcgcgc gcagctgcgg tagtcactgc gcctccccgc 60
ccccactcct ggatgccccc cttccctctc ccggccagac tctgagcagg agctccgccc 120
ccagcgcgcc gccccagccc cggcgcctta aaagccgggc gcaccgcccc gccgcgccct 180
gcctgccgca cctctccttt cttctgtagc tcgcgttgaa gccgcacgtc cggccccgat 240
cccggcacca tgagcttcgg ctcggagcac tacctgtgct cctcctcctc ctaccgcaag 300

<210> 33
<211> 1300
<212> DNA
<213> Homo Sapiens

<400> 33

```
atttccttct ttccccctct gcacgcttgc tagccccagc gatcgctgct ggcccccggg      60
taggaaagtg gggttcctgg ccgtttctgc gacgctggcc tagggcttgc agctgctgtt     120
gagtgaaagc acgcagactg gcgggagccg atcatttctc gaatgaagaa gaaaaagcgc     180
aattccctcc ttatgctcta gggaattgag ccgcgtccca gatcacccat tccagaaatg     240
tgaaaccggg ccctcacaaa gtcgtctctg gtgaagaggt ggcgtgcggg gtgggggttg     300
gtggagggtg aaggcataag caaacatatt ttaaaatcca gatcgtagga agtgtcacct     360
ggcccctcac ccaggcatgc tttctggggg aagcgcaggg ccaagctttc cctagaaaag     420
ctggggcgaa gagagagcag gcggcggcta aggagctcct ggcaggctgg gaaggtggag     480
aagtggggtg aggtattttt ctagaaagtg tagccctagc tcatctccta gattggggaa     540
gagggaactg agggaggagg gaaggagacc cagggcagct ccaggatagg gaaatgttga     600
agaagggact gcgttctcca accgaaccct ccctcctggg aaccgcagcc cagcgcggta     660
actgagttac cgcaaccggg cggtggggag gaagggtggt ccaggaaacc ggcgagggag     720
aaaagcggtg gaagggagag tcttctccct ggagcggccc cagcagtaca aagtgctggt     780
cacagcgccc cttccgcccc tagattgacg agcagtggcg tggagccagc gcggaggctg     840
ccccctcccc ctcccgagcc cgcagcgcgg agcgcggttt agcaccaacg gagccggggg     900
cggcgtcttt gggatggaaa agggccaaag gggaggagtg gggtggggt gggggtttca     960
ctggtccact ataaaaggac cgctcggctg cccggttctt gcactcgctg gaaagcggct    1020
ccgagccagg ggctattgca aagccagggt gcgctaccgg acggagaggg gagagccctg    1080
agcagagtga gcaacatcgc agccaaggcg gaggccgaag aggggcgcca ggcaccaatc    1140
tccgcgttgc ctcagccccg gaggcgcccc agagcgcttc ttgtcccagc agagccactc    1200
tgcctgcgcc tgcctctcag tgtctccaac tttgcgctgg aagaaaaact tcccgcgcgc    1260
cggcagaact gcagcgcctc cttttagtga ctccgggagc                          1300
```

<210> 34
<211> 1300
<212> DNA
<213> Homo Sapiens

<400> 34

```
tccgcacatt cgagcaaaga caggctttag cgagttatta aaaacttagg ggcgctcttg      60
tcccccacag ggcccgaccg cacacagcaa ggcgatggcc cagctgtaag ttggtagcac     120
```

```
tgagaactag cagcgcgcgc ggagcccgct gagacttgaa tcaatctggt ctaacggttt      180
cccctaaacc gctaggagcc ctcaatcggc gggacagcag ggcgcggtga gtcaccgccg      240
gtgactaagc gaccccaccc ctctccctcg ggctttcctc tgccaccgcc gtctcgcaac      300
tcccgccgtc cgaagctgga ctgagcccgt taggtccctc gacagaacct cccctccccc      360
caacatctct ccgccaaggc aagtcgatgg acagaggcgc gggccggagc agcccccctt      420
tccaagcggg cggcgcgcga ggctgcggcg aggcctgagc cctgcgttcc tgcgctgtgc      480
gcgcccccac cccgcgttcc aatctcaggc gctctttgtt tctttctccg cgacttcaga      540
tctgagggat tccttactct ttcctcttcc cgctcctttg cccgcgggtc tccccgcctg      600
accgcagccc cgagaccgcc gcgcacctcc tcccacgccc ctttggcgtg gtgccaccgg      660
acccctctgg ttcagtccca ggcggacccc cccctcaccg cgcgaccccg ccttttcag      720
caccccaggg tgagcccagc tcagactatc atccggaaag cccccaaaag tcccagccca      780
gcgctgaagt aacgggacca tgcccagtcc caggccccgg agcaggaagg ctcgagggcg      840
ccccccaccc acccgcccac cctcccgct tctcgctagg tccctattgg ctggcgcgct      900
ccgcggctgg gatggcagtg ggaggggacc ctctttccta acggggttat aaaaacagcg      960
ccctcggcgg ggtccagtcc tctgccactc tcgctccgag gtccccgcgc cagagacgca     1020
gccgcgctcc caccacccac acccaccgcg ccctcgttcg cctcttctcc gggagccagt     1080
ccgcgccacc gccgccgccc aggccatcgc caccctccgc agccatgtcc accaggtccg     1140
tgtcctcgtc ctcctaccgc aggatgttcg gcggcccggg caccgcgagc cggccgagct     1200
ccagccggag ctacgtgact acgtccaccc gcacctacag cctgggcagc gcgctgcgcc     1260
ccagcaccag ccgcagcctc tacgcctcgt ccccgggcgg                           1300
```

<210> 35
<211> 1300
<212> DNA
<213> Homo Sapiens

<400> 35

cttcctctac ctcgctccct cccagcctgc ccatcttttg tgcccgtctt ttgtgccggc 60

caccagtctt tacaaacagt gccctggggg cccatactcc agctttccag agcgcacagc 120

tgcaagggcg gctgtgtgtt agagcccgtg atgccttcga gtattgagtc atgccattca 180

tttaagaagg aaaaatctct ccaaaacggg gaaatggtgg gcagtcctgt gtgactggtg 240

agactcttgt aggggcgttt ctacaacgac gaaacccttc ctaggcactc actccaacag 300

aataacaagc ccattttatt agtatttcgt tttccatgta aagttctgct catacgaata 360

tatttataat tctgattttt ttacggcatt ggggagcaca ccgacaggct gctgaacggt 420

ggctggagat tcgagggaaa acgaagttcg ccgaggcggc ctcgggcggg caggtcccgg 480

gctccatcac agggcacacg cggctaccag ggacgcagcc ccccaacaca cacacacaca 540

cacacacaca cacacacaca cacacacaca ccctctccca ctcatgcctg gcaacccagc 600

agaaacttcg gactggggca aaacaagccc gggccccggc ggcacgcggg gctaggcgcg 660

ttcccgccag tacctggtcg cgaggccgct cgcggggtgc cctgcgtgcc ccccactccc 720

gcagcccgcg ccctgctcgc tcactgtggg ggcgcagcgg ccaggcttct ctgtttgttg 780

tttaaagaaa tcctagggcg ggcgagcggc ggcatctagg ggagggggcg cagccagaat 840

tcccttccag caagcgcgtg aggggcattc tcaacgcaaa accagaccca gaaagtagtg 900

accagccctc ctcggattac ccttcattgg ctcctccctt gctcccccca ccctccagat 960

ttgcataaaa aaggccaaga aaactctggc tgtgccccag caacggctca ttctgctccc 1020

ccgggtcgga gccccccgga gctgcgcgcg ggcttgcagc gcctcgcccg cgctgtcctc 1080

ccggtgtccc gcttctccgc gccccagccg ccggctgcca gcttttcggg gccccgagtc 1140

gcacccagcg aagagagcgg gcccgggaca agctcgaact ccggccgcct cgcccttccc 1200

cggctccgct ccctctgccc cctcggggtc gcgcgcccac gatgctgcag ggccctggct 1260

cgctgctgct gctcttcctc gcctcgcact gctgcctggg 1300

<210> 36
<211> 1300
<212> DNA
<213> Homo Sapiens

<400> 36

ttttctattt aaatatatat tatatattta aaaaagtgtc ctcccagagc taataccgtt 60
gctagcagct cttcctgccg ccacaccggg caaagtccac ccactgcccc agtgttgagg 120
gccaccatgg gcggccccac ctggagaggt gctgctcaca gcaaacagct ccaactgcgc 180
cttcgcctcg ccttccaggg agcccagcca ggcccactgg gtatttacaa gcagacctcc 240
ctcgcttcag ccttcctgaa cccctgttag ttgggaaacc acctgtctgc accgcagcta 300
gagaaccgag gagaggagcc gctagtctaa agggctgttg gttgaaatta ggaagcagtg 360
taaagaaaaa gaaaaaaaaa gtttgggagg ccaaggcagg agcatcacct gaggtcagga 420
gttcgagacc agcctggcta acatggtgaa accccgtctc tactaaaaat acaaaaaatt 480
agcgggggca tggtggcacg cggctgtaat cccagctact cgggaggctg aggcaggaga 540
atcgcttgaa cccgggaggc ggaggttgca gtcagcggag atagcgccat tgcactccag 600
cttaggcaac aagagcgaaa ctgtctcaaa aaaaaaaagt cttcataatt tcatgggttt 660
gcaagtatga tccaggctcc ccgcttctct gcaagccaat gcgagttaat tacagcgtcc 720
gccctggtct ctctccaccc cacgccgtga tccattcccc ttctttttct ccccttgtct 780
ctttcctcct ccccctttta tttatgtatt tttggttttg ttttttaagg ggtgttgagc 840
cgcgtctggt tctagtaaac cgaacccgct cgcgagggag gcgattggct cccgcgccgg 900
tgacggacgt ggtaacgagt gcggctcgcc ccgccgggag ctgattggct gcgcggggcg 960
gctccgaggg ctcggccgta ggagccccgc gcactccagc cctgcagcct ccggagtcag 1020
tgccgcgcgc ccgccgcccc gcgccttcct gctcgccgca cctccgggag ccggggcgca 1080
cccagcccgc agcgccgcct ccccgcccgc gccgcctccg accgcaggcc gagggccgcc 1140
actggccggg gggaccgggc agcagcttgc ggccgcggag ccgggcaacg ctggggactg 1200
cgccttttgt ccccggaggt ccctggaagt ttgcggcagg acgcgcgcgg ggaggcggcg 1260
gaggcagccc cgacgtcgcg gagaacaggg cgcagagccg 1300

<210> 37
<211> 24
<212> DNA
<213> Artificial Sequence

<220>
<223> PCR primer

<400> 37
ttcgggtttt tttgttttta attc 24

<210> 38
<211> 25
<212> DNA
<213> Artificial Sequence

<220>
<223> PCR primer

<400> 38
gaaaaccata acgacgtact aacgt 25

<210> 39
<211> 25
<212> DNA
<213> Artificial Sequence

<220>
<223> PCR primer

<400> 39
ttttgggttt ttttgttttt aattt 25

<210> 40
<211> 28
<212> DNA
<213> Artificial Sequence

<220>
<223> PCR primer

<400> 40
acaaaaacca taacaacata ctaacatc 28

<210> 41
<211> 21
<212> DNA
<213> Artificial Sequence

<220>
<223> PCR primer

<400> 41
gtaatttaga tttcggaggg c 21

<210> 42
<211> 19
<212> DNA
<213> Artificial Sequence

<220>
<223> PCR primer

<400> 42
cgaccaaaaa aaacgaaaa 19

<210> 43
<211> 24
<212> DNA
<213> Artificial Sequence

<220>
<223> PCR primer

<400> 43
tttgtaattt agattttgga gggt 24

<210> 44
<211> 22
<212> DNA
<213> Artificial Sequence

<220>
<223> PCR primer

<400> 44
ccaaccaaaa aaaacaaaaa ca 22

<210> 45
<211> 21
<212> DNA
<213> Artificial Sequence

<220>
<223> PCR primer

<400> 45
gtattttttt cgcgagaaat c 21

<210> 46
<211> 19
<212> DNA
<213> Artificial Sequence

<220>
<223> PCR primer

<400> 46
aatcgtaacc gctacaacc 19

<210> 47
<211> 24
<212> DNA
<213> Artificial Sequence

<220>
<223> PCR primer

<400> 47
aaagtatttt ttttgtgaga aatt 24

<210> 48
<211> 22
<212> DNA
<213> Artificial Sequence

<220>
<223> PCR primer

<400> 48
cccaatcata accactacaa cc 22

<210> 49
<211> 22
<212> DNA
<213> Artificial Sequence

<220>
<223> PCR primer

<400> 49
gttattttta attcgatcgg tc 22

<210> 50
<211> 18
<212> DNA
<213> Artificial Sequence

<220>
<223> PCR primer

<400> 50
aaaccgctcg aaaaaaaa 18

<210> 51
<211> 25
<212> DNA
<213> Artificial Sequence

<220>
<223> PCR primer

<400> 51
ttggttattt ttaatttgat tggtt 25

<210> 52
<211> 21
<212> DNA
<213> Artificial Sequence

<220>
<223> PCR primer

<400> 52
ccaaaaccac tcaaaaaaaa a 21

<210> 53
<211> 21
<212> DNA
<213> Artificial Sequence

<220>
<223> PCR primer

<400> 53
tcgttttttg gtatagtggt c 21

<210> 54
<211> 18
<212> DNA

<213> Artificial Sequence

<220>
<223> PCR primer

<400> 54
caaatccgcg caactaaa 18

<210> 55
<211> 24
<212> DNA
<213> Artificial Sequence

<220>
<223> PCR primer

<400> 55
gttttgtttt ttggtatagt ggtt 24

<210> 56
<211> 21
<212> DNA
<213> Artificial Sequence

<220>
<223> PCR primer

<400> 56
caaatccaca caactaaaaa c 21

<210> 57
<211> 20
<212> DNA
<213> Artificial Sequence

<220>
<223> PCR primer

<400> 57
tggaacgttt tggttgttac 20

<210> 58
<211> 20
<212> DNA
<213> Artificial Sequence

<220>
<223> PCR primer

<400> 58
tacctcgaaa actccctacg 20

<210> 59
<211> 21
<212> DNA
<213> Artificial Sequence

<220>

<223> PCR primer

<400> 59
gtggaatgtt ttggttgtta t 21

<210> 60
<211> 21
<212> DNA
<213> Artificial Sequence

<220>
<223> PCR primer

<400> 60
ttacctcaaa aactccctac a 21

<210> 61
<211> 20
<212> DNA
<213> Artificial Sequence

<220>
<223> PCR primer

<400> 61
cgttgtttgt cgggatattc 20

<210> 62
<211> 20
<212> DNA
<213> Artificial Sequence

<220>
<223> PCR primer

<400> 62
cccgtcgtct cctataatca 20

<210> 63
<211> 21
<212> DNA
<213> Artificial Sequence

<220>
<223> PCR primer

<400> 63
gtgttgtttg ttgggatatt t 21

<210> 64
<211> 21
<212> DNA
<213> Artificial Sequence

<220>
<223> PCR primer

<400> 64

ccccatcatc tcctataatc a 21

<210> 65
<211> 20
<212> DNA
<213> Artificial Sequence

<220>
<223> PCR primer

<400> 65
cgggttgtag cgtagatttc 20

<210> 66
<211> 18
<212> DNA
<213> Artificial Sequence

<220>
<223> PCR primer

<400> 66
cgtcgaataa ccactccc 18

<210> 67
<211> 23
<212> DNA
<213> Artificial Sequence

<220>
<223> PCR primer

<400> 67
gtgtgggttg tagtgtagat ttt 23

<210> 68
<211> 21
<212> DNA
<213> Artificial Sequence

<220>
<223> PCR primer

<400> 68
tcatcaaata accactccca a 21

<210> 69
<211> 20
<212> DNA
<213> Artificial Sequence

<220>
<223> PCR primer

<400> 69
agttaattgg tcgtcggttc 20

<210> 70

<211> 20
<212> DNA
<213> Artificial Sequence

<220>
<223> PCR primer

<400> 70
cgaataacga ctacaccgaa 20

<210> 71
<211> 23
<212> DNA
<213> Artificial Sequence

<220>
<223> PCR primer

<400> 71
attagttaat tggttgttgg ttt 23

<210> 72
<211> 23
<212> DNA
<213> Artificial Sequence

<220>
<223> PCR primer

<400> 72
acacaaataa caactacacc aaa 23

<210> 73
<211> 20
<212> DNA
<213> Artificial Sequence

<220>
<223> PCR primer

<400> 73
aggagtttcg tttttagcgc 20

<210> 74
<211> 20
<212> DNA
<213> Artificial Sequence

<220>
<223> PCR primer

<400> 74
acgacttcaa cgcgaactac 20

<210> 75
<211> 23
<212> DNA
<213> Artificial Sequence

<220>
<223> PCR primer

<400> 75
agtaggagtt ttgtttttag tgt 23

<210> 76
<211> 23
<212> DNA
<213> Artificial Sequence

<220>
<223> PCR primer

<400> 76
acaacttcaa cacaaactac aaa 23

<210> 77
<211> 20
<212> DNA
<213> Artificial Sequence

<220>
<223> sequencing primer

<400> 77
gggttttttt gtttttaatt 20

<210> 78
<211> 22
<212> DNA
<213> Artificial Sequence

<220>
<223> sequencing primer

<400> 78
accaaaaacc actcacaaac tc 22

<210> 79
<211> 22
<212> DNA
<213> Artificial Sequence

<220>
<223> sequencing primer

<400> 79
ggagggtaga tgattttgag aa 22

<210> 80
<211> 22
<212> DNA
<213> Artificial Sequence

<220>
<223> sequencing primer

<400> 80
cttccccttc ccctaactac ta 22

<210> 81
<211> 24
<212> DNA
<213> Artificial Sequence

<220>
<223> sequencing primer

<400> 81
agggggtgtt attttttttt tttt 24

<210> 82
<211> 19
<212> DNA
<213> Artificial Sequence

<220>
<223> sequencing primer

<400> 82
cccaatccct atccctacc 19

<210> 83
<211> 22
<212> DNA
<213> Artificial Sequence

<220>
<223> sequencing primer

<400> 83
ttttggayg agtttggtta tt 22

<210> 84
<211> 23
<212> DNA
<213> Artificial Sequence

<220>
<223> sequencing primer

<400> 84
ccacctaatt caaacataca acc 23

<210> 85
<211> 20
<212> DNA
<213> Artificial Sequence

<220>
<223> sequencing primer

<400> 85
ttttaygtag ttggtygagg 20

<210> 86
<211> 22
<212> DNA
<213> Artificial Sequence

<220>
<223> sequencing primer

<400> 86
ctccttaaac tataaccccc ct 22

<210> 87
<211> 22
<212> DNA
<213> Artificial Sequence

<220>
<223> sequencing primer

<400> 87
atttagtttg agtaggtygg tg 22

<210> 88
<211> 22
<212> DNA
<213> Artificial Sequence

<220>
<223> sequencing primer

<400> 88
ctccatccta acaatccata aa 22

<210> 89
<211> 17
<212> DNA
<213> Artificial Sequence

<220>
<223> sequencing primer

<400> 89
ggggggtgtt tagaggg 17

<210> 90
<211> 23
<212> DNA
<213> Artificial Sequence

<220>
<223> sequencing primer

<400> 90
cctcctacca aaaactacaa acc 23

<210> 91
<211> 19
<212> DNA

<213> Artificial Sequence

<220>
<223> sequencing primer

<400> 91
agaaggggtt taagagagg 19

<210> 92
<211> 19
<212> DNA
<213> Artificial Sequence

<220>
<223> sequencing primer

<400> 92
actatcccca aaaaaaacc 19

<210> 93
<211> 22
<212> DNA
<213> Artificial Sequence

<220>
<223> sequencing primer

<400> 93
atttgtgggt ttttagattg ga 22

<210> 94
<211> 22
<212> DNA
<213> Artificial Sequence

<220>
<223> sequencing primer

<400> 94
ccaaaaaacc actatattcc ca 22

<210> 95
<211> 22
<212> DNA
<213> Artificial Sequence

<220>
<223> sequencing primer

<400> 95
gatgtagatg gttttgttty gg 22

<210> 96
<211> 18
<212> DNA
<213> Artificial Sequence

<220>

<223> sequencing primer

<400> 96
caaacraaaa ccatcccc 18

<210> 97
<211> 20
<212> DNA
<213> Artificial Sequence

<220>
<223> sequencing primer

<400> 97
gggtttttt gtttttaatt 20

<210> 98
<211> 22
<212> DNA
<213> Artificial Sequence

<220>
<223> sequencing primer

<400> 98
accaaaaacc actcacaaac tc 22

<210> 99
<211> 25
<212> DNA
<213> Artificial Sequence

<220>
<223> sequencing primer

<400> 99
ggaaaaatga aggagattta aattt 25

<210> 100
<211> 18
<212> DNA
<213> Artificial Sequence

<220>
<223> sequencing primer

<400> 100
aataacctaa acrccccc 18

## Claims

**1.** A method for determining whether a subject has developed is developing or is predisposed for developing cancer, or whether a subject is relapsing after treatment of cancer, comprising the step of:

a) determining the methylation level, the number of methylated CpG sites or the methylation state of CpG sites in a nucleic acid sequence in the promoter region, first exon or intron, of at least one gene in a sample, obtained from said subject, wherein said gene is SNCA, e.g. as identified by ensembl gene id ENSG00000145335, entrez id 6622, and wherein said sequence comprises a nucleic acid sequence consisting of SEQ ID NO.: 13 and SEQ ID NO.:14.

2. The method of claim 1 for determining whether a subject has developed, is developing, is predisposed for developing or is relapsing after treatment of a cancer within the aero-digestive system, comprising the step of

a)

(i) determining the methylation level, the number of methylated CpG sites or the methylation state of CpG sites in a nucleic acid sequence in the promoter region, first exon or intron of SNCA (e.g. as identified by ensembl gene id ENSG00000145335, entrez id 6622) in a sample obtained from said subject and wherein said sequence comprises a nucleic acid sequence consisting of SEQ ID NO.: 13 and SEQ ID NO.: 14, or
(ii) determining the methylation level, the number of methylated CpG sites or the methylation state of CpG sites in a nucleic acid sequence comprising a sequence selected from the group consisting of:

1) A nucleic acid sequence as defined by SEQ ID NO: 13 or SEQ ID NO: 14;
2) A nucleic acid sequence which is complementary to a sequence as defined in 1);
3) A sub-sequence of a nucleic acid sequence as defined in 1) or 2);
4) A nucleic acid sequence which is at least 75% identical to a sequence as defined in 1), 2) or 3)
in a sample obtained from said subject,

b) comparing said methylation level, the number of methylated CpG sites or the methylation state of CpG sites to a reference; and
c) identifying the subject as being likely to develop, developing or being predisposed for developing said cancer, or relapsing after treatment of said cancer, if the methylation level, the number of methylated CpG sites, or the methylation state of CpG sites, are higher than the reference, and identifying the subject as unlikely to develop, developing or being predisposed for developing said cancer, or relapsing after treatment of said cancer, if the methylation level, the number of methylated CpG sites or the methylation state of CpG sites is below the reference.

3. A method according to claims 1-2, wherein the methylation level, the number of methylated CpG sites or the methylation state of CpG sites is determined by bisulphite sequencing, quantitative and/or qualitative methylation specific polymerase chain reaction (MSP), pyrosequencing, Southern blotting, restriction landmark genome scanning (RLGS), single nucleotide primer extension, CpG Island microarray, SNUPE, COBRA, mass spectrometry, by use of methylation specific restriction enzymes, by measuring the expression level of said genes or a combination thereof.

4. A method according to claim 3, wherein said methylation specific PCR comprises the use of nucleic acid primers which are capable of hybridizing to a nucleic acid sequence comprising 2 CpG sites and a cytosine residue which is not within a CpG site.

5. A method according to any of the preceding claims, wherein the cancer within the aero-digestive system is selected from the group consisting of: colorectal tumours, lung tumours, small cell lung cancer, non-small cell lung cancer, esophageal tumours, gastric tumours, pancreas tumours, liver tumours, tumours of the gall bladder and/or bile duct, tumours of the small bowel, and tumours of the large bowel.

6. A method according to any of the preceding claims, wherein sample is obtained from blood, stool, urine, pleural fluid, gall, bronchial fluid, oral washings, tissue biopsies, ascites, pus, cerebrospinal fluid, aspirate, follicular fluid, tissue or mucus.

7. A method according to any of the proceeding claims, where the methylation level, the number of methylated CpG sites or the methylation state of CpG sites is combined with at least one additional marker.

8. The use of a SNCA gene in a diagnostic assay wherein the methylation level, the number of methylated CpG sites or the methylation state of CpG sites is assessed as an indicator of whether a subject has developed, is developing or is predisposed for developing cancer within the aero-digestive system, or whether a subject is relapsing after treatment of cancer within the aero-digestive system.

**Patentansprüche**

1. Verfahren zur Bestimmung, ob ein Patient eine Krebserkrankung entwickelt hat, entwickelt oder dafür prädisponiert ist oder ob ein Patient nach der Behandlung einer Krebserkrankung rezidiviert, umfassend den Schritt:

   a) Bestimmen des Methylierungsgrads, der Anzahl methylierter CpG-Inseln oder des Methylierungszustands von CpG-Inseln in einer Nukleinsäuresequenz der Promotorregion, des ersten Exons oder Introns mindestens eines Gens einer vom Patienten genommenen Probe, wobei das Gen SNCA ist, z. B. identifiziert durch ENSEMBL Gen ID ENSG00000145335, Entrez ID 6622, und wobei die Sequenz eine Nukleinsäuresequenz umfasst, bestehend aus SEQ ID NR. 13 und SEQ ID NR. 14.

2. Verfahren nach Anspruch 1 zur Bestimmung, ob ein Patient eine Krebserkrankung der Luft- und Speisewege entwickelt hat, entwickelt, dafür prädisponiert ist oder nach deren Behandlung rezidiviert, umfassend den Schritt

   a)

   (i) Bestimmen des Methylierungsgrads, der Anzahl methylierter CpG-Inseln oder des Methylierungszustands von CpG-Inseln in einer Nukleinsäuresequenz der Promotorregion, des ersten Exons oder Introns von SNCA (z. B. identifiziert durch ENSEMBL Gen ID ENSG00000145335, Entrez ID 6622) einer vom Patienten genommenen Probe und wobei die Sequenz eine Nukleinsäuresequenz umfasst, bestehend aus SEQ ID NR. 13 und SEQ ID NR. 14,
   oder
   (ii) Bestimmen des Methylierungsgrads, der Anzahl methylierter CpG-Inseln oder des Methylierungszustands von CpG-Inseln in einer Nukleinsäuresequenz, umfassend eine Sequenz, ausgewählt aus der Gruppe, bestehend aus:

   1) einer Nukleinsäuresequenz, definiert durch SEQ ID Nr. 13 oder SEQ ID NR. 14;
   2) einer Nukleinsäuresequenz, die komplementär zu einer unter 1) definierten Sequenz ist;
   3) einer Untersequenz der unter 1) oder 2) definierten Sequenz;
   4) einer Nukleinsäuresequenz einer vom Patienten genommenen Probe, die zu mindestens 75 % mit einer unter 1), 2) oder 3) definierten Sequenz identisch ist,

   b) Vergleichen des Methylierungsgrads, der Anzahl methylierter CpG-Inseln oder des Methylierungszustands von CpG-Inseln mit einer Referenz; und
   c) Feststellen, dass der Patient die Krebserkrankung wahrscheinlich entwickeln wird, entwickelt oder dafür prädisponiert ist oder nach Behandlung der Krebserkrankung rezidiviert, wenn der Methylierungsgrad, die Anzahl methylierter CpG-Inseln oder der Methylierungszustands von CpG-Inseln höher ist als bei der Referenz, und Feststellen, dass der Patient die Krebserkrankung wahrscheinlich nicht entwickeln wird, entwickelt oder dafür prädisponiert ist oder nach Behandlung der Krebserkrankung rezidiviert, wenn der Methylierungsgrad, die Anzahl methylierter CpG-Inseln oder der Methylierungszustands von CpG-Inseln niedriger ist als bei der Referenz.

3. Verfahren nach Anspruch 1-2, wobei der Methylierungsgrad, die Anzahl methylierter CpG-Inseln oder der Methylierungszustands von CpG-Inseln mittels Bisulfitsequenzierung, quantitativer und/oder qualitativer methylierungsspezifischer Polymerase-Kettenreaktion (MSP), Pyrosequenzierung, Southern Blotting, Restriction Landmark Genome Scanning (RLGS), Extension eines Primers um ein Nukleotid (Single Nucleotide Primer Extension), CpG-Insel-Mikroarray, SNUPE, COBRA, Massenspektrometrie, durch Verwendung methylierungsspezifischer Restriktionsenzyme, Ermitteln des Expressionsgrads der Gene oder eine Kombination davon bestimmt wird.

4. Verfahren nach Anspruch 3, wobei die methylierungsspezifische Polymerase-Kettenreaktion die Verwendung von Nukleinsäureprimern umfasst, die an eine Nukleinsäuresequenz mit zwei CpG-Inseln und einem Cytosin-Rest, der sich nicht in einer CpG-Insel befindet, hybridisieren können.

5. Verfahren nach einem der vorhergehenden Ansprüche, wobei die Krebserkrankung in den Luft- und Speisewegen ausgewählt ist aus der Gruppe, bestehend aus: kolorektalen Tumoren, Lungentumoren, kleinzelligem Lungenkarzinom, nichtkleinzelligem Lungenkarzinom, Speiseröhretumoren, Magentumoren, Pankreastumoren, Lebertumoren, Tumoren der Gallenblase und/oder des Gallengangs, Tumoren des Dünndarms und Tumoren des Dickdarms.

6. Verfahren nach einem der vorhergehenden Ansprüche, wobei die Probe aus Blut, Stuhl, Urin, Pleuraflüssigkeit, Galle, Bronchialflüssigkeit, Mundspülungen, Gewebebiopsien, Aszites, Eiter, Rückenmarkflüssigkeit, Aspirat, Follikelflüssigkeit, Gewebe oder Schleim erhalten wird.

7. Verfahren nach einem der vorhergehenden Ansprüche, wobei der Methylierungsgrad, die Anzahl methylierter CpG-Inseln oder der Methylierungszustands von CpG-Inseln mit mindestens einem weiteren Marker kombiniert wird.

8. Verwendung des SNCA Gens in einem Diagnose-Assay, wobei der Methylierungsgrad, die Anzahl methylierter CpG-Inseln oder der Methylierungszustands von CpG-Inseln als Indikator dafür beurteilt wird, ob ein Patient eine Krebserkrankung der Luft- und Speisewege entwickelt hat, entwickelt oder dafür prädisponiert ist oder ob ein Patient nach der Behandlung einer Krebserkrankung der Luft- und Speisewege rezidividiert.

## Revendications

1. Méthode pour déterminer si un sujet a développé, développe ou est prédisposé à développer un cancer, ou si un sujet rechute après le traitement d'un cancer, comprenant l'étape suivante :

   a) détermination du niveau de méthylation, du nombre de sites CpG méthylés ou de l'état de méthylation de sites CpG dans une séquence d'acide nucléique de la région promotrice, du premier exon intron d'au moins un gène dans un échantillon prélevé sur ledit sujet, où ledit gène est SNCA, par exemple tel qu'identifié par l'identifiant de gène Ensembl ENSG00000145335, l'identifiant Entrez 6622, et où ladite séquence comprend une séquence d'acide nucléique consistant en SEQ ID NO.: 13 et SEQ ID NO.: 14.

2. La méthode de la revendication 1 pour déterminer si un sujet a développé, développe, est prédisposé à développer ou rechute après le traitement d'un cancer des voies aérodigestives, comprenant les étapes suivantes :

   a)

   (i) détermination du niveau de méthylation, du nombre de sites CpG méthylés ou de l'état de méthylation de sites CpG dans une séquence d'acide nucléique de la région promotrice, du premier exon ou intron de SNCA (par exemple tel qu'identifié par l'identifiant de gène Ensembl ENSG00000145335, l'identifiant Entrez 6622) dans un échantillon prélevé sur ledit sujet, et où ladite séquence comprend une séquence d'acide nucléique consistant en SEQ ID NO.: 13 et SEQ ID NO.: 14, ou
   (ii) détermination du niveau de méthylation, du nombre de sites CpG méthylés ou de l'état de méthylation de sites CpG dans une séquence d'acide nucléique comprenant une séquence choisie dans le groupe consistant en :

   1) une séquence d'acide nucléique telle que définie par SEQ ID NO: 13 ou SEQ ID NO: 14;
   2) une séquence d'acide nucléique complémentaire d'une séquence telle que définie dans 1) ;
   3) une sous-séquence d'une séquence d'acide nucléique telle que définie dans 1) ou 2) ;
   4) une séquence d'acide nucléique au moins 75% identique à une séquence telle que définie dans 1), 2) ou 3)
   dans un échantillon prélevé sur ledit sujet,

   b) comparaison dudit niveau de méthylation, du nombre de sites CpG méthylés ou de l'état de méthylation de sites CpG à une référence ; et
   c) identification du sujet tel qu'il soit probable qu'il ait développé, développe ou soit prédisposé à développer ledit cancer, ou ait rechuté après le traitement dudit cancer, si le niveau de méthylation, le nombre de sites CpG méthylés ou l'état de méthylation de sites CpG sont supérieurs à la référence, et identification du sujet tel qu'il ne soit pas probable qu'il ait développé, développe ou soit prédisposé à développer ledit cancer, ou ait rechuté après le traitement dudit cancer, si le niveau de méthylation, le nombre de sites CpG méthylés ou l'état de méthylation de sites CpG sont inférieurs à la référence.

3. Méthode selon les revendications 1 à 2, où le niveau de méthylation, le nombre de sites CpG méthylés ou l'état de méthylation de sites CpG est déterminé par séquençage au bisulfite, réaction en chaîne par polymérase spécifique de la méthylation (MSP) quantitative et/ou qualitative, pyroséquençage, transfert de Southern, méthode RLGS (restriction landmark genome scanning), extension d'amorce mononucléotidique, puce à ADN pour îlots CpG, SNUPE, COBRA, spectrométrie de masse, par utilisation d'enzymes de restriction spécifiques de la méthylation, par mesure du niveau d'expression desdits gènes ou une combinaison de ceux-ci.

4. Méthode selon la revendication 3, où ladite réaction en chaîne par polymérase spécifique de la méthylation comprend l'utilisation d'amorces d'acide nucléique qui sont capables de s'hybrider sur une séquence d'acide nucléique comprenant 2 sites CpG et un résidu cytosine qui ne se trouve pas dans un site CpG.

5. Méthode selon l'une quelconque des revendications précédentes, où le cancer des voies aérodigestives est choisi dans le groupe consistant en : tumeurs colorectales, tumeurs pulmonaires, cancer du poumon à petites cellules, cancer du poumon non à petites cellules, tumeurs oesophagiennes, tumeurs gastriques, tumeurs pancréatiques, tumeurs hépatiques, tumeurs de la vésicule biliaire et/ou du canal cholédoque, tumeurs de l'intestin grêle et tumeurs du gros intestin.

6. Méthode selon l'une quelconque des revendications précédentes, où l'échantillon est prélevé dans du sang, des fèces, de l'urine, du liquide pleural, de la bile, du liquide bronchique, des lavages buccaux, des biopsies de tissus, de l'ascite, du pus, du liquide céphalorachidien, des aspirats, du liquide folliculaire, du tissu ou du mucus.

7. Méthode selon l'une quelconque des revendications précédentes, où le niveau de méthylation, le nombre de sites CpG méthylés ou l'état de méthylation de sites CpG est combiné avec au moins un marqueur supplémentaire.

8. L'utilisation du gène SNCA dans un test diagnostic où le niveau de méthylation, le nombre de sites CpG méthylés ou l'état de méthylation de sites CpG est évalué en tant qu'indicateur du fait qu'un sujet a développé, développe ou est prédisposé à développer un cancer des voies aérodigestives, ou qu'un sujet rechute après le traitement d'un cancer des voies aérodigestives.

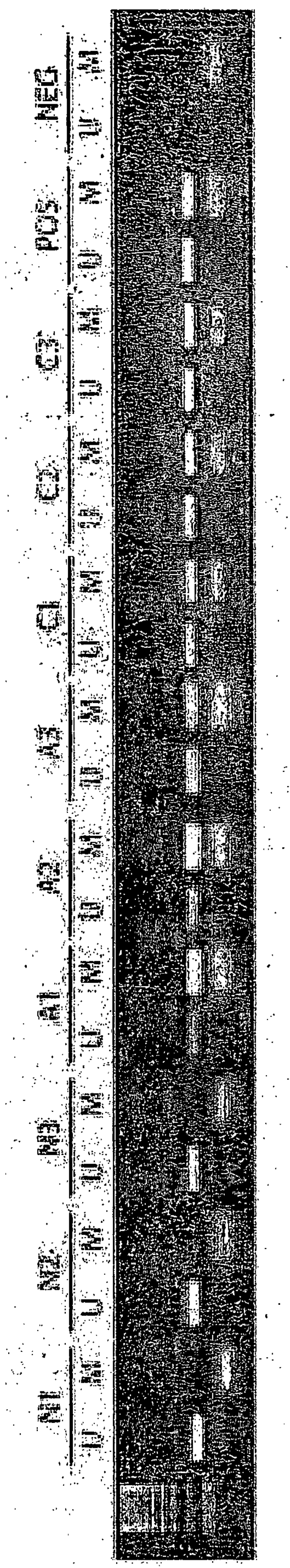


Fig. 1

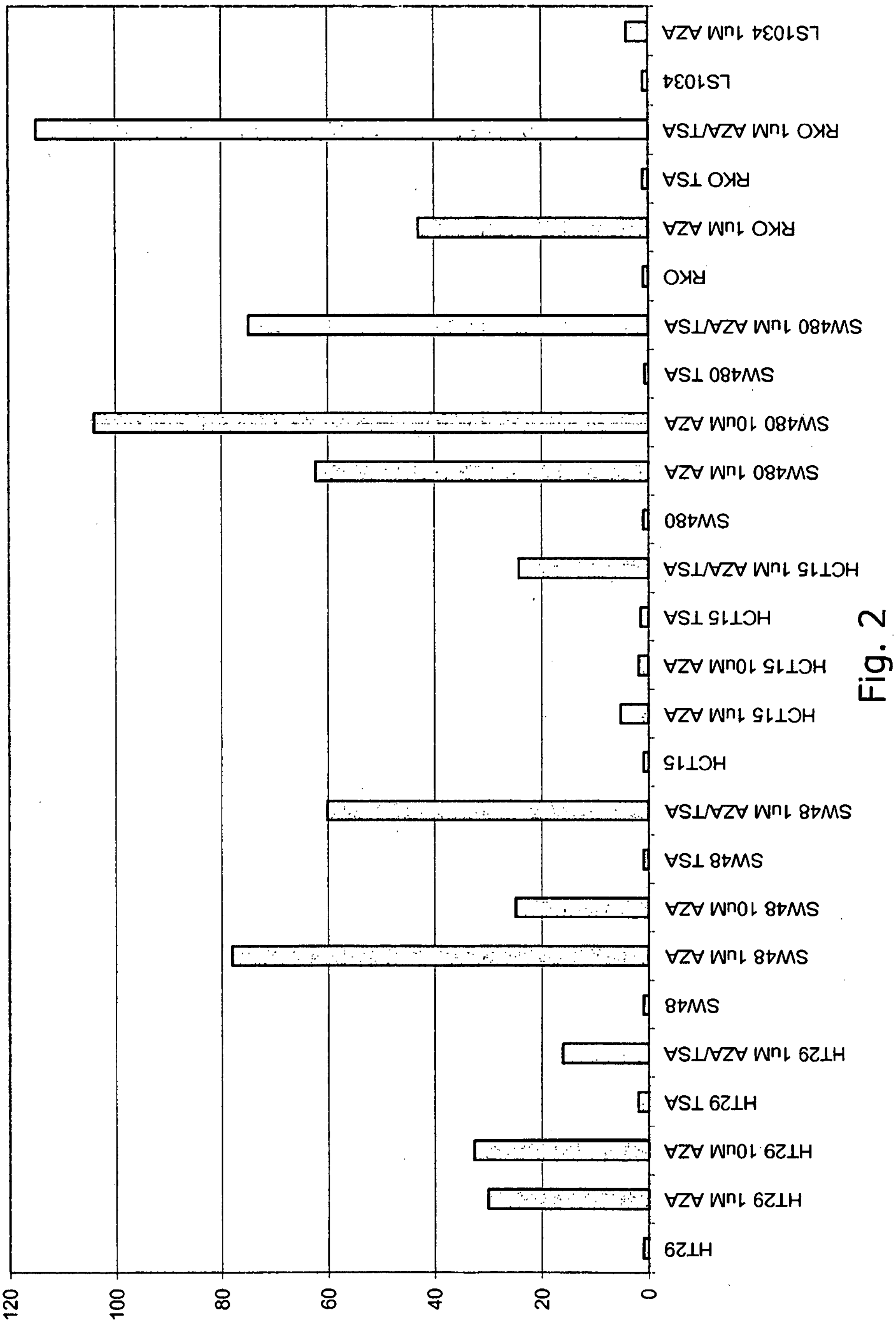
HT29
HT29 1uM AZA
HT29 10uM AZA
HT29 TSA
HT29 1uM AZA/TSA
SW48
SW48 1uM AZA
SW48 10uM AZA
SW48 TSA
SW48 1uM AZA/TSA
HCT15
HCT15 1uM AZA
HCT15 10uM AZA
HCT15 TSA
HCT15 1uM AZA/TSA
SW480
SW480 1uM AZA
SW480 10uM AZA
SW480 TSA
SW480 1uM AZA/TSA
RKO
RKO 1uM AZA
RKO TSA
RKO 1uM AZA/TSA
LS1034
LS1034 1uM AZA
0
20
40
60
80
100
120

Fig. 2

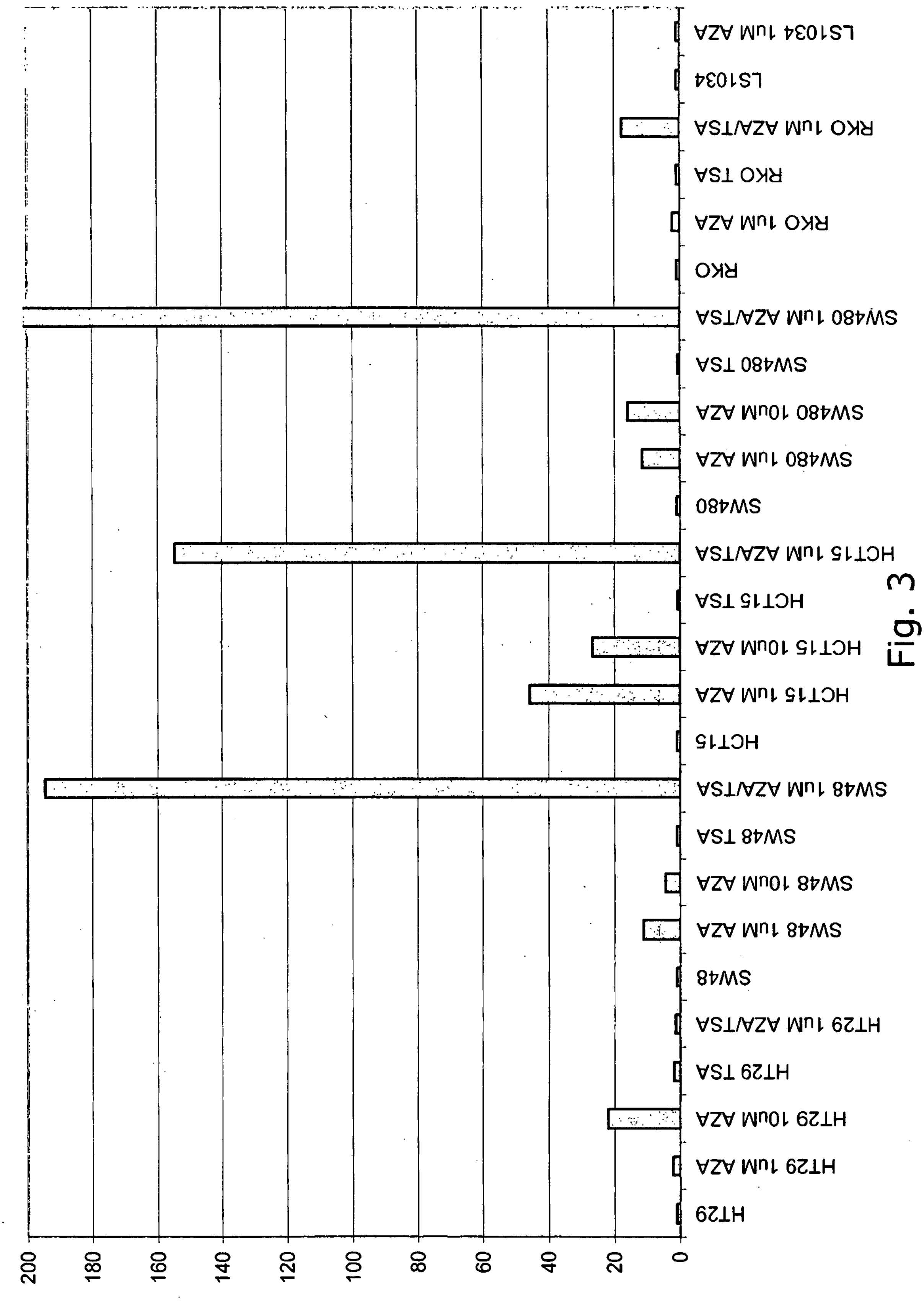

HT29
HT29 1uM AZA
HT29 10uM AZA
HT29 TSA
HT29 1uM AZA/TSA
SW48
SW48 1uM AZA
SW48 10uM AZA
SW48 TSA
SW48 1uM AZA/TSA
HCT15
HCT15 1uM AZA
HCT15 10uM AZA
HCT15 TSA
HCT15 1uM AZA/TSA
SW480
SW480 1uM AZA
SW480 10uM AZA
SW480 TSA
SW480 1uM AZA/TSA
RKO
RKO 1uM AZA
RKO TSA
RKO 1uM AZA/TSA
LS1034
LS1034 1uM AZA
0
20
40
60
80
100
120
140
160
180
200


Fig. 3

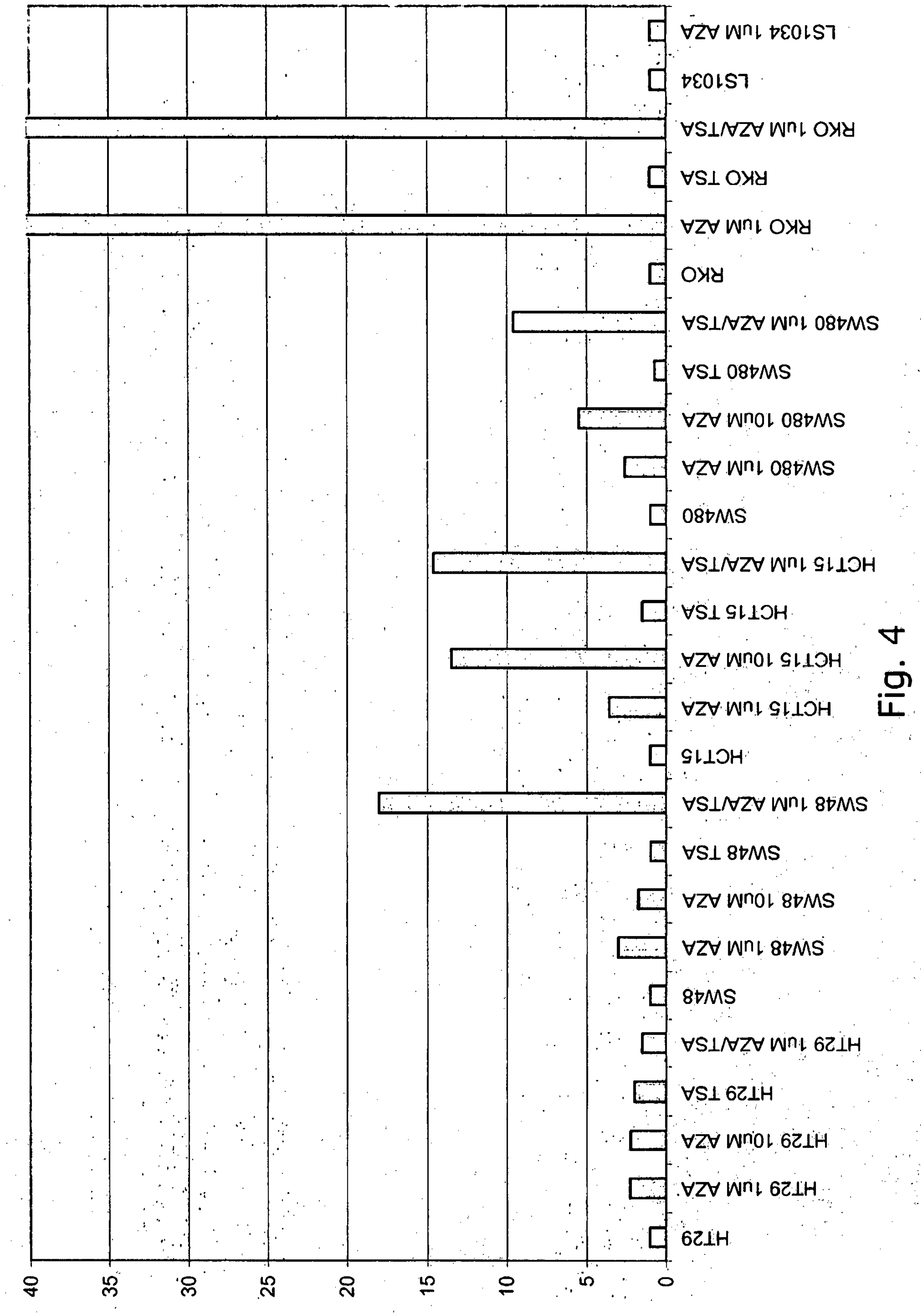
HT29
HT29 1uM AZA
HT29 10uM AZA
HT29 TSA
HT29 1uM AZA/TSA
SW48
SW48 1uM AZA
SW48 10uM AZA
SW48 TSA
SW48 1uM AZA/TSA
HCT15
HCT15 1uM AZA
HCT15 10uM AZA
HCT15 TSA
HCT15 1uM AZA/TSA
SW480
SW480 1uM AZA
SW480 10uM AZA
SW480 TSA
SW480 1uM AZA/TSA
RKO
RKO 1uM AZA
RKO TSA
RKO 1uM AZA/TSA
LS1034
LS1034 1uM AZA
0
5
10
15
20
25
30
35
40

Fig. 4

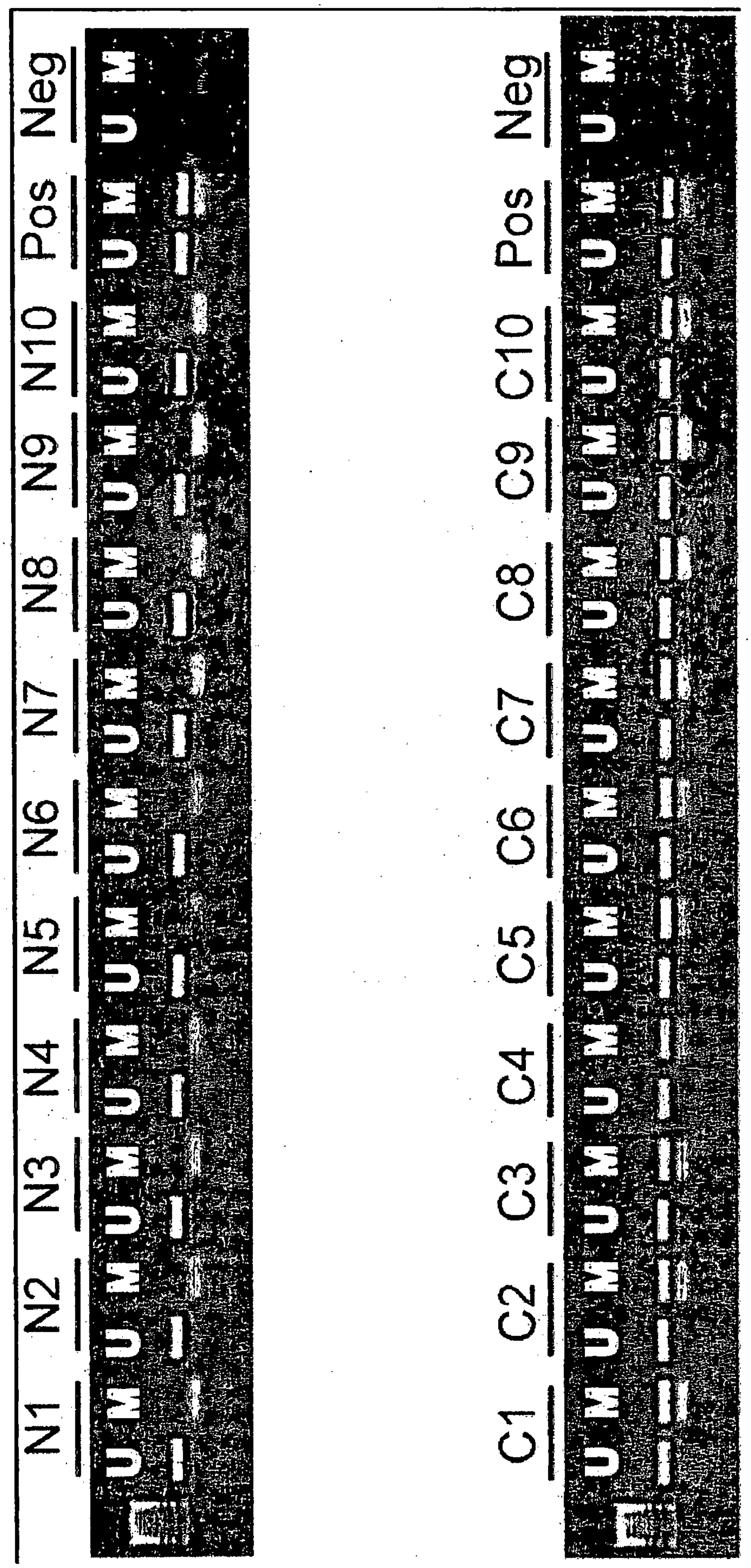
N1 N2 N3 N4 N5 N6 N7 N8 N9 N10 Pos Neg
U M U M U M U M U M U M U M U M U M U M U M U M
C1 C2 C3 C4 C5 C6 C7 C8 C9 C10 Pos Neg
U M U M U M U M U M U M U M U M U M U M U M U M

Fig. 5

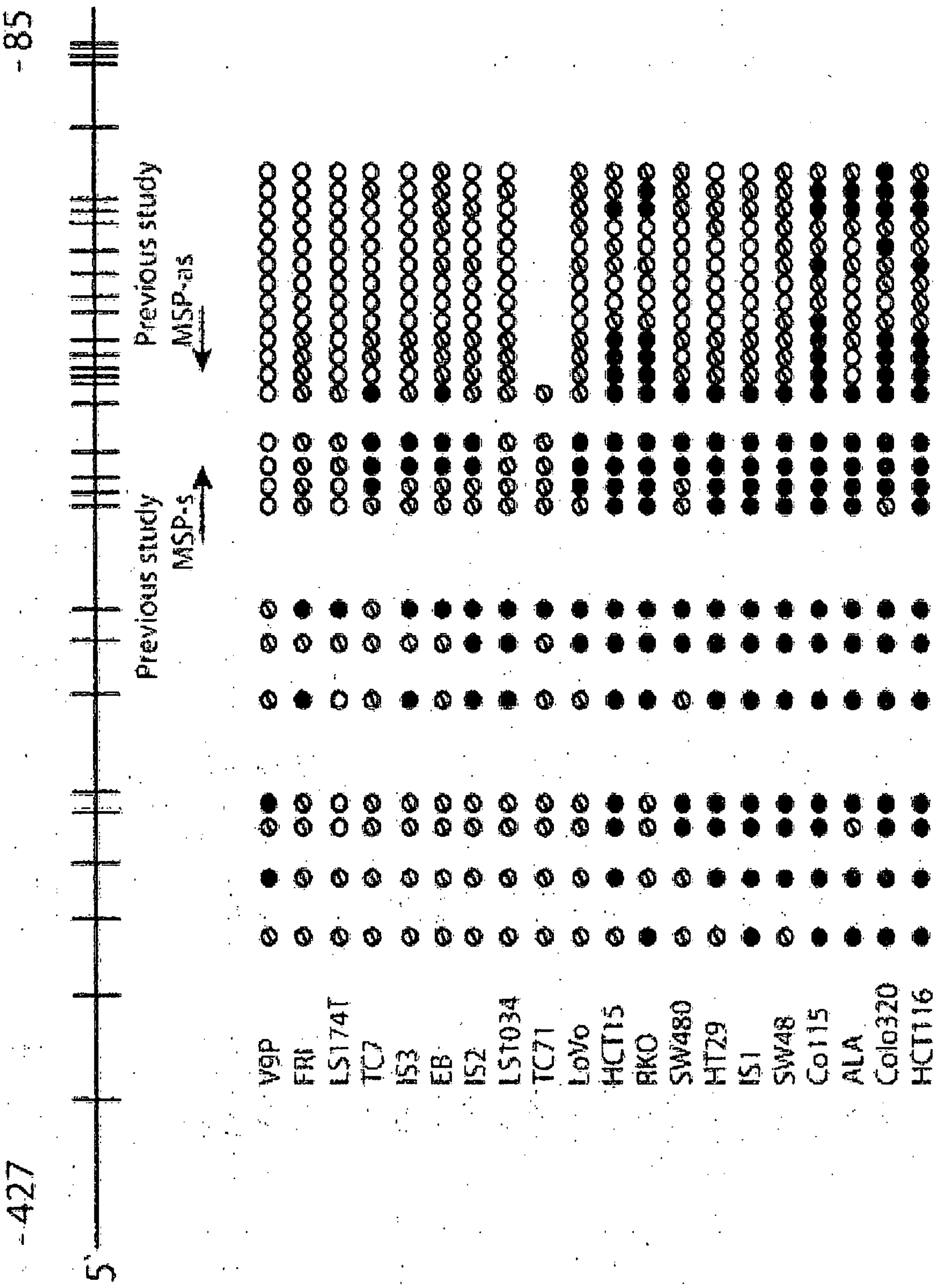


Fig. 6 (cont. next page)

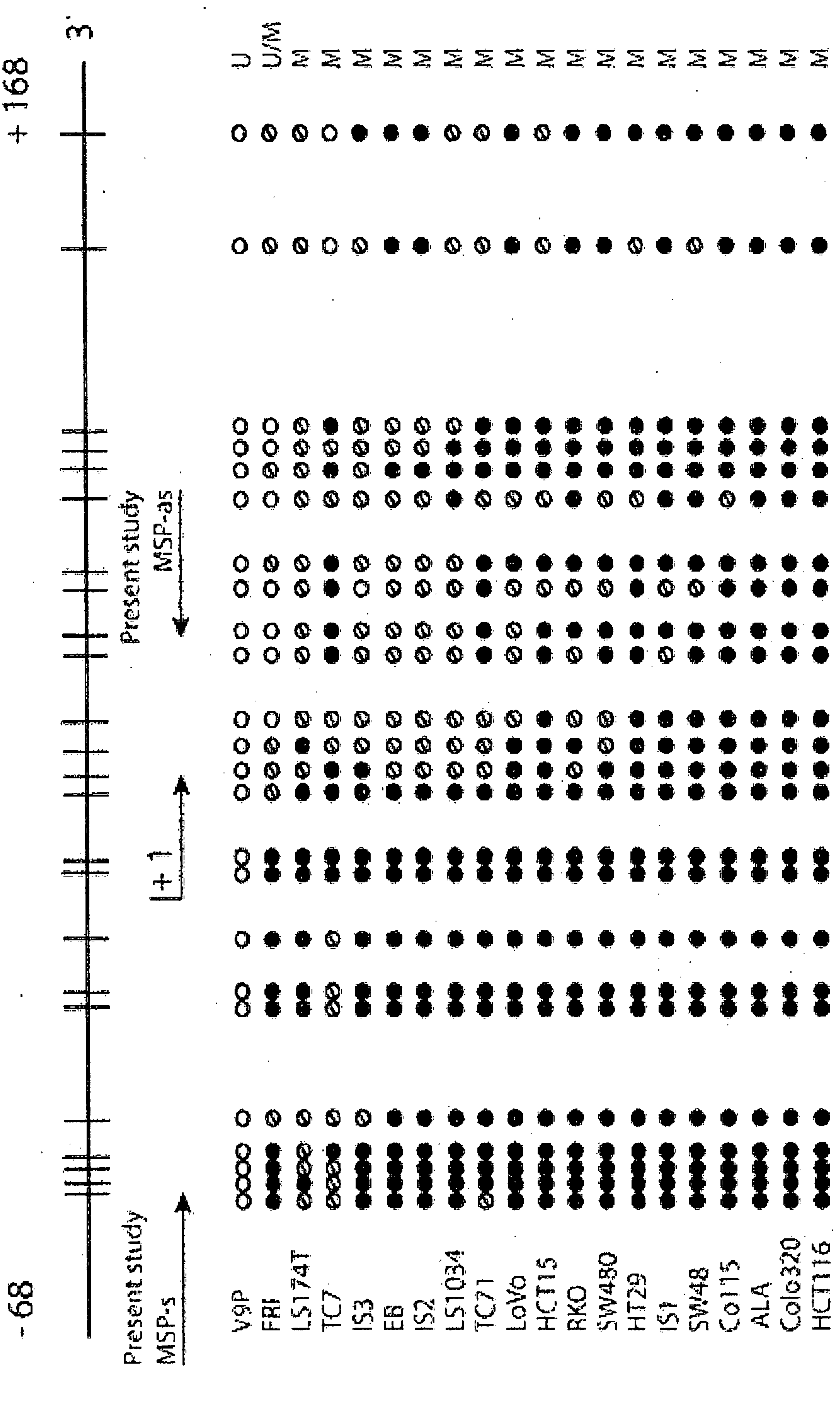


Fig. 6 (cont. from previous page)

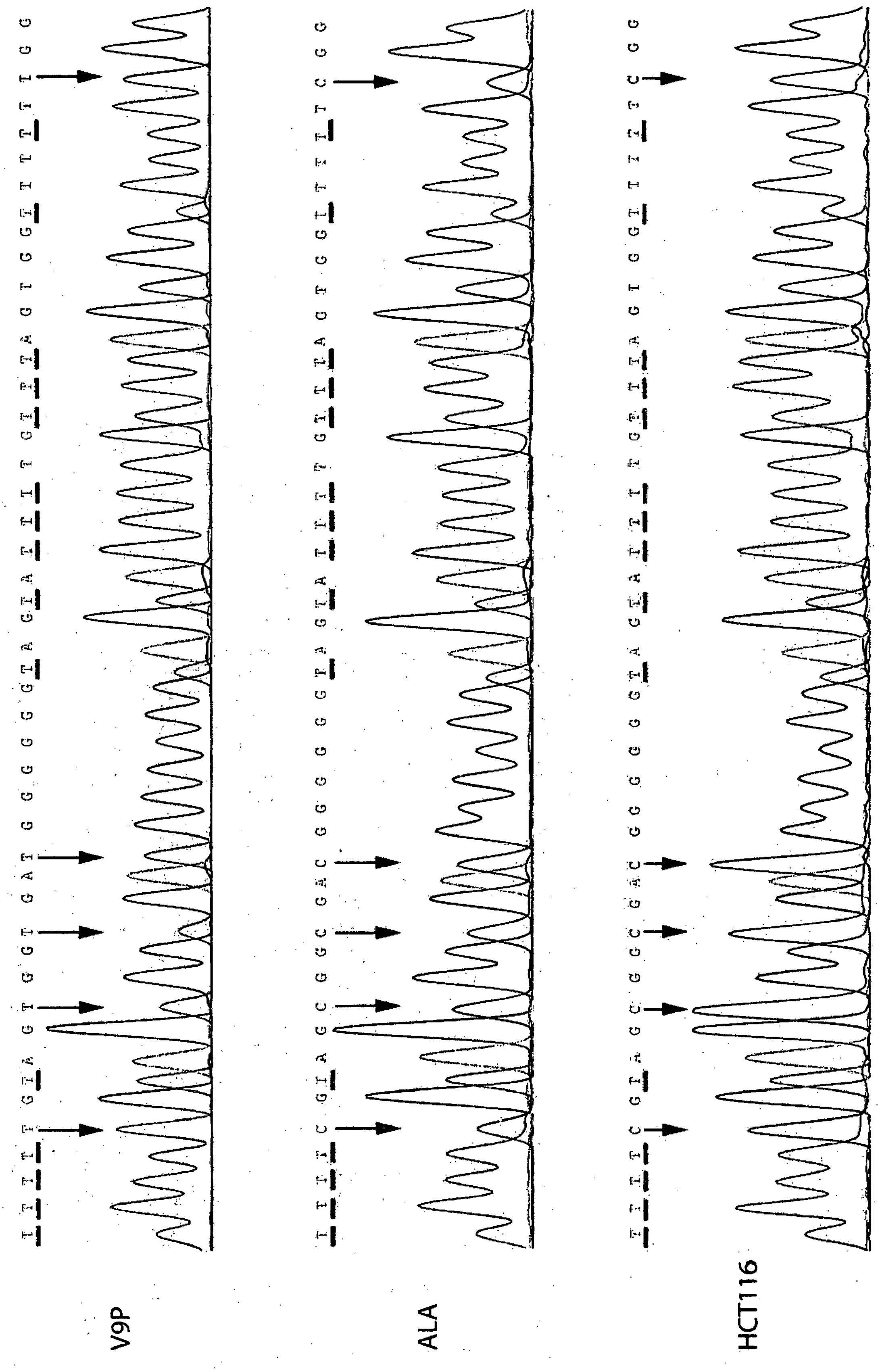


Fig. 7

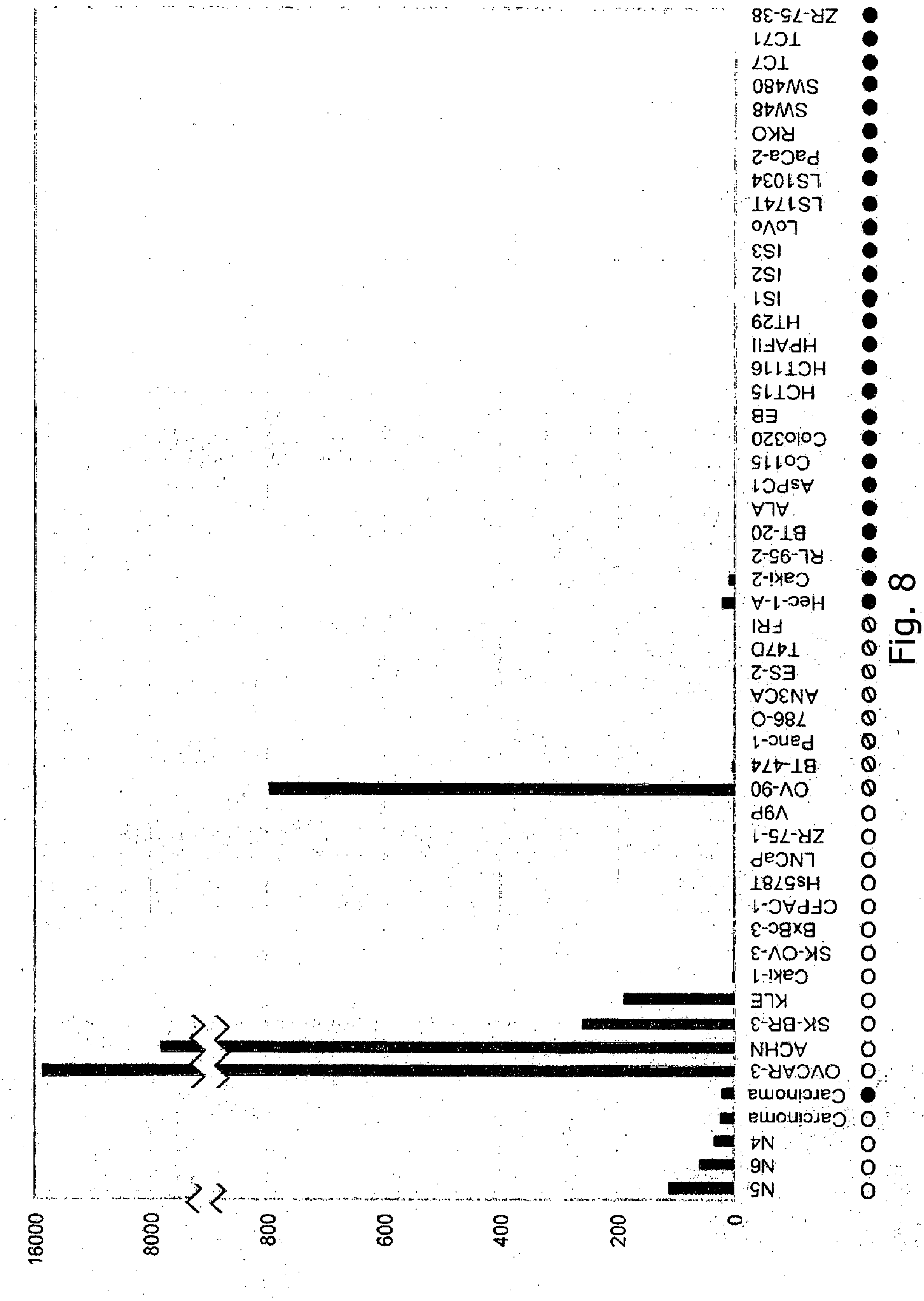
16000
8000
800
600
400
200
0
N5
N6
N4
Carcinoma
Carcinoma
OVCAR-3
ACHN
SK-BR-3
KLE
Caki-1
SK-OV-3
BxBc-3
CFPAC-1
Hs578T
LNCaP
ZR-75-1
V9P
OV-90
BT-474
Panc-1
786-O
AN3CA
ES-2
T47D
FRI
Hec-1-A
Caki-2
RL-95-2
BT-20
ALA
AsPC1
Co115
Colo320
EB
HCT15
HCT116
HPAFII
HT29
IS1
IS2
IS3
LoVo
LS174T
LS1034
PaCa-2
RKO
SW48
SW480
TC7
TC71
ZR-75-38

Fig. 8

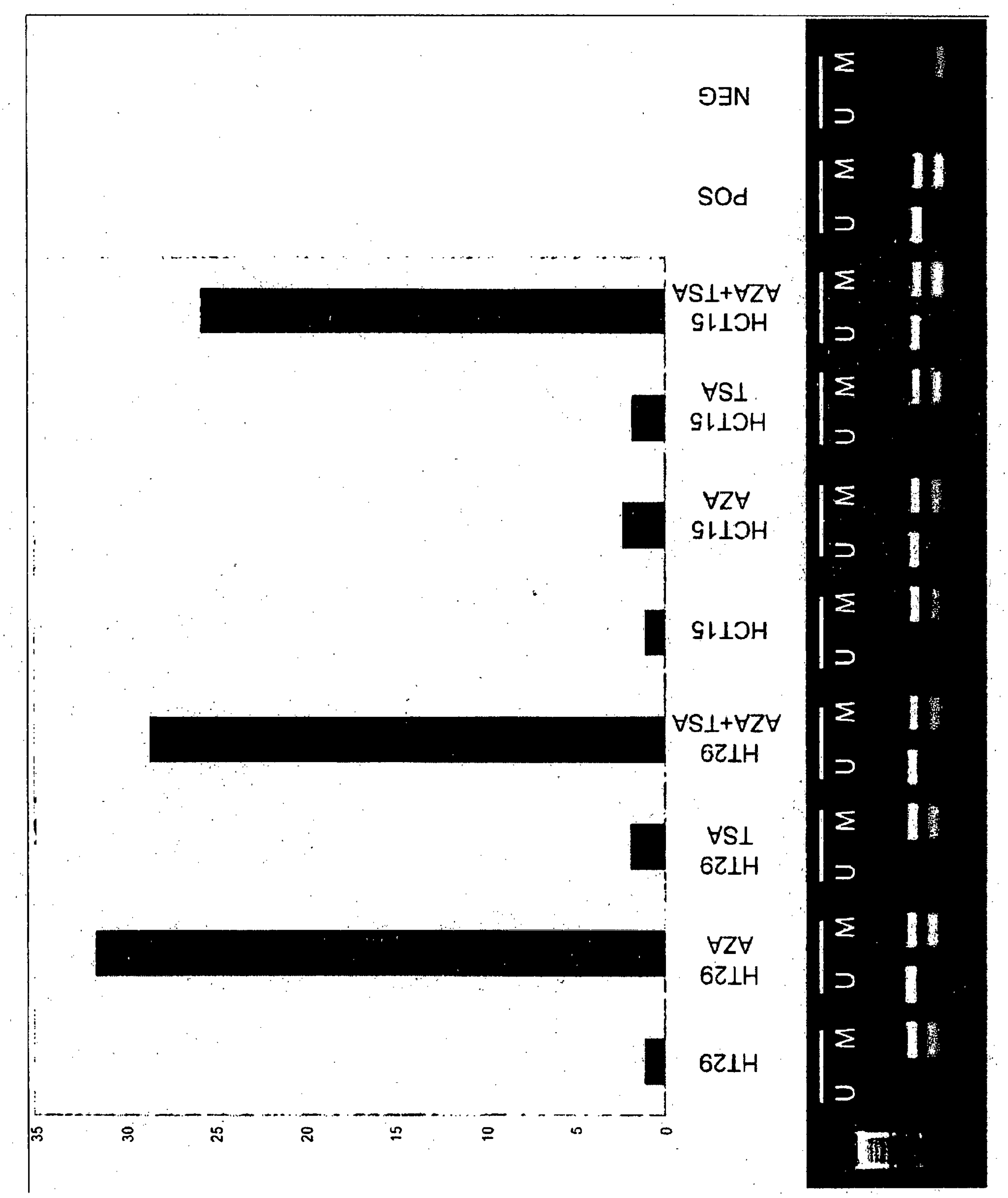

0
5
10
15
20
25
30
35
HT29
HT29 AZA
HT29 TSA
HT29 AZA+TSA
HCT15
HCT15 AZA
HCT15 TSA
HCT15 AZA+TSA
POS
NEG
U M


Fig. 9

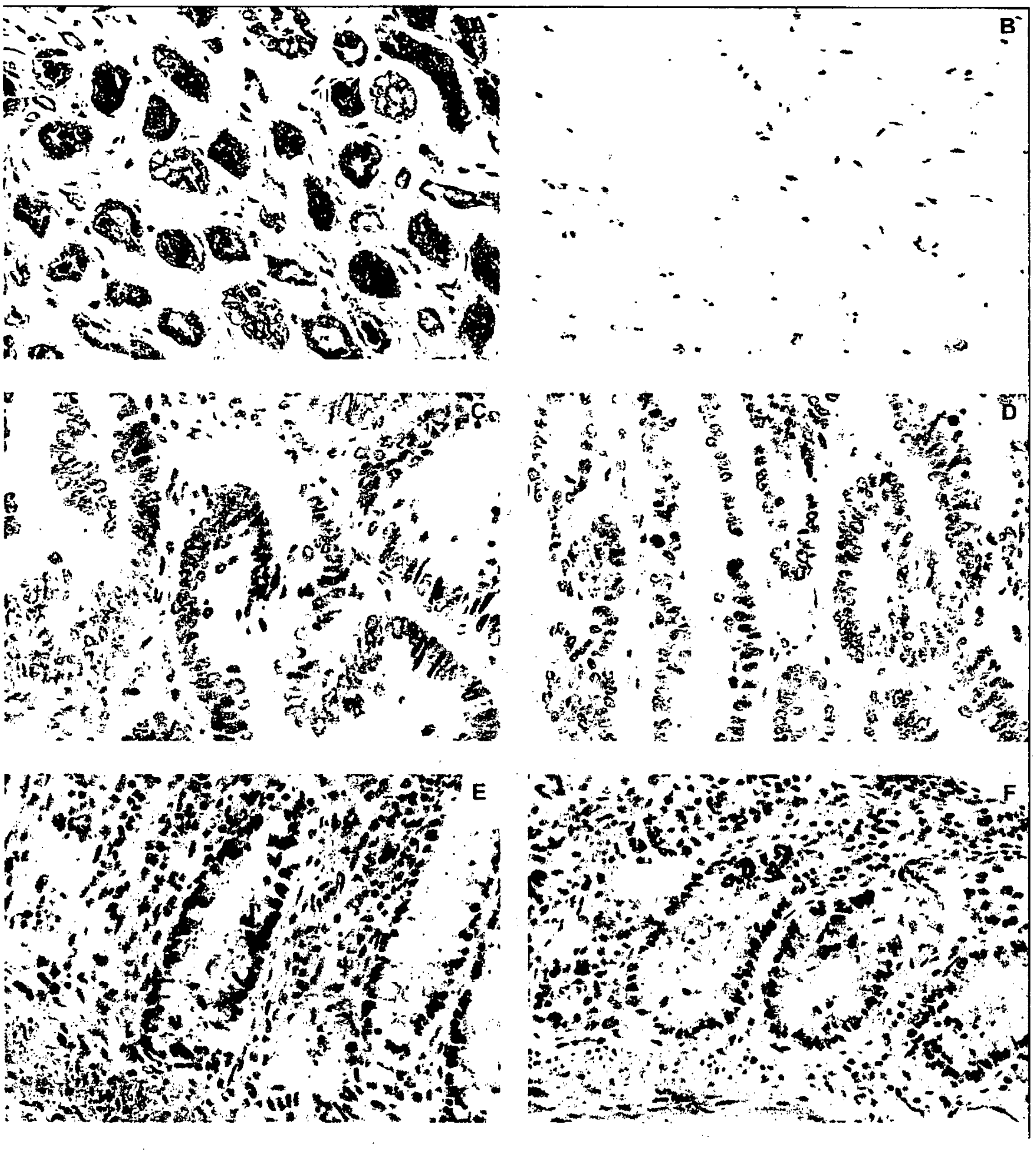


Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005005601 A
- WO 2005042725 A
- WO 0052204 A
- WO 0149879 A
- WO 0119845 A
- WO PA200700273 A
- WO PA200700601 A

### Non-patent literature cited in the description


- **KATRIN BEYER.** *Acta Neuropathologica,* 15 July 2006, vol. 112, 237-251
- **BÖNSCH DOMINIKUS et al.** *Neuroreport,* 08 February 2005, vol. 16 (2), 167-170
- **MARAZUELA M et al.** *J Histochem Cytochem,* 2003, vol. 51, 665-674
- **SAMBROOK et al.** Molecular Cloning: A Laboratory Manual. Cold Spring Harbor, 1989
- **ZWEIG, M. H. ; CAMPBELL, G.** *Clin. Chem.,* 1993, vol. 39, 561-577
- **MORI Y ; CAI K ; CHENG Y ; WANG S ; PAUN B ; HAMILTON JP ; JIN Z ; SATO F ; BERKI AT ; KAN T.** A genome-wide search identifies epigenetic silencing of somatostatin, tachykinin-1, and 5 other genes in colon cancer. *Gastroenterology,* 2006, vol. 131, 797-808
- **LIND GE ; KLEIVI K ; MELING GI ; TEIXEIRA MR ; THIIS-EVENSEN E ; ROGNUM TO ; LOTHE RA.** ADAMTS1, CRABP1, and NR3C1 Identified as Epigenetically Deregulated genes in Colorectal Tumourigenesis. *Cell Oncol,* 2006, vol. 28, 259-272
- **LAIRD PW.** The power and the promise of DNA methylation markers. *Nat Rev Cancer,* 2003, vol. 3, 253-266
- **MULLER HM ; OBERWALDER M ; FIEGL H ; MORANDELL M ; GOEBEL G ; ZITT M ; MUHLTHALER M ; OFNER D ; MARGREITER R ; WIDSCHWENDTER M.** Methylation changes in faecal DNA: a marker for colorectal cancer screening?. *Lancet,* 2004, vol. 363, 1283-1285
- **CHEN WD ; HAN ZJ ; SKOLETSKY J ; OLSON J ; SAH J ; MYEROFF L ; PLATZER P ; LU S ; DAWSON D ; WILLIS J.** Detection in fecal DNA of colon cancer-specific methylation of the nonexpressed vimentin gene. *J Natl Cancer Inst,* 2005, vol. 97, 1124-1132
- **C.GRUNAU ; S.J.CLARK ; A.ROSENTHAL.** Bisulfite genomic sequencing: systematic investigation of critical experimental parameters. *Nucleic Acids Res.,* 2001, vol. 29, E65
- **M.F.FRAGA ; M.ESTELLER.** DNA methylation: a profile of methods and applications. *Biotechniques,* 2002, vol. 33, 632-649
- **B.SMITH-SØRENSEN ; G.E.LIND ; R.I.SKOTHEIM ; S.D.FOSSA ; Ø.FODSTAD ; A.E.STENWIG ; K.S.JAKOBSEN ; R.A.LOTHE.** Frequent promoter hypermethylation of the 06-Methylguanine-DNA Methyltransferase (MGMT) gene in testicular cancer. *Oncogene,* 2002, vol. 21, 8878-8884
- **J.G.HERMAN ; J.R.GRAFF ; S.MYÖHÄNEN ; B.D.NELKIN ; S.B.BAYLIN.** Methylation-specific PCR: a novel PCR assay for methylation status of CpG islands. *Proc. Natl. Acad. Sci. U. S. A.,* 1996, vol. 93, 9821-9826
- **S.DERKS ; M.H.LENTJES ; D.M.HELLEBREKERS ; A.P.DE BRUINE ; J.G.HERMAN ; E.M.VAN.** Methylation-specific PCR unraveled. *Cell Oncol.,* 2004, vol. 26, 291-299
- **L.C.LI ; R.DAHIYA.** MethPrimer: designing primers for methylation PCRs. *Bioinformatics,* 2002, vol. 18, 1427-1431
- **J.R.MELKI ; P.C.VINCENT ; S.J.CLARK.** Concurrent DNA hypermethylation of multiple genes in acute myeloid leukemia. *Cancer Res.,* 1999, vol. 59, 3730-3740